# FASCICULE DE BREVET EUROPEEN

(11) **EP 3 472 174 B1**
(45) Date de publication et mention de la délivrance du brevet: **18.11.2020**
(21) Numéro de dépôt: 17742481.9
(22) Date de dépôt: 20.06.2017
(51) Int. Cl.: C07F 9/6553, C07D 333/48, C07D 513/04, C08C 19/00, C07F 9/40, C08G 77/00

(54) **PROCEDE DE PREPARATION DE THIOLACTONES SUBSTITUEES, NOUVELLES THIOLACTONES SUBSTITUEES ET UTILISATIONS**
VERFAHREN ZUR HERSTELLUNG VON SUBSTITUIERTEN THIOLACTONEN, NEUE SUBSTITUIERTE THIOLACTONE UND VERWENDUNGEN DAVON
METHOD FOR THE PREPARATION OF SUBSTITUTED THIOLACTONES, NEW SUBSTITUTED THIOLACTONES, AND USES THEREOF

(30) Priorité: 21.06.2016 FR 1655781; 08.11.2016 FR 1660808
(43) Date de publication de la demande: 24.04.2019
(73) Titulaire: Centre National de la Recherche Scientifique, 75016 Paris (FR); Université Toulouse III-Paul Sabatier, 31062 Toulouse (FR)
(72) Inventeur: COUTELIER, Olivier, 31320 Auzeville Tolosane (FR); DESTARAC, Mathias, 31130 Balma (FR); KULAI, Ihor, Kyiv 02154 (UA)
(74) Mandataire: Ipsilon
(86) Numéro de dépôt international: PCT/FR2017/051637
(87) Numéro de publication internationale: WO 2017/220925

(56) Documents cités:
- WO-A1-2013/033525
- BOIVIN J ET AL: "An Expedient Radical Based Approach to Difluorophosphonate Analogues of Thionucleosides", TETRAHEDRON LETTERS, PERGAMON, GB, vol. 39, no. 38, 17 septembre 1998 (1998-09-17), pages 6877-6880, XP004132628, ISSN: 0040-4039
- STAMENOVIC, MILAN M. ET AL: "Straightforward synthesis of functionalized cyclic polymers in high yield via RAFT and thiolactone-disulfide chemistry", POLYMER CHEMISTRY , 4(1), 184-193 CODEN: PCOHC2; ISSN: 1759-9962, 2013, XP002762508,

## Description

La présente invention est relative au domaine des thiolactones.

Plus particulièrement, la présente invention concerne un procédé de préparation de thiolactones substituées de formule (I), de nouvelles thiolactones substituées de formule (I') susceptibles d'être obtenues par la mise en œuvre de ce procédé, l'utilisation de thiolactones substituées de formule (I) ou de formule (I') pour la préparation de polymères ou pour la fonctionnalisation de surface ou de polymères.

Les thiolactones sont des composés hétérocycliques analogues des lactones, dans lesquelles un atome d'oxygène est remplacé par un atome de soufre. L'atome de soufre se situe dans le cycle et est adjacent à un groupe carbonyle. L'hétérocycle des thiolactones peut être substitué par au moins un groupement chimique, en particulier par un groupement alkyle ou aryle.

Plusieurs procédés de synthèse de thiolactones ont déjà été proposés.

F. Korte et al. (Chem. Ber., 1961, 94, 1966) ont par exemple proposé soit de réaliser une cyclisation thermique d'un acide mercaptocarboxylique portant un substituant alkyle soit de substituer directement une thiolactone par un radical alkyle en présence d'un groupement halogénure d'alkyle (R-X) et de dialkylamide de lithium (LiNR'₂). Ces deux voies de synthèse peuvent être représentées par le schéma réactionnel (1) suivant :

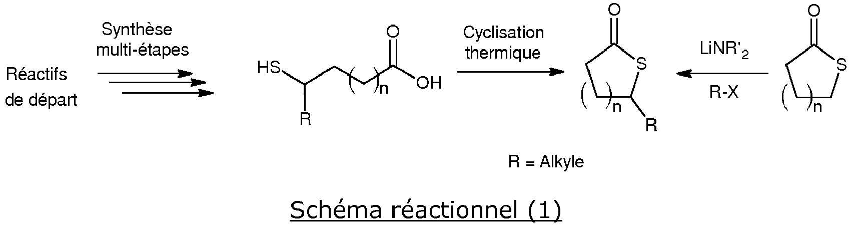

Selon cette voie de synthèse, seule la dernière étape de cyclisation thermique est générale, les étapes précédentes conduisant à l'acide mercaptocarboxylique et les réactifs mis en œuvre étant spécifiques au type de groupement R que l'on souhaite introduire sur l'hétérocycle. De plus, ces deux voies de synthèse ne permettent pas l'introduction de substituants autres que des groupements alkyle.

Un procédé de synthèse plus récent permet d'accéder à des thiolactones possédant des groupements alkyle ou aryle (J.-J. Filippi *et al.,* d'une thionolactone vers une thiolactone en présence de trifluorure de bore (BF₃) et d'éther diéthylique (Et₂O) dans un solvant organique tel que le toluène au reflux selon le schéma réactionnel (2) suivant :

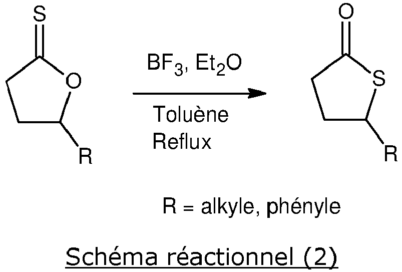

Cependant, ce procédé utilise un catalyseur de type acide de Lewis (le trifluorure de bore) et ne peut pas être facilement utilisé pour la synthèse de thiolactones portant des substituants autres que des groupements alkyle ou phényle tels que des fonctions organiques complexes et/ou incompatibles avec ce type de catalyseur. De plus les rendements isolés des thiolactones sont souvent faibles. Enfin, ce procédé nécessite la synthèse des thionolactones de départ à partir des lactones correspondantes.

Boivin et al. (Tetrahedron Letters, 1998, 39, 38, 6877-6880) décrit la préparation d'une thiolactone comportant un substituant fluoré. Stamenovic et al., (Polymer Chemistry, 2013, 4, 184-193) et WO 2013/03352 décrivent la synthèse de polymères cycliques fonctionnalisés à partir de thiolactones.

Il existe donc un besoin pour un procédé qui permette de synthétiser des thiolactones substituées par des groupements fonctionnels variés de façon souple et simple, et selon un procédé qui soit à la fois efficace et économique.

La présente invention a donc pour premier objet un procédé de préparation de thiolactones substituées de formule (I) suivante : 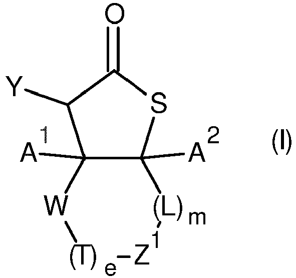 dans laquelle :
- A¹ et A², identiques ou différents, représentent un atome d'hydrogène ou un atome de fluor,
- Y représente un atome d'hydrogène ou un groupe choisi parmi les groupes alkyle, hydroxyalkyle, aryle et cyano, ou une chaîne polymère ;
- L est une chaine alkylène linéaire pouvant être interrompue par un ou plusieurs atomes d'oxygène, ladite chaine ayant de 1 à 12 atomes de carbone.
- Y représente un atome d'hydrogène ou un groupe choisi parmi les groupes alkyle, hydroxyalkyle, aryle et cyano, ou une chaîne polymère ;
- L est un bras de liaison,
- m est un nombre entier égal à 0 ou 1,
- T représente CH₂, -O- ou -NR⁶- dans lequel R⁶ représente un atome d'hydrogène ou un radical alkyle, aryle ou aralkyle éventuellement substitué par un groupement choisi parmi les groupes : maléimide, un groupement de formule : 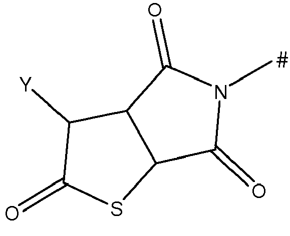 dans lequel le sigle # est le point d'attache dudit groupement à R⁶ et dans lequel Y à la même signification que celle choisie pour le radical Y de la formule (I), OH ; P(O)(OR⁷)(OR^{7'}) dans lequel les radicaux R⁷ et R^{7'}, identiques ou différents, représentent un atome d'hydrogène ou un radical alkyle ; CₙF₂ₙ₊₁ dans lequel n est un nombre entier allant de 1 à 20 ; SiR⁸ₚ(OR⁹)₃₋ₚ dans lequel les radicaux R⁸ et R⁹, identiques ou différents, représentent un atome d'hydrogène ou un radical alkyle et p est un nombre entier égal à 0, 1 ou 2 ; BF₃M⁺ dans lequel M = K ou Na ; B(OR¹⁰)₂ dans lequel les deux radicaux R¹⁰, identiques ou différents, représentent un atome d'hydrogène, un radical alkyle ou forment un cycle carboné avec les deux atomes d'oxygène auxquels ils sont liés ; OR¹¹ dans lequel R¹¹ représente un atome d'hydrogène ou un radical alkyle, aryle ou aralkyle ; O(C=O)R¹² dans lequel R¹² représente un atome d'hydrogène ou un radical alkyle, aryle ou aralkyle ; O(C=O)OR¹³ dans lequel R¹³ représente un atome d'hydrogène ou un radical alkyle, aryle ou aralkyle ; N⁺R¹⁴R^{14'}R^{14"}A⁻ dans lequel les radicaux R¹⁴, R^{14'} et R^{14"}, identiques ou différents, représentent un atome d'hydrogène ou un radical alkyle, aryle ou aralkyle et A représente un atome de chlore ou de brome ; NR^{15'}(C=O)R¹⁵ dans lequel les radicaux R¹⁵ et R^{15'}, identiques ou différents, représentent un atome d'hydrogène ou un radical alkyle ou aryle ou sont reliés entre eux et forment un cycle tel qu'un cycle pyrrolidone ou caprolactame ; NR^{16'}(C=O)OR¹⁶ dans lequel R¹⁶ et R^{16'}, identiques ou différents, représentent un atome d'hydrogène ou un radical alkyle, aryle ou aralkyle ; CN ; un atome d'halogène choisi parmi Cl, F, et Br ; NCS ; OCH₂-époxy ; COOR¹⁷ dans lequel R¹⁷ représente un atome d'hydrogène, un radical alkyle, aryle ou aralkyle ; CONR¹⁸R^{18'} dans lequel R¹⁸ et R^{18'}, identiques ou différentes, représentent un atome d'hydrogène ou un radical alkyle ou aryle ; SO₂R¹⁹ dans lequel R¹⁹ représente un radical alkyle ou aryle ; azoture N₃ et alcyne,
- e est un nombre entier égal à 0 ou 1,

Etant entendu que :
1) lorsque A¹ = A² = H et que e = 0, alors W représente un atome d'hydrogène et Z¹ représente un groupe choisi parmi les groupes alkyle ; aryle ; P(O) (OR⁷)(OR^{7'}) dans lequel les radicaux R⁷ et R^{7'}, identiques ou différents, représentent un atome d'hydrogène ou un radical alkyle ; CₙF₂ₙ₊₁ dans lequel n est un nombre entier allant de 1 à 20 ; SiR⁸ₚ(OR⁹)₃₋ₚ dans lequel les radicaux R⁸ et R⁹, identiques ou différents, représentent un atome d'hydrogène ou un radical alkyle et p est un nombre entier égal à 0, 1 ou 2 ; BF₃M⁺ dans lequel M = K ou Na ; B(OR¹⁰)₂ dans lequel les deux radicaux R¹⁰, identiques ou différents, représentent un atome d'hydrogène, un radical alkyle ou forment un cycle carboné avec les deux atomes d'oxygène auxquels ils sont liés ; OR¹¹ dans lequel R¹¹ représente un atome d'hydrogène ou un radical alkyle, aryle ou aralkyle ; O(C=O)R¹² dans lequel R¹² représente un atome d'hydrogène ou un radical alkyle, aryle ou aralkyle ; O(C=O)OR¹³ dans lequel R¹³ représente un atome d'hydrogène ou un radical alkyle, aryle ou aralkyle ; N⁺R¹⁴R^{14'}R^{14"}A⁻ dans lequel les radicaux R¹⁴, R^{14'} et R^{14"}, identiques ou différents, représentent un atome d'hydrogène ou un radical alkyle, aryle ou aralkyle et A représente un atome de chlore ou de brome ; NR^{15'}(C=O)R¹⁵ dans lequel les radicaux R¹⁵ et R^{15'}, identiques ou différents, représentent un atome d'hydrogène ou un radical alkyle ou aryle ou sont reliés entre eux et forment un cycle tel qu'un cycle pyrrolidone ou caprolactame ; NR^{16'}(C=O)OR¹⁶ dans lequel R¹⁶ et R^{16'}, identiques ou différents, représentent un atome d'hydrogène ou un radical alkyle, aryle ou aralkyle ; CN ; un atome d'halogène choisi parmi Cl, F, et Br ; NCS ; OCH₂-époxy ; COOR¹⁷ dans lequel R¹⁷ représente un atome d'hydrogène, un radical alkyle, aryle ou aralkyle ; CONR¹⁸R^{18'} dans lequel R¹⁸ et R^{18'}, identiques ou différentes, représentent un atome d'hydrogène ou un radical alkyle ou aryle ; SO₂R¹⁹ dans lequel R¹⁹ représente un radical alkyle ou aryle ; azoture N₃ et alcyne ; et un cycle thiolactone de formule 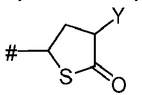 dans laquelle le sigle # est le point d'attache du cycle thiolactone à L et dans laquelle Y à la même signification que celle choisie pour le radical Y de la formule (I),
2) lorsque A¹ = A² = H, m = 1 et que e = 0, alors W représente un atome d'hydrogène et Z¹ peut en outre représenter un atome d'hydrogène ;
3) lorsque A¹ = A² = H, e = 1 et que m = 0, alors Z¹ et W sont identiques et représentent -CH₂- ou CO ;
4) lorsque A¹ = A² = H, e = 1, m = 1 et que T= CH₂ alors Z¹ = W = -CH₂,
5) lorsque A¹ = A² = F, e = 0 et que m = 0, alors W représente un atome de fluor et Z¹ = F ou représente une chaine OC_{q}F_{2q+1} linéaire ou ramifiée dans laquelle q est un nombre entier allant de 1 à 5, [OCF₂CF(CF₃)]ᵣOC₃F₇ avec r = un nombre entier allant de 0 à 20, OC₂F₄SO₂F, OCF₂CF(CF₃)OC₂F₄SO₂F, ou OCF₂CF(CF₃)OC₂F₄CO₂CH₃ ;
6) lorsque A¹ = A² = F, e=1 et que m = 0, alors W = Z¹ = CF₂ et T = (CF₂)ₛ avec s = un nombre entier allant de 1 à 5 ; et
7) lorsque A¹ = H, A² = F, et que e = m = 0, alors W représente un atome d'hydrogène et Z¹ = F ou CₙF₂ₙ₊₁ dans lequel n est un nombre entier allant de 1 à 20 ;
ledit procédé étant caractérisé en ce qu'il comprend au moins les étapes suivantes :
1) une étape au cours de laquelle on fait réagir, en présence d'un amorceur radicalaire, un xanthate de formule (II) suivante : 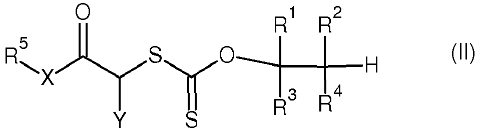 dans laquelle :
   - R¹, R², R³ et R⁴, identiques ou différents, représentent un atome d'hydrogène ou un groupe choisi parmi les groupes alkyle, acyle, aryle, alcène, alcyne, cycloalkyle, hétérocycloalkyle saturé ou insaturé, hétérocycloaryle, et les chaines de polymères, étant entendu que les radicaux R¹, R², R³ et R⁴ peuvent également former ensemble un groupement cycloalkyle ou hétérocycloalkyle saturé, insaturé ou aromatique ; étant entendu qu'au moins un des radicaux R¹ et R³ est différent d'un atome d'hydrogène ;
   - Y a la même signification que dans la formule (I) ci-dessus,
   - X représente NR²⁰ dans lequel R²⁰ représente un atome d'hydrogène ou un radical alkyle, ou -O-,
   - R⁵ est choisi parmi un groupe alkyle, acyle, aryle, aralkyle, cycloalkyle ou hétérocycloalkyle saturé, insaturé ou aromatique ;
   avec un monomère comportant au moins une insaturation éthylénique de formule (III) suivante : 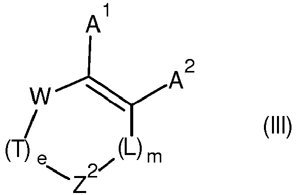 dans laquelle :
   - A¹ et A², identiques ou différents, représentent un atome d'hydrogène ou un atome de fluor,
   - L, m, T et e ont la même signification que dans la formule (I) ci-dessus ;

Etant entendu que :
1) lorsque A¹ = A² = H et que e = 0, alors W représente un atome d'hydrogène et Z² représente un groupe choisi parmi les groupes alkyle ; aryle ; P(O)(OR⁷)(OR^{7'}) dans lequel les radicaux R⁷ et R^{7'}, identiques ou différents, représentent un atome d'hydrogène ou un radical alkyle ; CₙF₂ₙ₊₁ dans lequel n est un nombre entier allant de 1 à 20 ; SiR⁸ₚ(OR⁹)₃₋ₚ dans lequel les radicaux R⁸ et R⁹, identiques ou différents, représentent un atome d'hydrogène ou un radical alkyle et p est un nombre entier égal à 0, 1 ou 2 ; BF₃M⁺ dans lequel M = K ou Na ; B(OR¹⁰)₂ dans lequel les deux radicaux R¹⁰, identiques ou différents, représentent un atome d'hydrogène, un radical alkyle ou forment un cycle carboné avec les deux atomes d'oxygène auxquels ils sont liés ; OR¹¹ dans lequel R¹¹ représente un atome d'hydrogène ou un radical alkyle, aryle ou aralkyle ; O(C=O)R¹² dans lequel R¹² représente un atome d'hydrogène ou un radical alkyle, aryle ou aralkyle ; O(C=O)OR¹³ dans lequel R¹³ représente un atome d'hydrogène ou un radical alkyle, aryle ou aralkyle ; N⁺R¹⁴R^{14'}R^{14"}A⁻ dans lequel les radicaux R¹⁴, R^{14'} et R^{14"}, identiques ou différents, représentent un atome d'hydrogène ou un radical alkyle, aryle ou aralkyle et A représente un atome de chlore ou de brome ; NR^{15'}(C=O)R¹⁵ dans lequel les radicaux R¹⁵ et R^{15'}, identiques ou différents, représentent un atome d'hydrogène ou un radical alkyle ou aryle ou sont reliés entre eux et forment un cycle tel qu'un cycle pyrrolidone ou caprolactame ; NR^{16'}(C=O)OR¹⁶ dans lequel R¹⁶ et R^{16'}, identiques ou différents, représentent un atome d'hydrogène ou un radical alkyle, aryle ou aralkyle ; CN ; un atome d'halogène choisi parmi Cl, F, et Br ; NCS ; OCH₂-époxy ; COOR¹⁷ dans lequel R¹⁷ représente un atome d'hydrogène, un radical alkyle, aryle ou aralkyle ; CONR¹⁸R^{18'} dans lequel R¹⁸ et R^{18'}, identiques ou différentes, représentent un atome d'hydrogène ou un radical alkyle ou aryle ; SO₂R¹⁹ dans lequel R¹⁹ représente un radical alkyle ou aryle ; azoture N₃ ; alcyne et C₂H₃ (i.e. CH=CH₂) ;
2) lorsque A¹ = A² = H, m = 1 et que e = 0, alors W représente un atome d'hydrogène et Z² peut en outre représenter un atome d'hydrogène,
3) lorsque A¹ = A² = H, e = 1 et que m = 0, alors Z² et W sont identiques et représentent -CH₂- ou CO ;
4) lorsque A¹ = A² = H, e = 1, m = 1 et que T= CH₂ alors Z² = W = -CH₂,
5) lorsque A¹ = A² = F, e = 0 et que m = 0, alors W représente un atome de fluor et Z² = F ou représente une chaine OC_{q}F_{2q+1} linéaire ou ramifiée dans laquelle q est un nombre entier allant de 1 à 5, [OCF₂CF(CF₃)]ᵣOC₃F₇ avec r = un nombre entier allant de 0 à 20, OC₂F₄SO₂F, OCF₂CF(CF₃)OC₂F₄SO₂F, ou OCF₂CF(CF₃)OC₂F₄CO₂CH₃ ; et
6) lorsque A¹ = A² = F, e=1 et que m = 0, alors W = Z² = CF₂ et T = (CF₂)ₛ avec s = un nombre entier allant de 1 à 5 ;
7) lorsque A¹ = H, A² = F, et que e = m = 0, alors W représente un atome d'hydrogène et Z² = F ou CₙH₂ₙ₊₁ dans lequel n est un nombre entier allant de 1 à 20 ;
pour former un mono-adduit de formule (IV) suivante : 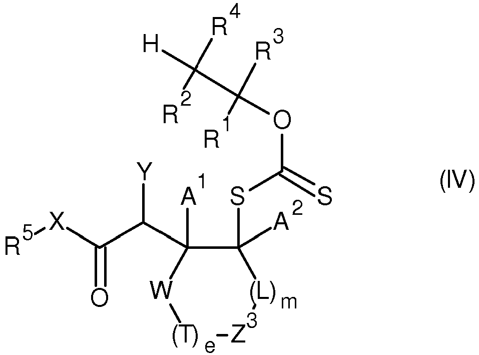 dans laquelle :
- A¹ et A², identiques ou différents, représentent un atome d'hydrogène ou un atome de fluor,
- R¹, R², R³, R⁴, R⁵, X et Y ont la même signification que dans la formule (II) ci-dessus,
- L, m, T et e ont la même signification que dans la formule (I) ci-dessus,

Etant entendu que :
1) lorsque A¹ = A² = H et que e = 0, alors W représente un atome d'hydrogène et Z³ représente un groupe choisi parmi les groupes alkyle ; aryle ; P(O)(OR⁷)(OR^{7'}) dans lequel les radicaux R⁷ et R^{7'}, identiques ou différents, représentent un atome d'hydrogène ou un radical alkyle ; CₙF₂ₙ₊₁ dans lequel n est un nombre entier allant de 1 à 20 ;SiR⁸ₚ(OR⁹)₃₋ₚ dans lequel les radicaux R⁸ et R⁹, identiques ou différents, représentent un atome d'hydrogène ou un radical alkyle et p est un nombre entier égal à 0, 1 ou 2 ; BF₃M⁺ dans lequel M = K ou Na ; B(OR¹⁰)₂ dans lequel les deux radicaux R¹⁰, identiques ou différents, représentent un atome d'hydrogène, un radical alkyle ou forment un cycle carboné avec les deux atomes d'oxygène auxquels ils sont liés ; OR¹¹ dans lequel R¹¹ représente un atome d'hydrogène ou un radical alkyle, aryle ou aralkyle ; O(C=O)R¹² dans lequel R¹² représente un atome d'hydrogène ou un radical alkyle, aryle ou aralkyle ; O(C=O)OR¹³ dans lequel R¹³ représente un atome d'hydrogène ou un radical alkyle, aryle ou aralkyle ; N⁺R¹⁴R^{14'}R^{14"}A dans lequel les radicaux R¹⁴, R^{14'} et R^{14"}, identiques ou différents, représentent un atome d'hydrogène ou un radical alkyle, aryle ou aralkyle et A représente un atome de chlore ou de brome; NR^{15'}(C=O)R¹⁵ dans lequel les radicaux R¹⁵ et R^{15'}, identiques ou différents, représentent un atome d'hydrogène ou un radical alkyle ou aryle ou sont reliés entre eux et forment un cycle tel qu'un cycle pyrrolidone ou caprolactame ; NR^{16'}(C=O)OR¹⁶ dans lequel R¹⁶ et R^{16'}, identiques ou différents, représentent un atome d'hydrogène ou un radical alkyle, aryle ou aralkyle ; CN ; un atome d'halogène choisi parmi Cl, F, et Br ; NCS ; OCH₂-époxy ; COOR¹⁷ dans lequel R¹⁷ représente un atome d'hydrogène, un radical alkyle, aryle ou aralkyle ; CONR¹⁸R^{18'} dans lequel R¹⁸ et R^{18'}, identiques ou différentes, représentent un atome d'hydrogène ou un radical alkyle ou aryle ; SO₂R¹⁹ dans lequel R¹⁹ représente un radical alkyle ou aryle ; azoture N₃ ; alcyne ; et un groupement de formule (V) suivante : 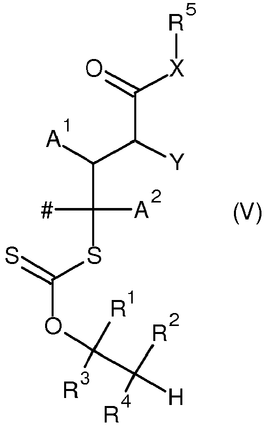 dans lequel le sigle # est le point d'attache du groupement de formule (V) à L et R¹, R², R³, R⁴, R⁵ et Y ont respectivement la même signification que celle choisie pour R¹, R², R³, R⁴, R⁵ et Y dans la formule (IV),
2) lorsque A¹ = A² = H, m = 1 et que e = 0, alors W représente un atome d'hydrogène et Z³ peut en outre représenter un atome d'hydrogène,
3) lorsque A¹ = A² = H, e = 1 et que m = 0, alors Z³ et W sont identiques et représentent -CH₂- ou CO ;
4) lorsque A¹ = A² = H, e = 1, m = 1 et que T= CH₂ alors Z³ = W = -CH₂,
5) lorsque A¹ = A² = F, e = 0 et que m = 0, alors W représente un atome de fluor et Z³ = F ou représente une chaine OC_{q}F_{2q+1} linéaire ou ramifiée dans laquelle q est un nombre entier allant de 1 à 5, [OCF₂CF(CF₃)]ᵣOC₃F₇ avec r = un nombre entier allant de 0 à 20, OC₂F₄SO₂F, OCF₂CF(CF₃)OC₂F₄SO₂F, ou OCF₂CF(CF₃)OC₂F₄CO₂CH₃ ; et
6) lorsque A¹ = A² = F, e = 1 et que m = 0, alors W = Z³ = CF₂ et T = (CF₂)ₛ avec s = un nombre entier allant de 1 à 5 ;
7) lorsque A¹ = H, A² = F, et que e = m = 0, alors W représente un atome d'hydrogène et Z³ = F ou CₙF₂ₙ₊₁ dans lequel n est un nombre entier allant de 1 à 20 ;
   puis
2) une étape de thermolyse du mono-adduit de formule (IV) obtenu ci-dessus à l'étape précédente pour former une thiolactone substituée de formule (I) correspondante.

Le procédé de préparation des thiolactones substituées de formule (I) conforme à l'invention peut être représenté par le schéma réactionnel (3) suivant :

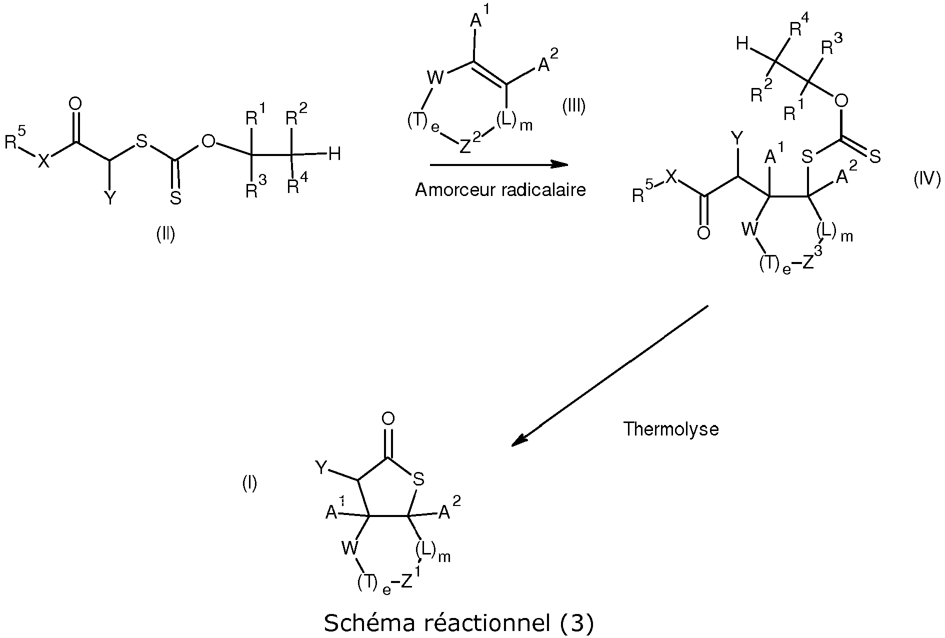

Grâce au procédé conforme à la présente invention et tel que décrit ci-dessus, il est désormais possible d'accéder de façon simple, rapide et avec un bon rendement à des thiolactones substituées par des groupements organiques variés.

Selon une première forme de réalisation particulièrement avantageuse, le procédé de l'invention conduit à la formation d'une di-thiolactone de formule (Ia), dans laquelle A¹, A², L, m et Y ont la même définition que dans la formule (I) et W=H, ladite di-thiolactone étant obtenue selon le schéma réactionnel (4a) 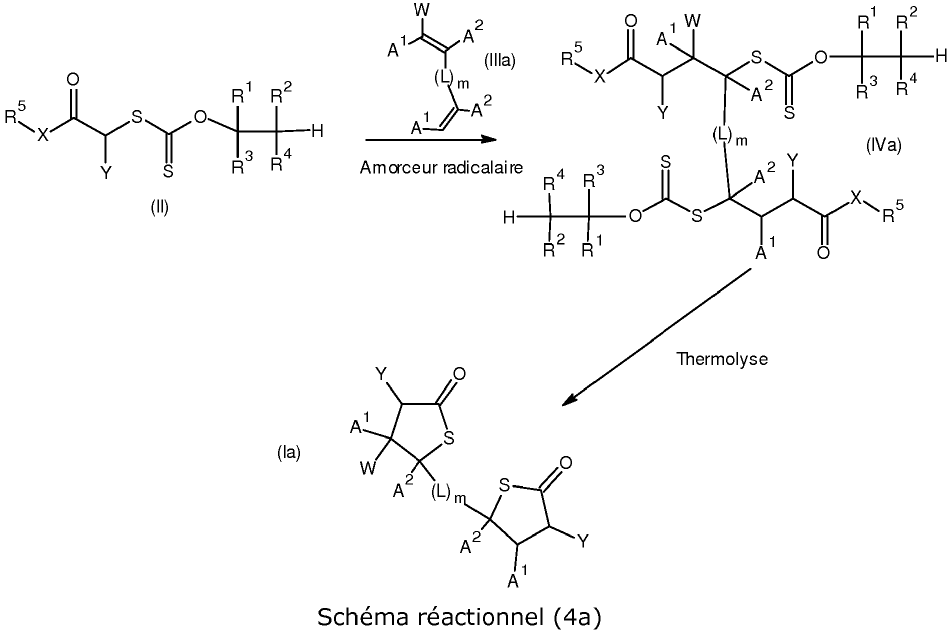 dans lequel
- le monomère comportant une insaturation éthylénique de formule générale (IIIa) correspond à un monomère de formule (III) dans lequel e=0, Z²=C₂H₃ (i.e. CH=CH₂) et W=H et A¹, A², L et m ayant la même signification que dans la formule (III),
- le mono-adduit de formule générale (IVa) correspond à un mono-adduit de formule (IV) dans lequel e=0, Z³ est un groupement de formule (V) tel que défini précédemment, dans lequel W=H et A¹, A², L, m, X, R¹, R², R³, R⁴ et R⁵ ont la même signification que dans la formule (IV).

Selon une autre forme de réalisation particulièrement avantageuse, le procédé de l'invention conduit à la formation d'une di-thiolactone de formule (Ib), dans laquelle A¹, A² et Y ont la même définition que dans la formule (I) m = 0, W et Z¹ = CO, e = 1, T = NR⁶ dans lequel R⁶ a la même signification que celle choisie pour le radical R⁶ de la formule (I), ladite di-thiolactone étant obtenue selon le schéma réactionnel (4b) suivant : 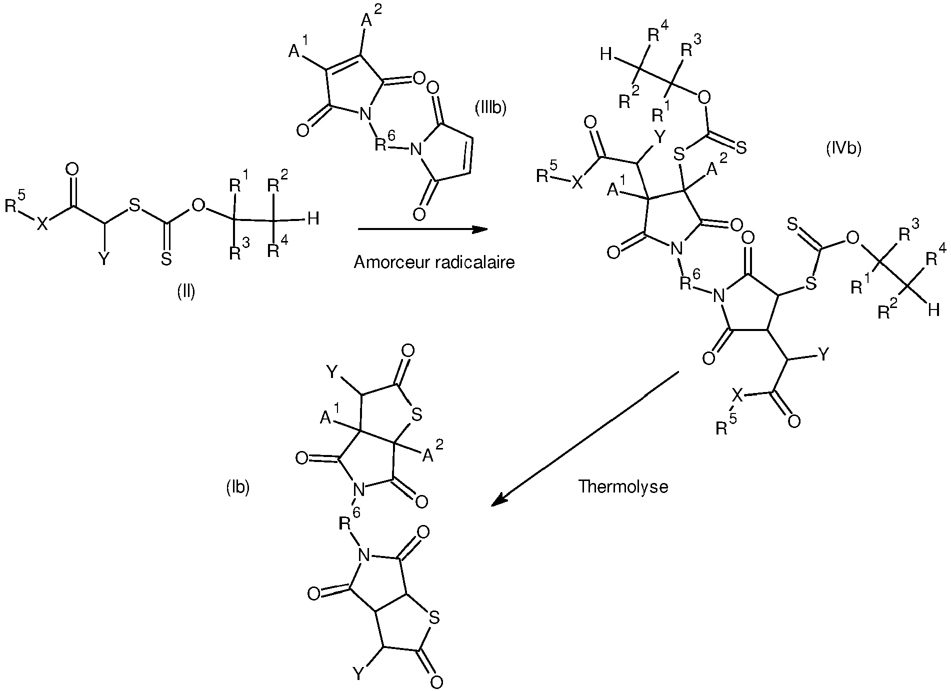 dans lequel le monomère comportant une insaturation éthylénique de formule générale (IIIb) correspond à un monomère de formule (III) dans lequel A¹ et A² ont la même signification que dans la formule (I), m = 0, Z² = W = CO, e = 1 et T = NR⁶ avec R⁶ = a la même signification que celle choisie pour le radical R⁶ de la formule (I), et
- le mono-adduit de formule générale (IVb) correspond à un mono-adduit de formule (IV) dans lequel m = 0, Z³ = W = CO, e = 1 et T = NR⁶ avec R⁶ = un radical alkyle, aryle ou aralkyle substitué par un groupe maléimide, et A¹, A², Y, X, R¹, R², R³, R⁴ et R⁵ ont la même définition que dans la formule (I).

Selon l'invention, les thiolactones de formule (I) dans lesquelles A¹ et A² représentent un atome d'hydrogène sont préférées.

Les radicaux alkyle mentionnés pour R¹, R², R³, R⁴, R⁵, R⁶, Y et les radicaux Z (Z¹, Z² et Z³) peuvent être linéaires, ramifiés, substitués ou non substitués et comporter de 1 à 12 atomes de carbone. Ils sont de préférence choisis parmi les radicaux méthyle, éthyle, *n*-propyle, *iso*-propyle, *n*-butyle, 2-butyle, *iso*-butyle, *tert*-butyle, *n*-pentyle, *iso-*pentyle, *néo*-pentyle, *tert*-pentyle, hexyle, *n*-octyle, *iso*-octyle, 2-éthyl-1-hexyle, 2,2,4-triméthylpentyle, nonyle, décyle, dodécyle et benzyle. Parmi de tels radicaux, on préfère tout particulièrement les radicaux méthyle, éthyle, *n*-propyle, *iso-*propyle et *n*-butyle.

Au sens de la présente invention, un groupe acyle désigne un groupement de formule -C(=O)-D, dans lequel D désigne un atome d'hydrogène ou une chaine hydrocarbonée linéaire ou ramifiée, saturée ou insaturée et comportant de 1 à 12 atomes de carbone. Parmi de tels groupes acyle mentionnés pour R¹, R², R³, R⁴, R⁵, R⁶, Z¹, Z² et Z³, on peut notamment citer les groupements formyle, acétyle, propanoyle et pivaloyle.

Au sens de la présente invention, on entend par groupe aryle, un groupement hydrocarboné aromatique monocyclique ou polycyclique éventuellement mono ou polysubstitué. A titre de radical aryle mentionné pour R¹, R², R³, R⁴, R⁵, R⁶, Z¹, Z² et Z³, on peut en particulier citer les groupements naphtyle, anthranyle, phénantryle, o-tolyle, p-tolyle, xylyle, éthylphényle, mésityle et phényle Parmi de tels groupements, le groupement phényle est particulièrement préféré.

Au sens de la présente invention, le groupe cycloalkyle est un groupement cyclique saturé comportant de 3 à 10 atomes de carbone. Parmi de tels groupes cycloalkyle mentionnés pour R¹, R², R³, R⁴, R⁵ et R⁶ on peut en particulier citer les groupes cyclopropyle, cyclobutyle, cyclopentyle, cyclohexyle et cycloheptyle.

Toujours au sens de la présente invention, un groupe hétérocycloalkyle est un groupement cyclique saturé comportant de 3 à 9 atomes de carbone et au moins un hétéroatome choisi parmi N, O, P, Si et S. Parmi de tels groupes hétérocycloalkyles mentionnés pour R¹, R², R³, R⁴, R⁵ et R⁶ on peut en particulier citer les groupes oxacyclopropanyle, azacyclopropanyle, thiacyclopropanyle, tetrahydrofuranyle, pyrrolidinyle, tetrahydrothiophényle, tetrahydropyranyle, pipéridinyle, pipérazinyle et thiacyclohexane.

Un groupe hétérocycloaryle, au sens de la présente invention, est un groupement aromatique monocyclique ou polycyclique comportant de 5 à 6 atomes de carbone et au moins un hétéroatome choisi parmi N, O, P, Si et S. particulier citer les groupes oxacyclopropanyle, azacyclopropanyle, thiacyclopropanyle, tetrahydrofuranyle, pyrrolidinyle, tetrahydrothiophényle, tetrahydropyranyle, pipéridinyle, pipérazinyle et thiacyclohexane.

Un groupe hétérocycloaryle, au sens de la présente invention, est un groupement aromatique monocyclique ou polycyclique comportant de 5 à 6 atomes de carbone et au moins un hétéroatome choisi parmi N, O, P, Si et S. Parmi de tels groupes hétérocycloaryles mentionnés pour R¹, R², R³, R⁴, R⁵ et R⁶ on peut en particulier citer les groupes furanyle, thiophényle, pyrrolyle, pyridinyle, pyranyle, oxazinyle, thazinyle, pyrimidinyle, pipérazinyle et thiinyle.

Dans un mode de réalisation particulier, R¹, R², R³ et R⁴, identiques ou différents, représentent un atome d'hydrogène ou un groupe alkyle, sous réserve bien sûr, et comme indiqué précédemment, qu'au moins un des groupes R¹ et R³ soit différent d'un atome d'hydrogène.

Selon une forme de réalisation particulièrement préférée de l'invention, R¹ (respectivement R³) est un groupe alkyle, notamment un groupe méthyle, et R³ (respectivement R¹) est un atome d'hydrogène.

De préférence, au moins un des groupes R² et R⁴ est différent d'un atome d'hydrogène.

De préférence encore, R² (respectivement R⁴) est un groupe alkyle, notamment un groupe méthyle, et R⁴ (respectivement R²) est un atome d'hydrogène ou un groupe alkyle, notamment un groupe méthyle.

Enfin, selon la présente invention, on entend par chaîne de polymère tout enchaînement d'unités monomères obtenu par un procédé de polymérisation radicalaire contrôlée par addition-fragmentation réversible (procédé RAFT/MADIX tel que décrit par exemple par Moad, G. et al., Aust. J. Chem., 2012, 65(8), 985-1076) ou transfert d'atome (procédé ATRP de l'expression anglophone « *Atom Transfer Radical Polymerization* » tel que décrit par exemple par Matyjaszewski, K., Chem. Rev., 2001, 101(9), 2921-2990) mis en œuvre de telle sorte que l'unité monomère terminale connectée respectivement à l'atome de soufre du groupement thiocarbonylthio (RAFT/MADIX) ou halogène (Cl, Br) pour l'ATRP soit de type acrylate, par exemple acrylate de méthyle, ou acrylamido comme le N-isopropylacrylamide.

Au sens de la présente invention, dans les composés de formule (I), (III) ou (IIIa) et (IV) ou (IVa), quand e = 0 alors T est absent et W et, respectivement, Z¹, Z² et Z³ ne sont pas reliés.

Le procédé conforme à l'invention est de préférence mis en œuvre pour la préparation de thiolactones substituées de formule (I) telle que définie ci-dessus dans laquelle :
- Z¹ est de préférence un groupe choisi parmi les groupes P(O)(OR⁷)(OR^{7'}), en particulier un groupe diméthylphosphonate (R⁷ = R^{7'} = - CH₃) ou diéthylphosphonate (R⁷ = R^{7'} = -CH₂CH₃) ; CₙF₂ₙ₊₁ dans lequel n est de préférence un nombre entier allant de 1 à 10 ; B(OR¹⁰)₂ ; OR¹¹ ; SiR⁸ₚ(OR⁹)₃₋ₚ ; NR^{15'}(C=O)R¹⁵ dans lequel R^{15'} est un atome d'hydrogène et NR^{16'}(C=O)OR¹⁶ dans lequel R^{16'} est un atome d'hydrogène, et/ou
- Y est de préférence un atome d'hydrogène ou un groupe choisi parmi un radical alkyle, en particulier méthyle et hydroxyméthyle.

Selon une forme de réalisation particulièrement préférée de l'invention, R⁵ est un groupe alkyle, notamment un groupe méthyle.

Le procédé conforme à l'invention est de préférence mis en œuvre pour préparer des thiolactones de formule (I) dans lesquelles e = 0, m = 1 et Z¹ est différent d'un atome d'hydrogène.

Selon une forme de réalisation particulièrement avantageuse, le procédé de l'invention conduit à la formation d'une thiolactone de formule (I) choisie parmi :
- le diméthyl 5-oxo-tetrahydrothiophèn-2-ylphosphonate (TL1) ;
- le diéthyl (4-méthyl-5-oxo-tetrahydrothiophèn-2-yl) méthylphosphonate (TL2) ;
- le diéthyl (5-oxo-tetrahydrothiophèn-2-yl)méthylphosphonate (TL3) ;
- la 3-méthyl-5-pentyl-dihydrothiophèn-2(3H)-one (TL4) ;
- la 5-pentyl-dihydrothiophèn-2(3H)-one (TL5) ;
- la 3-méthyl-5-(perfluorooctyl)dihydrothiophèn-2(3H)-one (TL6),
- la 3-méthyl-5-phényldihydro-2H-thiéno[2,3-c]pyrrole-2,4,6(3H,5H)-trione (TL7),
- la 3-méthyl-5-(perfluorobutyl)dihydrothiophèn-2(3H)-one (TL8),
- l'acide (4-méthyl-5-oxo-tetrahydrothiophèn-2-yl)phosphonique (TL9),
- l'acide ((4-méthyl-5-oxotetrahydrothiophèn-2-yl)méthyl)phosphonique (TL10),
- l'acide (5-oxo-tetrahydrothiophèn-2-yl)méthylphosphonique (TL11),
- la 3-méthyl-5-(triméthoxysilyl)dihydrothiophèn-2(3H)-one (TL12),
- la 5-(triméthoxysilyl)dihydrothiophèn-2(3H)-one (TL13),
- le tert-butyl-N-(4-méthyl-5-oxo-tetrahydrothiophèn-2-yl)carbamate (TL14),
- le tert-butyl (5-oxotetrahydrothiophèn-2-yl)carbamate (TL15),
- la 3-méthyl-5-(oxiran-2-ylméthoxy)dihydrothiophèn-2(2H)-one (TL16),
- la 5-((oxiran-2-yloxy)méthyl)dihydrothiophèn-2(3H)-one (TL17),
- la 3-méthyl-5-(4,4,5,5-tetraméthyl-1,3,2-dioxaborolan-2-yl)dihydrothiophèn-2(3H)-one (TL18),
- l'acide (5-oxo-tetrahydrothiophèn-2-yl) phosphonique (TL19),
- la 5-(4,4,5,5-tetraméthyl-1,3,2-dioxaborolan-2-yl)dihydrothiophèn-2(3H)-one (TL20),
- la 5-(perfluorooctyl)dihydrothiophèn-2(3H)-one (TL21),
- la 5-(perfluorobutyl)dihydrothiophèn-2(3H)-one (TL22),
- la dihydro-5-(3-(tétrahydro-4-méthyl-5-oxothiophén-2-yl)propyl)-3-méthylthiophén-2(3H)-one (TL23),
- la 5-(9-hydroxynonyl)3-méthyldihydrothiophèn-2(3H)-one (TL24),
- la 5-(9-bromononyl)3-méthyldihydrothiophèn-2(3H)-one (TL25),
- la 5,5'-(éthane-1,2-diyl)bis(3-méthyldihydrothiophèn-2(3H)-one (TL26), et
- la 5,5'-(hexane-1,6-diyl)bis(3-méthyldihydrothiophèn-2(3H)-one (TL27) .

Lorsqu'ils ne sont pas commerciaux, les xanthates de formule (II) peuvent être obtenus selon un procédé analogue à celui utilisé dans la demande internationale WO 2004/024681 et consistant :
a) dans une première étape à faire réagir, dans un solvant organique, un alcool de formule (VI) suivante : 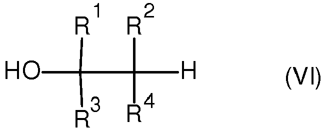 dans laquelle les radicaux R¹, R², R³ et R⁴ ont la même signification que dans les composés de formule (II) ci-dessus
   avec du disulfure de carbone en présence d'une base pour obtenir un sel de formule (VII) suivante : 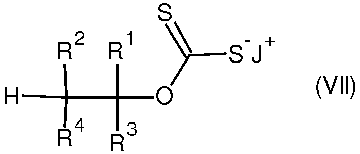 dans laquelle les radicaux R¹, R², R³ et R⁴ ont la même signification que dans le xanthate de formule (II) ci-dessus et J⁺ est un cation choisi parmi les métaux alcalins tels qu'un cation K⁺ ou Na⁺, puis
b) dans une deuxième étape, à faire réagir le composé de formule (VII) obtenu ci-dessus à l'étape précédente avec un composé de formule (VIII) suivante : 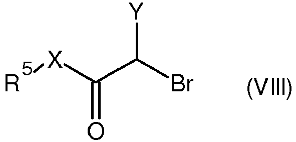 dans laquelle R⁵ et X ont la même signification que dans le xanthate de formule (II) ci-dessus, pour obtenir un xanthate de formule (II) correspondant.

La première étape a) de préparation d'un composé de formule (VII) est de préférence réalisée à température ambiante, dans un solvant organique tel que le tetrahydrofurane et en utilisant une base forte, de préférence la potasse. La durée de l'étape a) est généralement de 20 à 24 heures environ.

La deuxième étape b) de préparation d'un xanthate de formule (II) est de préférence réalisée dans un solvant organique tel que l'acétone et dans un bain de glace, la réaction d'addition du composé de formule (VIII) étant fortement exothermique. Une fois l'addition du composé de formule (VIII) terminée, la réaction est de préférence conduite à température ambiante pendant une durée de 2 à 4 heures environ. Lorsque la réaction est terminée, le xanthate de formule (II) ainsi obtenu est filtré, puis le filtrat est de préférence concentré sous vide. Le xanthate de formule (II) peut ensuite être engagé dans le procédé conforme à la présente invention sans purification supplémentaire.

Selon un premier mode de réalisation de l'invention, le xanthate de formule (II) est le O-1,2-diméthylpropyl S-méthoxycarbonylméthyl xanthate (**XA1**) ou le O-1,2-diméthylpropyl S-(1-méthoxycarbonyl)éthyl xanthate **(XA2).**

Selon un deuxième mode de réalisation de l'invention, le xanthate de formule (II) est tel que défini dans l'invention [i.e. avec R¹, R², R³, R⁴, Y, X et R⁵ tels que définis dans l'invention], en excluant les xanthates du premier mode de réalisation ci-dessus.

L'étape 1) de préparation du mono-adduit de formule (IV) du procédé conforme à l'invention peut être réalisée sans solvant, dans l'eau ou dans un solvant organique. Elle est de préférence réalisée dans un solvant organique ou dans l'eau, et encore plus préférentiellement dans un solvant organique. Le solvant organique utilisable lors de cette étape 1) est alors de préférence choisi parmi le toluène, le tetrahydrofurane (THF), l'acétate d'éthyle et le 1,4-dioxane. Parmi de tels solvants organiques, le toluène est particulièrement préféré.

Au sens de la présente invention, on entend par amorceur radicalaire, une espèce chimique capable de former des radicaux libres c'est-à-dire une espèce chimique possédant un ou plusieurs électrons non appariés sur sa couche externe.

Selon le procédé conforme à l'invention, l'amorceur radicalaire utilisé lors de l'étape 1) est de préférence choisi parmi les peroxydes organiques, les dérivés azoïques, les couples oxydo-réducteurs générateurs de radicaux et les systèmes redox.

Parmi les peroxydes organiques, on peut en particulier mentionner le peroxyde de dilauroyle (LPO), le peroxyacétate t-butyle, le peroxybenzoate de t-butyle, le peroxyoctoate de t-butyle, le peroxydodécanoate de t-butyle, le peroxyisobutyrate de t-butyle, le peroxypyvalate de t-amyle, le peroxypyvalate de t-butyle, le peroxydicarbonate de di-isopropyle, le peroxydicarbonate de dicyclohexyle, le peroxyde de dicumyle, le peroxyde de dibenzoyle, le peroxydisulfate de potassium, le peroxydisulfate de sodium et le peroxydisulfate d'ammonium. Parmi ces peroxydes organiques, le LPO est particulièrement préféré.

Parmi les dérivés azoïques, on peut en particulier mentionner le 2,2'-azobis(isobutyronitrile), le 2,2'-azobis(2-cyano-2-butane), le diméthyl-2,2'-azobisdiméthylisobutyrate, le 4,4'-azobis-(acide 4-cyanopentanoique), le 1,1'-azobis-(cyclohexanecarbonitrile), le 2-(t-butylazo)-2-cyanopropane, le 2,2'-azobis-[2-méthyl-N(1,1)-bis (hydroxyméthyl)-2-hydroxyéthyl] propanamide, le 2,2'-azobis-[2-méthyl-N-hydroxyéthyl]-propanamide, le dichlorhydrate de 2,2'-azobis-(N,N'-diméthylèneisobutyramidine), le dichlorhydrate de 2,2'-azobis-(2-amidinopropane), le 2,2'-azobis-(N,N'-diméthylène isobutyramine), le 2,2'-azobis-(2-méthyl-N-[1,1bis-(hydroxyméthyl)-2-hydroxyéthyl]propionamide), le 2,2'-azobis-(2-méthyl-N-[1,1-bis-(hydroxyméthyl)propionamide], le 2,2'-azobis-[2-méthyl-N-(2-hydroxyéthyl)propionamide], le 2,2'-azobis-(isobutyramide)dihydrate, le 2,2'-azobis-(2,2,4-triméthylpentane) et le 2,2'-azobis-(2-méthylpropane).

Les systèmes redox sont par exemple choisis parmi les systèmes comportant des combinaisons telles que :
- les mélanges de peroxyde d'hydrogène, d'un alkyle, d'un perester, d'un percarbonate et composés similaires et d'un sel de fer, d'un sel de titane, de formaldéhyde sulfoxylate de zinc ou de formaldéhyde sulfoxylate de sodium, et d'un sucre réducteur,
- les mélanges d'un persulfate, perborate ou perchlorate de métal alcalin ou d'ammonium avec un bisulfite de métal alcalin, tel que le métabisulfite de sodium, et un sucre réducteur, et
- les mélanges d'un persulfate de métal alcalin avec un acide arylphosphinique, tel que l'acide benzène phosphonique et autres similaires, et un sucre réducteur.

Parmi de tels systèmes redox, on préfère tout particulièrement l'association de persulfate d'ammonium et de formaldéhyde sulfoxylate de sodium.

Par ailleurs, lors de l'étape 1), l'amorceur radicalaire peut être rajouté au milieu réactionnel en une fois ou en plusieurs fois, c'est-à-dire par portions. Selon une forme de réalisation préférée du procédé de l'invention, l'amorceur radicalaire est ajouté au milieu réactionnel par portions.

Selon un premier mode de réalisation de l'invention, le mono-adduit de formule (IV) tel qu'obtenu à l'issue de l'étape 1) est choisi parmi l'O-1,2-diméthylpropyl S-(1-(diméthylphosphoryl)-3-(méthoxycarbonyl))propyl xanthate (**XA1VP**), l'O-1,2-diméthylpropyl S-(1-(diéthylphosphoryl)-4-(méthoxycarbonyl))pent-2-yl xanthate **(XA2AP),** l'O-1,2-diméthylpropyl S-(1-(diéthylphosphoryl)-4-(méthoxycarbonyl))but-2-yl xanthate **(XA3AP),** l'O-1,2-diméthylpropyl S-2-(méthoxycarbonyl)non-4-yl xanthate **(XA4H),** l'O-1,2-diméthylpropyl S-1-(méthoxycarbonyl)oct-3-yl xanthate **(XA5H),** le méthyl 5,5,6,6,7,7,8,8,9,9,10,10,11,11,12,12,12-heptadecafluoro-2-méthyl-4-((((3-méthylbutan-2-yl)oxy)carbonothioyl)thio)dodécanoate **(XA6DF),** le mono-adduit de l'exemple 7 ci-après **(XA7MAL)** et le méthyl 5,5,6,6,7,7,8,8,8-nonafluoro-2-méthyl-4-((((3-méthylbutan-2-yl)oxy)carbonothioyl)thio)octanoate **(XA8HF).**

Selon un deuxième mode de réalisation de l'invention, le mono-adduit de formule (IV) est tel que défini dans l'invention [i.e. avec R¹, R², R³, R⁴, Y, X, R⁵, Z³, W, T, e, L et m tels que définis dans l'invention], en excluant les mono-adduits du premier mode de réalisation ci-dessus.

Les monomères de formule (III) sont de préférence choisis parmi les composés qui sont des monomères peu ou pas polymérisables dans les conditions de température et de pression utilisés lors de l'étape 1) du procédé conforme à l'invention, c'est-à-dire qui conduisent à un mono-adduit de formule (IV) sans présence notable de diadduit, triadduit, etc... Parmi de tels monomères de formule (III), on peut en particulier mentionner les alcènes et les composés allyliques.

Parmi les alcènes utilisables à titre de monomère de formule (III), on peut en particulier mentionner l'éthylène, le propylène le 1-butène, le 1-pentène, le 1-hexène, le 1-heptène, le 1-octène, le 1-nonène, le 1-décène, le perfluorohexyl éthylène et le perfluorooctyl éthylène.

Parmi les composés allyliques utilisables à titre de monomère de formule (III), on peut en particulier mentionner l'alcool allylique, la N-allyl benzamide, l'éthyl N-allyl carbamate, le terbutyl N-allyl carbamate, le 2-allyl-4,4,5,5-tétraméthyl-1,3,2-dioxaborolane, le 2-allyl-6-méthyl-1,3,6,2-dioxazaborocane-4,8-dione, l'acide allylboronique, le phosphonate de diéthyl allyle, le dichlorure d'allyle phosphonique, le diméthyl allylphosphonate, le cyanure d'allyle, l'isothiocyanate d'allyle, le glycidyléther d'allyle, le benzyléther d'allyle, phényléther d'allyle, le butyléther d'allyle, l'éthyléther d'allyle, la méthylsulfone d'allyle, la phénylsulfone d'allyle, le chlorure d'allyle, le bromure d'allyle et le fluorure d'allyle.

Les monomères de formule (III) peuvent également être choisis parmi des composés polymérisables, en particulier parmi des composés vinyliques styréniques, l'acide acrylique et les acrylates, et l'acrylamide et ses dérivés. Dans ce cas, la synthèse n'est pas sélective mais l'obtention majoritaire du mono-adduit pourra être favorisée en utilisant une quantité de monomère de formule (III) inférieure à celle du xanthate de formule (II) de façon à limiter au maximum le risque de polymérisation du monomère de formule (III). De plus, le mono-adduit de formule (IV) peut être isolé par les techniques classiques et à la portée de l'homme du métier.

Parmi les composés vinyliques utilisables à titre de monomère de formule (III), on peut en particulier mentionner le phosphonate de diméthylvinyle, le phosphonate de diéthylvinyle, l'acide vinylphosphonique, les monomères N-vinyliques tels que la N-vinylpyrrolidone, la N-vinylcaprolactame et la N-méthyl-N-vinylacétamide, les vinylsulfones telles que la méthyl vinylsulfone et la phényl vinylsulfone, les vinylsilanes tels que le vinyltriméthylsilane et le vinyltriméthoxysilane, et les esters vinyliques. Tels que l'acétate de vinyle, le pivalate de vinyle, le butyrate de vinyle, le valérate de vinyle, le propionate de vinyle, le benzoate de vinyle, le néodécanoate de vinyle et le trifluoroacétate de vinyle.

Parmi les composés styréniques utilisables à titre de monomère de formule (III), on peut en particulier mentionner le styrène, l'acide vinyl benzoïque, le 3-chloro styrène, le 2-méthylstyrène, le p-*t*-butyl-styrène, le p-méthoxystyrène, le p-méthylstyrène, le p-chlorométhylstyrène et l'acide vinylphénylboronique.

Parmi les acrylates, on peut en particulier mentionner l'acrylate de méthyle, l'acrylate de n-butyle, l'acrylate de *t*-butyle et l'acrylate de 2-hydroxyethyle.

Parmi les dérivés d'acrylamide, on peut en particulier mentionner la N-isopropyl acrylamide, la N,N-diméthylacrylamide, la N-*t*-butylacrylamide et la N-octyl acrylamide.

Les monomères de formules (III) sont généralement disponibles dans le commerce. Lorsqu'ils ne sont pas disponibles dans le commerce, ils peuvent être obtenus facilement par des voies de synthèse bien connues de l'homme du métier.

L'étape 1) du procédé conforme à l'invention est généralement réalisée à une température variant de 10 à 140°C environ, et de préférence de 40 à 110°C environ, et encore plus préférentiellement entre 65 et 90°C environ.

La durée de ladite étape 1) varie généralement de 3 à 48 heures environ, et encore plus préférentiellement de 4 à 24 heures environ.

Selon une forme de réalisation particulière et préférée de l'invention, le mono-adduit de formule (IV) obtenu à l'issue de l'étape 1) est purifié, par exemple par chromatographie sur gel de silice, avant d'être engagé dans la deuxième étape de thermolyse.

L'étape 2) de thermolyse du procédé conforme à la présente invention peut être réalisée avec ou sans solvant. Selon une forme de réalisation préférée de l'invention, l'étape de thermolyse est réalisée sans solvant. La température de thermolyse est généralement comprise entre 40 et 210°C, de préférence entre 100 et 200°C et plus particulièrement entre 160 et 190°C.

L'étape de thermolyse est généralement effectuée à une température suffisante pour décomposer le mono-adduit de formule (IV).

Dans la présente invention, l'expression « thermolyse » signifie une décomposition thermique. C'est une réaction de décomposition chimique causée par la chaleur. Dans le cas présent, l'action de la chaleur conduit à la décomposition du mono-adduit de formule (IV), permettant la formation de la thiolactone de formule (I).

En d'autres termes, l'étape 2) du procédé de l'invention ne met pas en œuvre d'autres réactifs que le mono-adduit de formule (IV) issu de l'étape 1). Seule l'action de la chaleur permet de conduire aux thiolactones de formule (I).

De façon surprenante, le mono-adduit de formule (IV) obtenu à l'étape 1) a une structure chimique appropriée, notamment de par la définition des groupes R¹, R², R³, R⁴, R⁵, L (si m = 1), Y, X, W, T (si e = 1) et Z³, pour permettre la formation d'une thiolactone substituée de formule (I) par thermolyse. En d'autres termes, la cyclisation en thiolactone (I) est favorisée.

Lorsque l'étape de thermolyse est réalisée dans un solvant, alors ledit solvant est de préférence choisi parmi les solvants de haut point d'ébullition (c'est-à-dire ayant un point d'ébullition supérieur ou égale à la température de thermolyse), tels que par exemple le 1,2-dichlorobenzène.

Par ailleurs, l'étape de thermolyse peut être effectuée à pression atmosphérique ou sous vide, notamment dans ce dernier cas, pour éliminer les sous-produits volatiles éventuellement formés en cours de réaction.

Selon un mode de réalisation particulier, l'étape de thermolyse est effectuée dans un contenant fermé (e.g. tube de schlenk), et de préférence sous vide.

Selon une forme particulière et préférée de l'invention, l'étape de thermolyse est réalisée sans solvant et sous vide.

A la fin de l'étape 2) de thermolyse, la thiolactone de formule (I) est de préférence purifiée, par exemple par chromatographie sur colonne de silice.

Certaines des thiolactones substituées de formule (I) directement obtenues en mettant en œuvre le procédé de préparation conforme au premier objet de l'invention sont nouvelles en soi et constituent à ce titre le deuxième objet de l'invention.

La présente invention a donc également pour objet des thiolactones substituées de formule (I') suivante : 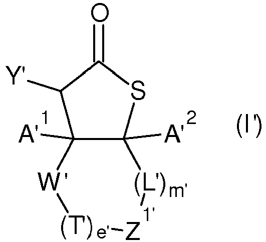 dans laquelle :
A'¹, A'², Y', Z¹', L', m', T' et e' peuvent respectivement prendre les mêmes significations que celles définies précédemment pour A¹, A², Y, Z¹, L, m, T et e pour les thiolactones de formule (I), sous réserve que :
   - lorsque e' = 0 et que W' = H et que m' = 0 et que Y' = hydrogène, alors Z^{1'} est différent d'un atome d'hydrogène, d'une chaine alkyle linéaire ou d'un cycle phényle ; et
   - lorsque e' = 0 et que W' = H et que m' = 0 et que Y' est un substituant possédant un atome d'azote directement lié au cycle thiolactone, alors Z¹' est différent d'un atome d'hydrogène,
dans la mesure où de telles thiolactones sont décrites dans l'article de Espeel P. et al., European Polymer Journal, 2015, 62, 247-272.

Parmi les thiolactones substituées de formule (I') ci-dessus, on peut notamment citer :
- le diméthyl 5-oxo-tetrahydrothiophèn-2-ylphosphonate (TL1),
- le diéthyl (4-méthyl-5-oxo-tetrahydrothiophèn-2-yl) méthylphosphonate (TL2),
- le diéthyl (5-oxo-tetrahydrothiophèn-2-yl)méthylphosphonate (TL3),
- la 3-méthyl-5-pentyl-dihydrothiophèn-2(3H)-one (TL4),
- la 5-pentyl-dihydrothiophèn-2(3H)-one (TL5),
- la 3-méthyl-5-(perfluorooctyl)dihydrothiophèn-2(3H)-one (TL6),
- la 3-méthyl-5-phényldihydro-2H-thiéno[2,3-c]pyrrole-2,4,6(3H,5H)-trione (TL7),
- la 3-méthyl-5-(perfluorobutyl)dihydrothiophèn-2(3H)-one, (TL8),
- l'acide (4-méthyl-5-oxo-tetrahydrothiophèn-2-yl)phosphonique (TL9),
- l'acide ((4-méthyl-5-oxotetrahydrothiophèn-2-yl)méthyl)phosphonique (TL10),
- l'acide (5-oxo-tetrahydrothiophèn-2-yl)méthylphosphonique (TL11),
- la 3-méthyl-5-(triméthoxysilyl)dihydrothiophèn-2(3H)-one (TL12),
- la 5-(triméthoxysilyl)dihydrothiophèn-2(3H)-one (TL13),
- le tert-butyl-N-(4-méthyl-5-oxo-tetrahydrothiophèn-2-yl)carbamate (TL14),
- le tert-butyl (5-oxotetrahydrothiophèn-2-yl)carbamate (TL15),
- la 3-méthyl-5-(oxiran-2-ylméthoxy)dihydrothiophèn-2(2H)-one (TL16),
- la 5-((oxiran-2-yloxy)méthyl)dihydrothiophèn-2(3H)-one (TL17),
- la 3-méthyl-5-(4,4,5,5-tetraméthyl-1,3,2-dioxaborolan-2-yl)dihydrothiophèn-2(3H)-one (TL18),
- l'acide (5-oxo-tetrahydrothiophèn-2-yl) phosphonique (TL19),
- la 5-(4,4,5,5-tetraméthyl-1,3,2-dioxaborolan-2-yl)dihydrothiophèn-2(3H)-one (TL20),
- la 5-(perfluorooctyl)dihydrothiophèn-2(3H)-one (TL21),
- la 5-(perfluorobutyl)dihydrothiophèn-2(3H)-one (TL22),
- la dihydro-5-(3-(tétrahydro-4-méthyl-5-oxothiophén-2-yl)propyl)-3-méthylthiophén-2(3H)-one (TL23),
- la 5-(9-hydroxynonyl)3-méthyldihydrothiophen-2(3H)-one (TL24),
- la 5-(9-bromononyl)3-méthyldihydrothiophen-2(3H)-one (TL25),
- la 5,5'-(éthane-1,2-diyl)bis(3-méthyldihydrothiophen-2(3H)-one (TL26), et
- la 5,5'-(hexane-1,6-diyl)bis(3-méthyldihydrothiophen-2(3H)-one (TL26).

Les thiolactones substituées de formule (I) susceptibles d'être obtenues par la mise en œuvre du procédé conforme à la présente invention, et en particulier, les thiolactones de formule (I') conformes au deuxième objet de la présente invention peuvent avantageusement être utilisées pour la synthèse de polymères ou pour la fonctionnalisation de surfaces ou de polymères.

Ainsi la présente invention a donc également pour troisième objet l'utilisation d'au moins une thiolactone substituée de formule (I) obtenue selon le procédé tel que défini selon le premier objet de l'invention, en particulier d'au moins une thiolactone substituée de formule (I'), pour la synthèse de polymères ou pour la fonctionnalisation de surfaces planes de type métal, verre, céramique, particule, ou de polymères.

En ce qui concerne la préparation de polymères, les thiolactones de formule (I), et en particulier de formule (I') peuvent être engagées dans une réaction de polymérisation comprenant au moins une étape de réaction d'une thiolactone de formule (I), en particulier de formule (I') avec un composé nucléophile permettant d'ouvrir le cycle de la thiolactone et d'obtenir un thiol qui peut ensuite être engagé dans un processus de polymérisation par addition ou condensation avec par exemple un monomère de type diacrylate tel que décrit dans la référence par Lei Yu et al, Polym. Chem., 2015, 6, 1527-1532.

En ce qui concerne la fonctionnalisation de surface ou de polymères, il est ainsi possible :
- selon une première forme de réalisation, d'effectuer le greffage de thiolactones substituées de formule (I) (respectivement de formule (I')), sur une surface solide ou sur un polymère à l'état liquide, ladite surface ou ledit polymère comportant des fonctions chimiques aptes à réagir avec le groupement Z¹ ou Y (respectivement Z¹' ou Y') des thiolactones de formule (I), respectivement (I'), afin de former une liaison covalente, ou des interactions fortes de type liaison hydrogène. A titre d'exemple, il est ainsi possible de greffer une thiolactone comportant un groupement phosphonate tel que le groupement acide phosphonique de la thiolactone TL8 à titre de groupement Z¹, sur une surface métallique. Selon cette première forme de réalisation, après fonctionnalisation, l'intégrité du cycle thiolactone est préservée.
- selon une deuxième forme de réalisation, d'effectuer le greffage de la thiolactone substituée par ouverture du cycle thiolactone sur une surface solide ou sur un polymère à l'état liquide et mise en réaction avec toute substance réactive avec les thiols tels les acrylates ou les halogénures d'alkyle par exemple.

En fonction de la nature des groupes Y ou Z¹ (respectivement Y' et Z^{1'}), il devient alors possible de conférer à un matériau ou à un polymère les propriétés correspondant au type de groupements Y ou Z¹ (respectivement Y' ou Z^{1'}) greffés, par exemple des propriétés antiadhésives lorsque que les groupes Y ou Z¹ (respectivement Y' ou Z^{1'}) sont des groupes perfluorés. Il est aussi possible d'introduire une fonction supplémentaire lors de la mise en réaction du thiol obtenu par ouverture de la thiolactone avec un composé fonctionnel réactif avec les thiols.

La présente invention est illustrée par les exemples de réalisation suivants, auxquels elle n'est cependant pas limitée.

### EXEMPLES

### Exemple 1 : Synthèse du diméthyl 5-oxo-tetrahydrothiophèn-2-ylphosphonate (TL1) selon le procédé conforme à l'invention

Dans cet exemple, on a préparé la thiolactone de formule suivante **(TL1)** :

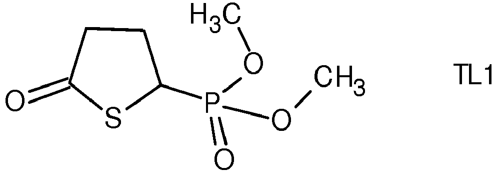

### 1) Première étape : Préparation du O-1,2-diméthylpropyl S-méthoxycarbonylméthyl xanthate (Xanthate de formule (II) ; (XA1))

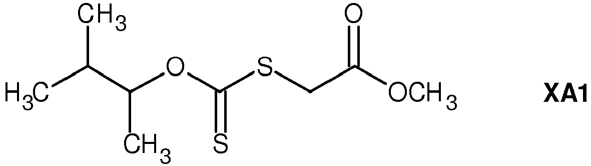

### 1.1) Sous-étape 1: Préparation de l'O-(1,2-diméthylpropyl)xanthogénate de potassium (XA0)

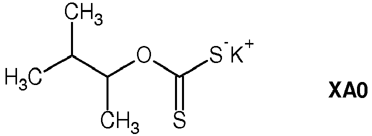

On a placé en suspension dans 500 mL de tetrahydrofurane (THF, Sigma-Aldrich), 100 g (1,13 mol) de 3-méthylbutan-2-ol (Alfa Aesar), 63,65 g de potasse (KOH, Sigma-Aldrich) et du disulfure de carbone (CS₂, Sigma-Aldrich) à température ambiante pendant 24 heures.

Après totale dissolution de KOH, l'émulsion jaune a été concentrée sous pression réduite, puis triturée avec du pentane (Sigma-Aldrich) et finalement filtrée pour obtenir 185 g du produit attendu **XA0** sous la forme d'un solide jaune (185 g, rendement 80 %).

RMN ¹H (300,13 MHz, D₂O, 298K) δ 5,31 (p, *³J_{H,H}* = 6,4 Hz, 1H, (CH₃)₂CHCH(O)CH₃) ; 2,07-1,84 (m, 1H, (CH₃)₂CHCH(O)CH₃) ; 1,27 (d, *³J_{H,H}* = 6,4 Hz, 3H, (CH₃)₂CHCH(O)CH₃) ; 0,96 (d, *³J_{H,H}* = 6,9 Hz, 6H, (CH₃)₂CHCH(O)CH₃).

RMN ¹³C{¹H} (75,47 MHz, D₂O, 298K) δ 232,6 (C=S) ; 86,3 ((CH₃)₂CHCH(O)CH₃) ; 32,8 ((CH₃)₂CHCH(O)CH₃) ; 17,7 ((CH₃)₂CHCH(O)CH₃) ; 17,6 ((CH₃)₂CHCH(O)CH₃) ; 15,8 ((CH₃)₂CHCH(O)CH₃) ppm.

### 1.2) Sous-étape 2: Préparation du O-1,2-diméthylpropyl S-méthoxycarbonylméthyl xanthate (XA1)

0,21 mole (32,12 g) de 2-bromoacétate de méthyle (Sigma-Aldrich) a été ajouté à une suspension de 40,5 g (0,2 mol) du composé **XA0** obtenu ci-dessus à l'étape 1.1) précédente dans 200 ml d'acétone (Sigma-Aldrich), dans un bain de glace. Une fois l'ajout terminé, le milieu réactionnel a été agité à température ambiante pendant 3 heures puis filtré. Le filtrat a été concentré sous vide afin d'obtenir le produit attendu **XA1** sous la forme d'une huile jaune (42 g, rendement 89 %) qui sera utilisé dans l'étape suivante sans purification.

### 2) Deuxième étape : Préparation de l'O-1,2-diméthylpropyl S-(1-(diméthylphosphoryl)-3-(méthoxycarbonyl))propyl xanthate (XA1VP : mono-adduit de formule (IV))

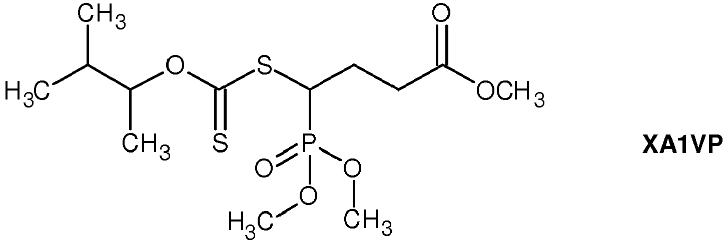

14,2 g (60 mmol) du xanthate **XA1** obtenu ci-dessus à l'étape précédente, 2,72 g (20 mmol) et diméthylvinylphosphonate et 1,2 g (3 mmol) de peroxyde de dilauroyle (LPO : amorceur radicalaire) ont été mélangés dans un tube de Schlenk. Le mélange a ensuite été dégazé par 3 congélations sous vide. Après 5 heures de chauffage à une température de 90°C, le brut réactionnel a été purifié par chromatographie sur silice (éluant acétate d'éthyle/éther de pétrole (95:5, v:v) pour récupérer le xanthate **XA1** n'ayant pas réagi, et avec un éluant constitué d'un mélange acétate d'éthyle/dichlorométhane (1:4, v:v) pour récupérer le mono-adduit **XA1VP** (3,13 g, rendement 42%, huile jaune).
RMN ¹H (300,13 MHz, CDCl₃, 298K) δ 5,53-5,33 (m, 1H, (CH₃)₂CHCH(O)CH₃) ; 4,31-4,11 (m, 1H, CHP) ; 3,75-3,65 (m, 6H, O=P(OCH₃)₂) ; 3,59 (s, 3H, CO₂CH₃) ; 2,56-2,44 (m, 2H, CH₂CH₂CO₂CH₃) ; 2,43-1,87 (m, 2H, CH₂CH₂CO₂CH₃) ; 2,02-1,87 (m, 1H, (CH₃)₂CHCH(O)CH₃) ; 1,25-1,21 (m, 3H, (CH₃)₂CHCH(O)CH₃) ; 0,89-0,86 (m, 6H, (CH₃)₂CHCH(O)CH₃).
RMN ¹³C{¹H} (75,47 MHz, CDCl₃, 298K) δ 211,8-211,5 (Ç=S) ; 175,7 (CO₂CH₃) ; 87,3 ; 87.2 ((CH₃)₂CHCH(O)CH₃) ; 53,9-53,4 (O=P(OCH₃)₂) ; 51,7 (CO₂CH₃) ; 44,6-42,5 (CHP) ; 32,7 ((CH₃)₂CHCH(O)CH₃) ; 31,0-30,8 (CH₂CH₂CO₂CH₃) ; 25,1-24,9 (CH₂CH₂CO₂CH₃) ; 18,0-17,8 ((CH₃)₂CHCH(O)CH₃) ; 15,7 ; 15,6 ((CH₃)₂CHCH(O)CH₃).
RMN ³¹P{¹H} (121,49 MHz, CDCl₃, 298K) δ 26,3 ; 26,2 ppm.
IR : 1738,5 cm⁻¹ (C=O), 1035,1 cm⁻¹ (C=S).
Masse molaire : IC(CH4), MH⁺ :
Trouvé : 373,0916 g/mol
Calculé : 373,0908 g/mol.

### 3) Troisième étape : Préparation du diméthyl 5-oxo-tetrahydrothiophèn-2-ylphosphonate (TL1)

3,70 g (10 mmoles) de **XA1VP** obtenu ci-dessus à l'étape précédente ont été placés dans un tube de Schlenk fermé sous vide et portés à une température de 190°C pendant 15 minutes. Le mélange réactionnel a ensuite été refroidi à température ambiante et les composés volatils formés ont été éliminés sous vide. La thiolactone **TL1** ainsi obtenue sous la forme d'une huile incolore (1,2 g, rendement 57 %) a ensuite été purifiée sur une colonne chromatographique de silice (éluant acétate d'éthyle/dichlorométhane : 1:4 (v:v)).
RMN ¹H (300,13 MHz, CDCl₃, 298K) δ 3,87-3,76 (m, 1H, CHP) ; 3,72-3,57 (m, 6H, O=P(OCH₃)₂) ; 2,63-2,33 (m, 2H, CH₂CH₂CHP) ; 2,32-2,17 (m, 2H, CH₂CH₂CHP).
RMN ¹³C{¹H} (75,47 MHz, CDCl₃, 298K) δ 205,7-205,6 (C=O) ; 53,2-53,1 (O=P(OCH₃)₂) ; 42,3-40,2 (CHP) ; 39,6-39,5 (CH₂CH₂CHP) ; 25,40-25,35 (CH₂CH₂CHP).
RMN ³¹P{¹H} (121,49 MHz, CDCl₃, 298K) δ 25,7 ppm.
IR : 1713,9 cm⁻¹ (C=O).
Masse molaire : IC(CH4), MH⁺
Trouvé : 211,0198 g/mol
Calculé : 211,0194 g/mol.

### Exemple 2 : Synthèse du diéthyl (4-méthyl-5-oxo-tetrahydrothiophèn-2-yl)méthylphosphonate (TL2) selon le procédé conforme à l'invention

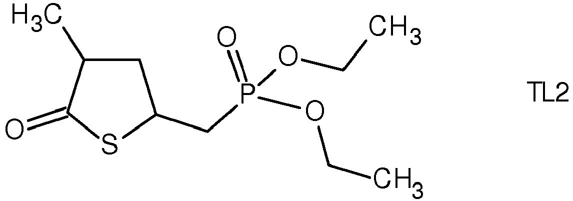

### 1) Première étape : Préparation du O-1,2-diméthylpropyl S-(1-(diéthylphosphoryl)-4-(méthoxycarbonyl))pent-2-yl xanthate (mono-adduit de formule (IV) : XA2AP)

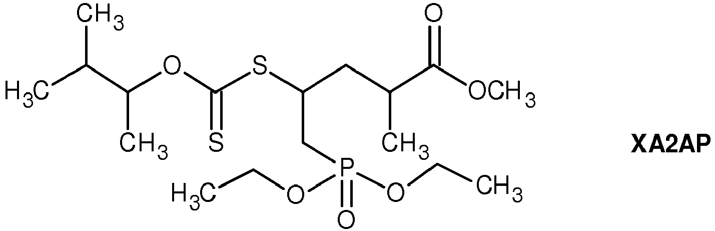

### 1.1) Sous-étape 1: Préparation de l'O-1,2-diméthylpropyl S-(1-méthoxycarbonyl)éthyl xanthate (XA2).

0,21 mole (35,07 g) de 2-bromopropionate de méthyle (Sigma-Aldrich) a été ajouté à une suspension de 40,5 g (0,2 mol) du composé **XA0** obtenu ci-dessus à l'étape 1.1) de l'exemple 1, dans 200 ml d'acétone (Sigma-Aldrich), dans un bain de glace. Une fois l'ajout terminé, le milieu réactionnel a été agité à température ambiante pendant 3 heures puis filtré. Le filtrat a été concentré sous vide afin d'obtenir le produit attendu **XA2** sous la forme d'une huile jaune (43 g, rendement 86 %) qui sera utilisé dans l'étape suivante sans purification.
RMN ¹H (300,13 MHz, CDCl₃, 298K) δ 5,51 (p, *³J_{H,H}* = 6,3 Hz, 1H, (CH₃)₂CHCH(O)CH₃) ; 4,42-4,30 (m, 1H, CH(CH₃)CO₂CH₃) ; 3,73 (s, 3H, CO₂CH₃) ; 2,09-1,88 (m, 1H, (CH₃)₂CHCH(O)CH₃) ; 1,56-1,52 (m, *³J_{H,H}* = 7,4 Hz, 3H, CH(CH₃)CO₂CH₃) ; 1,29-1,25 (m, *³J_{H,H}* = 6,4 Hz, 3H, (CH₃)₂CHCH(O)CH₃) ; 1,02-0,91 (m, 6H, (CH₃)₂CHCH(O)CH₃).
RMN ¹³C{¹H} (75,47 MHz, CDCl₃, 298K) δ 211,4 ; 211,4 (C=S) ; 172,0 (CO₂CH₃) ; 86,2-86,1 ((CH₃)₂CHCH(O)CH₃) ; 52,8 (CO₂CH₃) ; 46,5 (CH(CH₃)CO₂CH₃) ; 32,7 ((CH₃)₂CHCH(O)CH₃) ; 18,2-17,8 ((CH₃)₂CHCH(O)CH₃) ; 17,0-16,9 (CH(CH₃)CO₂CH₃) ; 15,8-15,7 ((CH₃)₂CHCH(O)CH₃) ppm.
IR : 1738,5 cm⁻¹ (C=O) ; 1046,2 cm⁻¹ (C=S)

### 1.2) Sous-étape 2 : Préparation du O-1,2-diméthylpropyl S-(1-(diéthylphosphoryl)-4-(méthoxycarbonyl))pent-2-yl xanthate (XA2AP)

9,26 g (37 mmol) du xanthate **XA2** obtenu ci-dessus à l'étape 1.1), 5,87 g (33 mmol) de phosphonate de diéthyle allyle (DEAP, Alfa Aesar) et 2 g (5 mmol) de LPO ont été dissous dans 5 ml de toluène (Sigma-Aldrich). La solution ainsi obtenue a été transférée dans un tube de Schlenk, dégazée par 3 congélations sous vide successives. Après 18 heures de chauffage à 90°C, on a obtenu 10,1 g de brut réactionnel sous la forme d'une huile jaune (rendement 79 %) qui a été purifiée par chromatographie sur silice (éluant acétate d'éthyle/dichlorométhane : 1:4, v:v).

On a ainsi obtenu 12,7 g de **XA2AP** (rendement 90 %).
RMN ¹H (300,13 MHz, CDCl₃, 298K) δ 5,54-5,46 (m, 1H, (CH₃)₂CHCH(O)CH₃) ; 4,15-4,02 (m, 4H, O=P(OCH₂CH₃)₂) ; 4,02-3,89 (m, 1H, CHS) ; 3,62 (s, 3H, CO₂CH₃) ; 2,75-1,63 (m, 6H, CH₂P, (CH₃)₂CHCH(O)CH₃, CH₂CHCO₂CH₃) ; 1,32-1,23 (m, 9H, O=P(OCH₂CH₃)₂, CH₃CHCO₂CH₃) ; 1,17-1,14 (m, 3H, (CH₃)₂CHCH(O)CH₃) ; 0,92-0,90 (m, 6H, (CH₃)₂CHCH(O)CH₃).
RMN ¹³C{¹H} (75,47 MHz, CDCl₃, 298K) δ 212,7-215,6 (C=S) ; 176,2-176,0 (CO₂CH₃) ; 85,8-85,7 ((CH₃)₂CHCH(O)CH₃) ; 62,0-61,8 (O=P(OCH₂CH₃)₂) ; 51,8 (CO₂CH₃) ; 43,8-43,6 (CHS) ; 37,8-36,6 (CH₂P) ; 37,3-37,1 ((CH₃)₂CHCH(O)CH₃) ; 33,4-31,1 (CH₂CH₂CO₂CH₃) ; 32,7 (CHCO₂CH₃) ; 18,3-17,9 ((CH₃)₂CHCH(O)CH₃) ; 18,3-17,8 (CH₃CHCO₂CH₃) ; 16,6-165 (O=P(OCH₂CH₃)₂) ; 15,9-15,8 ((CH₃)₂CHCH(O)CH₃).
RMN ³¹P{¹H} (121,49 MHz, CDCl₃, 298K) δ 26,7 ppm.
IR : 1736,9 cm⁻¹ (C=O) ; 1043,6 cm⁻¹ (C=S)
Masse molaire : IC(CH4), MH⁺
Trouvé : 429,1533 g/mol
Calculé : 429,1534 g/mol.

### 2) Deuxième étape : Préparation du diéthyl (4-méthyl-5-oxo-tetrahydrothiophèn-2-yl)méthylphosphonate (TL2)

4,28 g (10 mmoles) de **XA2AP** obtenu ci-dessus à l'étape précédente ont été placés dans un tube de Schlenk fermé sous vide et portés à une température de 190°C pendant 15 minutes. Le mélange réactionnel a ensuite été refroidi à température ambiante et les composés volatils formés ont été éliminés sous vide. La thiolactone **TL2** ainsi obtenue sous la forme d'une huile incolore (2,4 g, rendement 90 %) a ensuite été purifiée sur une colonne chromatographique de silice (éluant acétate d'éthyle/dichlorométhane : 1:4 (v:v)).
RMN ¹H (300,13 MHz, CDCl₃, 298K) δ 4,18-4,05 (m, 4H, O=P(OCH₂CH₃)₂) ; 4,05-3,90 (m, 1H, CHCH₂P) ; 2,77-2,29 (m, 2H, CH₂CH(CH₃)) ; 2,28-2,07 (m, 2H, CHCH₂P) ; 1,62-1,20 (m, 1H, CHCH₂P) ; 1,35-1,29 (m, 6H, O=P(OCH₂CH₃)₂) ; 1,19-1,14 (m, 3H, CH₂CH(CH₃)).
RMN ¹³C{¹H} (75,47 MHz, CDCl₃, 298K) δ 210,0-209,0 (C=O) ; 62,2-62,1 (O=P(OCH₂CH₃)₂) ; 48,7-45,4 (CHCH₂P) ; 42,6-42,4 (CH₂CH(CH₃)); 41,0-40,9 (CH₂CH(CH₃)) ; 34,2-31,7 (CHCH₂P) ; 16,6-16,5 (O=P(OCH₂CH₃)₂) ; 15,2-14,3 (CH₂CH(CH₃)).
RMN ³¹P{¹H} (121,49 MHz, CDCl₃, 298K) δ 26,40, 26,36 ppm.
IR : 1700,5 cm⁻¹ (C=O) ; 1245,7 cm⁻¹ (P=O) ; 1025,4 cm⁻¹ (P-O)
Masse molaire : IC(CH4), MH⁺
Trouvé : 267,0826 g/mol
Calculé : 267,0820 g/mol.

### Exemple 3 : Synthèse du diéthyl (5-oxo-tetrahydrothiophèn-2-yl)méthylphosphonate (TL3) selon le procédé conforme à l'invention

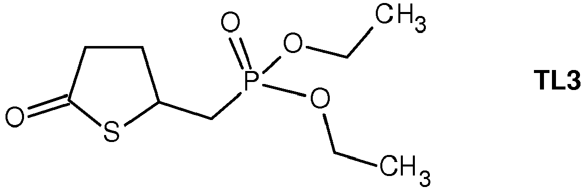

### 1) Première étape : Préparation du O-1,2-diméthylpropyl S-(1-(diéthylphosphoryl)-4-(méthoxycarbonyl))but-2-yl xanthate (mono-adduit de formule (IV) : XA3AP)

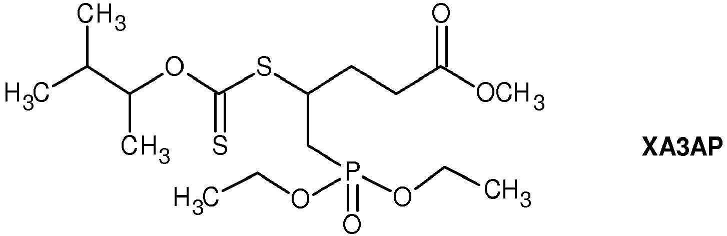

Le mono-adduit **XA3AP** a été préparé selon le mode opératoire utilisé ci-dessus à l'étape 1.2) de l'exemple 2 pour la préparation du mono-adduit **XA2AP,** mais en utilisant 8,74 g (37 mmol) du xanthate **XA1** tel que préparé ci-dessus à l'étape 1) de l'exemple 1, 5,88 g (33 mmol) de **DEAP** (Sigma-Aldrich) et 2 g (5 mmol) de LPO.

On a ainsi obtenu 12,31 g de **XA3AP** (rendement 90 %).
RMN ¹H (300,13 MHz, CDCl₃, 298K) δ 5,49-5,39 (m, 1H, (CH₃)₂CHCH(O)CH₃) ; 4,09-3,96 (m, 4H, O=P(OCH₂CH₃)₂) ; 3,96-3,87 (m, 1H, CHS) ; 3,55 (s, 3H, CO₂CH₃) ; 2,48-1,84 (m, 7H, CH₂P, (CH₃)₂CHCH(O)CH₃, CH₂CH₂CO₂CH₃) ; 1,26-1,20 (m, 6H, O=P(OCH₂CH₃)₂) ; 1,20-1,17 (m, 3H, (CH₃)₂CHCH(O)CH₃) ; 0,86-0,83 (m, 6H, (CH₃)₂CHCH(O)CH₃).
RMN ¹³C{¹H} (75,47 MHz, CDCl₃, 298K) δ 212,2 (Ç=S) ; 172,8 (CO₂CH₃) ; 85,6 ((CH₃)₂CHCH(O)CH₃) ; 61,9-61,7 (O=P(OCH₂CH₃)₂) ; 51,5 (CO₂CH₃) ; 44,5 (CHS) ; 32,5 ((CH₃)₂CHCH(O)CH₃) ; 32,5-30,5 (CH₂P) ; 31,2-31,1 (CH₂CH₂CO₂CH₃) ; 29,1-28,7 (CH₂CH₂CO₂CH₃) ; 18,1-17,8 ((CH₃)₂CHCH(O)CH₃) ; 16,4-16,3 (O=P(OCH₂CH₃)₂) ; 15,6 ((CH₃)₂CHCH(O)CH₃).
RMN ³¹P{¹H} (121,49 MHz, CDCl₃, 298K) δ 26,53 ; 26,51 ppm.
IR : 1738,8 cm⁻¹ (C=O) ; 1044,9 cm⁻¹ (C=S)
Masse molaire : IC(CH4), MH⁺
Trouvé : 415,1390 g/mol
Calculé : 415,1378 g/mol.

### 2) Deuxième étape : Préparation du diéthyl (5-oxo-tetrahydrothiophèn-2-yl)méthylphosphonate (TL3)

La thiolactone **TL3** a été préparée selon le même mode opératoire que celui utilisé ci-dessus à l'exemple 2, étape 2) pour la préparation de la thiolactone **TL2,** mais en utilisant 4,14 g (10 mmoles) du mono-adduit **XA3AP** préparé ci-dessus à l'étape précédente au lieu du mono-adduit **XA2VP.**

On a ainsi obtenu 2,3 g de **TL3** sous la forme d'une huile incolore (rendement 91 %).
RMN ¹H (300,13 MHz, CDCl₃, 298K) δ 4,15-4,00 (m, 5H, CHCH₂P, O=P(OCH₂CH₃)₂) ; 2,63-2,45 (m, 3H, CH₂CH₂CO, CH₂CH₂CO) ; 2,29-2,08 (m, 2H, CHCH₂P) ; 2,05-1,89 (m, 1H, CH₂CH₂CO) ; 1,32-1,25 (m, 6H, O=P(OCH₂CH₃)₂).
¹³C{¹H} RMN ¹³C{¹H} (75,47 MHz, CDCl₃, 298K) δ 207,4 (C=O) ; 62,1-62,0 (O=P(OCH₂CH₃)₂) ; 44,44-44,39 (CHCH₂P) ; 41,8 (CH₂CH₂CO) ; 33,7-31,8 (CHCH₂P) ; 33,4-33,3 (CH₂CH₂CO) ; 16,5-16,4 (O=P(OCH₂CH₃)₂).
RMN ³¹P{¹H} (121,49 MHz, CDCl₃, 298K) δ 26,2 ppm.
IR : 1704,4 cm⁻¹ (C=O) ; 1250,5 cm⁻¹ (P=O) ; 1025,2 cm⁻¹ (P-O)
Masse molaire : IC(CH4), MH⁺
Trouvé : 253,0667 g/mol
Calculé : 253,0663 g/mol.

### Exemple 4 : Synthèse de la 3-méthyl-5-pentyl-dihydrothiophèn-2(3H)-one (TL4) selon le procédé conforme à l'invention

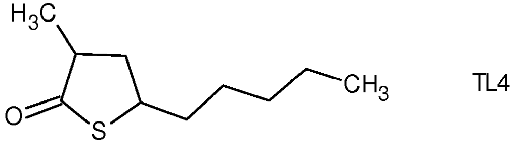

### 1) Première étape : Préparation du O-1,2-diméthylpropyl S-2-(méthoxycarbonyl)non-4-yl xanthate (mono-adduit de formule (IV) : XA4H)

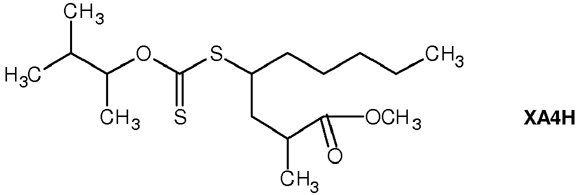

Le mono-adduit **XA4H** a été préparé selon le même mode opératoire que celui utilisé ci-dessus à l'étape 1.2) de l'exemple 2 pour la préparation du mono-adduit **XA2AP,** mais en utilisant 9,26 g (37 mmol) du xanthate **XA2** préparé à l'étape 1.1) de l'exemple 2, 3,24 g (33 mmol) de 1-heptène (Sigma Aldrich) et 2 g (5 mmol) de (LPO).

On a ainsi obtenu 10,7 g de **XA4H** (huile jaune ; éluant acétate d'éthyle/éther de pétrole (95:5 ; v:v), sous la forme d'un racémate (rendement 92 %).
RMN ¹H (300,13 MHz, CDCl₃, 298K) δ 5,59-5,46 (m, 1H, (CH₃)₂CHCH(O)CH₃) ; 3,78-3,64 (m, 1H, SCHCH₂CH(CH₃)CO₂CH₃) ; 3,64-3,61 (m, 3H, CO₂CH₃) ; 2,73-2,54 (m, 1H, SCHCH₂CH(CH₃)CO₂CH₃) ; 2,13-1,87 (m, 2H, SCHCH₂CH(CH₃)CO₂CH₃) ; 1,75-1,47 (m, 3H, (CH₃)₂CHCH(O)CH₃, CH₂(CH₂)₃CH₃) ; 1,47-1,31 (m, 2H, CH₂CH₂(CH₂)₂CH₃) ; 1,31-1,18 (m, 7H, CH(CH₃)CO₂CH₃, (CH₂)₂(CH₂)₂CH₃) ; 1,18-1,09 (m, 3H, (CH₃)₂CHCH(O)CH₃) ; 0,97-0,87 (m, 6H, (CH₃)₂CHCH(O)CH₃) ; 0,87-0,79 (m, 3H, (CH₂)4CH₃).
RMN ¹³C{¹H} (75,47 MHz, CDCl₃, 298K) δ 213,8-213,7 (C=S) ; 176,3-176,3 (CO₂CH₃) ; 85,2-85,0 ((CH₃)₂CHCH(O)CH₃) ; 51,6 (CO₂CH₃) ; 49,1-48,6 (SCHCH₂CH(CH₃)CO₂CH₃) ; 38,1-37,5 (SCHCH₂CH(CH₃)CO₂CH₃) ; 37,2-37,1 (SCHCH₂CH(CH₃)CO₂CH₃) ; 35,2-34,7 (CH₂(CH₂)₃CH₃) ; 32,7 ((CH₃)₂CHCH(O)CH₃) ; 31,6 ((CH₂)₂CH₂CH₂CH₃) ; 26,3-26,2 (CH₂CH₂(CH₂)₂CH₃) ; 22,4 ((CH₂)₃CH₂CH₃) ; 18,1-17,0 ((CH₃)₂CHCH(O)CH₃) ; 17,9-17,8 (CH(CH₃)CO₂CH₃) ; 15,71-15,69 ((CH₃)₂CHCH(O)CH₃) ; 13,9 (SCH(CH₂)₄CH₃) ppm.
IR : 1738,5 cm⁻¹ (C=O) ; 1047,2 cm⁻¹ (C=S).
Masse molaire : IC(CH4), MH⁺
Trouvé : 349,1861 g/mol
Calculé : 349,1871 g/mol.

### 2) Deuxième étape : Préparation de la 3-méthyl-5-pentyl-dihydrothiophèn-2(3H)-one (TL4)

La thiolactone **TL4** a été préparée selon le même mode opératoire que celui utilisé ci-dessus à l'exemple 2, étape 2) pour la préparation de la thiolactone **TL2,** mais en utilisant 3,48 g (10 mmoles) du mono-adduit **XA4H** préparé ci-dessus à l'étape précédente au lieu du mono-adduit **XA2AP.**

On a ainsi obtenu 1,73 g de thiolactone **TL4** (rendement 93 %) sous la forme d'un liquide incolore (éluant : acétate d'éthyle/éther de pétrole : 9:1 ; v:v).
RMN ¹H (300 MHz, CDCl₃, 298K) δ 3,79-3,64 (m, 1H, SCHCH₂CH) ; 2,76-2,46 (m, 2H, SCHCH₂CH) ; 2,20-1,97 (m, 1H, SCHCH₂CH) ; 1,83-1,62 (m, 2H, CH₂CH₂(CH₂)₂CH₃) ; 1,55-1,35 (m, 2H, CH₂(CH₂)₃CH₃) ; 1,35-1,21 (m, 4H, (H₂)₂(CH₂)₂CH₃) ; 1,20-1,13 (m, 3H, CHCH₃) ; 0,92-0,84 (m, 3H, (CH₂)₄CH₃).
RMN ¹³C{¹H} (75,47 MHz, CDCl₃, 298K) δ 211,2-210,0 (C=O) ; 48,92-47,89 (SCHCH₂CH) ; 47,5-456 (SCHCH₂CH) ; 415-39,7 (SCHCH₂CH) ; 36,8-36,5 (CH₂(CH₂)₃CH₃) ; 31,7-31,6 ((CH₂)₂CH₂CH₂CH₃) ; 28,2-28,0 (CH₂CH₂(CH₂)₂CH₃) ; 22,60-22,56 ((CH₂)₃CH₂CH₃) ; 15,5-14,6 (CHCH₃) ; 14,1 ((CH₂)₄CH₃) ppm.
IR : 1700,9 cm⁻¹ (C=O).
Masse molaire : IE
Trouvé : 186,1075 g/mol
Calculé : 186,1078 g/mol.

### Exemple 5 : Synthèse de la 5-pentyl-dihydrothiophèn-2(3H)-one (TL5) selon le procédé conforme à l'invention

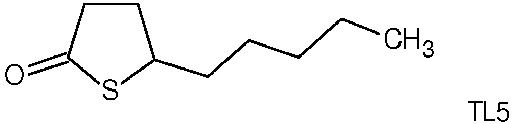

### 1) Première étape : Préparation du 0-1,2-diméthylpropyl S-1-(méthoxycarbonyl)oct-3-yl xanthate (mono-adduit de formule (IV) : XA5H)

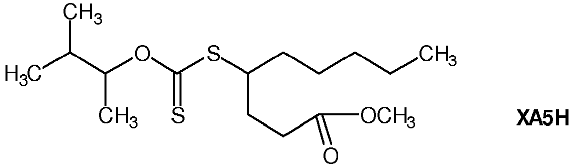

Le mono-adduit **XA5H** a été préparé selon le mode opératoire utilisé ci-dessus à l'étape 1.2) de l'exemple 2 pour la préparation du mono-adduit **XA2AP,** mais en utilisant 8,74 g (37 mmol) du xanthate **XA1** tel que préparé ci-dessus à l'étape 1.2) de l'exemple 1, 3,24 g (33 mmol) de 1-heptène et 2 g (5 mmol) de LPO.

On a ainsi obtenu 10 g de **XA5H** sous la forme d'une huile jaune (éluant acétate d'éthyle/éther de pétrole : 95:5 ; v:v) sous la forme d'un racémate (rendement 90 %).
RMN ¹H (300,13 MHz, CDCl₃, 298K) δ 5,53 (p, *³J_{H,H}* = 6,3 Hz, 1H, (CH₃)₂CHCH(O)CH₃) ; 3,78-3,66 (m, 1H, SCHCH₂CH₂CO₂CH₃) ; 3,64 (s, 3H, CO₂CH₃) ; 2,49-2,36 (m, 2H, SCHCH₂CH₂CO₂CH₃) ; 2,12-1,94 (m, 2H, CH₂(CH₂)₃CH₃) ; 1,93-1,77 (m, 1H, (CH₃)₂CHCH(O)CH₃) ; 1,69-1,52 (m, 2H, SCHCH₂CH₂CO₂CH₃) ; 1,52-1,33 (m, 2H, CH₂CH₂(CH₂)₂CH₃) ; 1,33-1,16 (m, 7H, (CH₂)₂(CH₂)₂CH₃ ; (CH₃)₂CHCH(O)CH₃) ; 0,93 (d, J = 6,8 Hz, 6H, (CH₃)₂CHCH(O)CH₃) ; 0,89-0,79 (m, 3H, (CH₂)₄CH₃).
RMN ¹³C{¹H} (75,47 MHz, CDCl₃, 298K) δ 214,0-213,9 (C=S) ; 173,5-173,5 (CO₂CH₃) ; 85,4-85,4 ((CH₃)₂CHCH(O)CH₃) ; 51,7 (CO₂CH₃) ; 50,3-50,2 (SCHCH₂CH₂CO₂CH₃) ; 34,6-34,3 (CH₂(CH₂)₃CH₃) ; 32,8 ((CH₃)₂CHCH(O)CH₃) ; 31,7 ((CH₂)₂CH₂CH₂CH₃) ; 31,5-31,4 (SCHCH₂CH₂CO₂CH₃) ; 29.7-29,3 (SCHCH₂CH₂CO₂CH₃) ; 26,6-26,5 (CH₂CH₂(CH₂)₂CH₃) ; 22,6 ((CH₂)₃CH₂CH₃) ; 18,3-18,0 ((CH₃)₂CHCH(O)CH₃) ; 15,8 ((CH₃)₂CHCH(O)CH₃) ; 14,1 ((CH₂)₄CH₃) ppm.
IR : 1740,9 cm⁻¹ (C=O) ; 1048,6 cm⁻¹ (C=S).
Masse molaire : IC(CH₄), MH⁺
Trouvé : 335,1701 g/mol
Calculé : 335,1715 g/mol

### 2) Deuxième étape : Préparation de la 5-pentyl-dihydrothiophèn-2(3H)-one (TL5)

La thiolactone **TL5** a été préparée selon le même mode opératoire que celui utilisé ci-dessus à l'exemple 2, étape 2) pour la préparation de la thiolactone **TL2,** mais en utilisant 3,34 g (10 mmoles) du mono-adduit **XA5H** préparé ci-dessus à l'étape précédente au lieu du mono-adduit **XA2AP.**

On a ainsi obtenu 1,55 g de **TL5** sous la forme d'un liquide incolore (éluant : acétate d'éthyle/éther de pétrole : 9:1 ; v:v) (rendement 90 %).
RMN ¹H (300,13 MHz, CDCl₃, 298K) δ 3,82 (tt, *³J_{H,H}* = 8,5 ; 5,8 Hz, 1H, SCHCH₂CH₂) ; 2,66-2,44 (m, 2H, SCHCH₂CH₂) ; 2,43-1,79 (m, 2H, SCHCH₂CH₂) ; 1,79-1,62 (m, 2H, CH₂CH₂(CH₂)₂CH₃) ; 1,47-1,33 (m, 2H, CH₂(CH₂)₃CH₃) ; 1,32-1,19 (m, 4H, (CH₂)₂(CH₂)₂CH₃) ; 0,86 (t, *³J_{H,H} =* 7,0 Hz, 3H, (CH₂)₄CH₃).
RMN ¹³C{¹H} (75,47 MHz, CDCl₃, 298K) δ 208,6 (C=O) ; 51,4 (SCHCH₂CH₂) ; 42,1 (SCHCH₂CH₂) ; 36,5 (CH₂(CH₂)₃CH₃) ; 32,3 ((CH₂)₂CH₂CH₂CH₃) ; 31,5 (SCHCH₂CH₂) ; 28,0 (CH₂CH₂(CH₂)₂CH₃) ; 22,5 ((CH₂)₃CH₂CH₃) ; 14,0 ((CH₂)₄CH₃) ppm.
IR : 1704,6 cm⁻¹ (C=O)
Masse : IE
Trouvé : 172,0910 g/mol
Calculé : 172,0922 g/mol.

### Exemple 6 : Synthèse de la 3-méthyl-5-(perfluorooctyl)dihydrothiophèn-2(3H)-one (TL6) selon le procédé conforme à l'invention

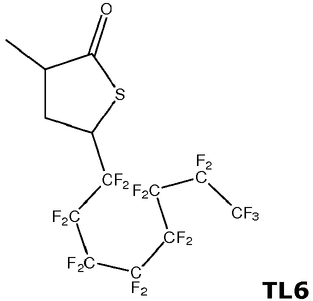

### 1) Première étape : Préparation du méthyl 5,5,6,6,7,7,8,8,9,9,10,10,11,11,12,12,12-heptadecafluoro-2-méthyl-4-((((3-méthylbutan-2-yl)oxy)carbonothioyl)thio)dodécanoate (mono-adduit de formule (IV) : XA6DF)

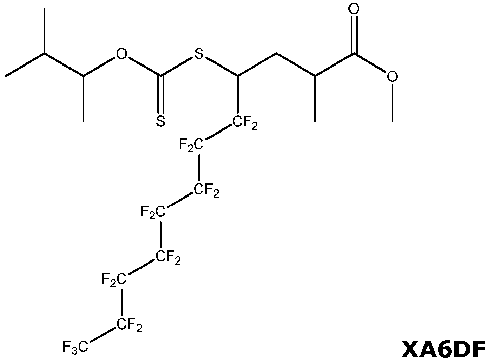

Le mono-adduit **XA6DF** a été préparé selon le mode opératoire utilisé ci-dessus à l'étape 1.2) de l'exemple 2 pour la préparation du mono-adduit **XA2AP,** mais en utilisant 3,09 g (1,23 10⁻² mol) de xanthate **XA2** tel que préparé ci-dessus à l'étape 1.1) de l'exemple 2, 5 g (1,12 10⁻² mol) de 1H,1H,2H-perfluoro-1-décène (Aldrich), 0,67 g de **LPO,** et un solvant de réaction composé de 4 mL de toluène et 3 mL de trifluorotoluène.

Le mono-adduit **XA6DF** obtenu sous la forme d'une huile visqueuse jaune a été purifié par chromatographie sur silice (éluant hexane/acétate d'éthyle : 9:1, v:v).

On a ainsi obtenu 3,2 g de **XA6DF** (rendement 41%).
RMN ¹H (300,13 MHz, CDCl₃, 298K) : δ (ppm) 5,58 - 5,47 (s, 1H, (CH₃)₂CHCH(O)CH₃) ; 4,87 - 4,67 (m, 1H, SCHCH₂CH(CH₃)) ; 3,70 - 3,68 (m, 3H, CO₂CH₃) ; 2,92 - 2,68 (1H, m, SCHCH₂CH(CH₃)) ; 2,14 - 1,92 (m, 1H, (CH₃)₂CHCH(O)CH₃) ; 1,58 - 1,53 (m, 2H, SCHCH₂CH(CH₃)) ; 1,32 - 1,20 (m, 6H ; (CH₃)₂CHCH(O)CH₃ et SCHCH₂CH(CH₃)) ; 0,97 - 0,93 (m, 6H, (CH₃)₂CHCH(O)CH₃).
RMN ¹³C{¹H} (75,47 MHz, CDCl₃, 298K) : δ (ppm) 209,33 (C=S) ; 175,54 (CO₂CH₃) ; 129,01 - 115,52 (CH(CF₂)₇CF₃) ; 87,48 ((CH₃)₂CHCH(O)CH₃) ; 52,71 - 51,79 (CO₂CH₃) ; 46,50 (CH(CF₂)₃CF₃) ; 36,48 (SCHCH₂CH(CH₃)) ; 32,71 ((CH₃)₂CHCH(O)CH₃) ; 18,30 - 15,44 ((CH₃)₂CHCH(O)CH₃ et (SCHCH₂CH(CH₃)).
RMN ¹⁹F{¹H} (282,38 MHz, CDCl₃, 298K) : δ (ppm) -80,83 (CH(CF₂-CF₂-CF₂-CF₂-CF₂-CF₂-CF₂-CF₃)) ; -105,65 - -116,25 (CH(CF₂-CF₂-CF₂-CF₂-CF₂-CF₂-CF₂-CF₃)) ; -118,87 - -119,33 (CH(CF₂-CF₂-CF₂-CF₂-CF₂-CF₂-CF₂-CF₃)) ; -121,69 - -121,91 (CH(CF₂-CF₂-CF₂-CF₂-CF₂-CF₂-CF₂-CF₃)) ; -122,72 (CH(CF₂-CF₂-CF₂-CF₂-CF₂-CF₂-CF₂-CF₃)) ; -126,16 (CH(CF₂-CF₂-CF₂-CF₂-CF₂-CF₂-CF₂-CF₃)).

### 2) Deuxième étape : Préparation de la 3-méthyl-5-(perfluorooctyl)dihydrothiophèn-2(3H)-one (TL6)

2 g (2,87 mmoles) de **XA6DF** obtenu ci-dessus à l'étape précédente ont été placés dans un tube de Schlenk fermé sous vide et portés à une température de 190°C pendant 48 heures. Le mélange réactionnel a ensuite été refroidi à température ambiante et les composés volatils formés ont été éliminés sous vide. La thiolactone **TL6** ainsi obtenue sous la forme d'une huile jaune a ensuite été purifiée sur une colonne chromatographique de silice (éluant acétate d'éthyle/hexane : 1:1 (v:v)).

On a ainsi obtenu 0,8 g de **TL6** sous la forme d'une poudre blanche (rendement 52 %).
RMN ¹H (300,13 MHz, CDCl₃, 298K) : δ (ppm) 4,49 - 4,36 (m, 1H, CHCF₂) ; 2,75 - 2,59 (m, 2H, C(O)CH(CH₃)CH₂CH) ) ; 1,99 - 1,87 (q, 1H, C(O)CH(CH₃)CH₂CH) ; 1,26 - 1,23 (d, 3H, C(O)CH(CH₃)).
RMN ¹⁹F{¹H} (282,38 MHz, CDCl₃, 298K) : δ (ppm) -81,03 (CH(CF₂-CF₂-CF₂-CF₂-CF₂-CF₂-CF₂-CF₃)) ; -112,13 - -119,71 (CH(CF₂-CF₂-CF₂-CF₂-CF₂-CF₂-CF₂-CF₃)) ; -121,08 (CH(CF₂-CF₂-CF₂-CF₂-CF₂-CF₂-CF₂-CF₃)) ; -121,96 (CH(CF₂-CF₂-CF₂-CF₂-CF₂-CF₂-CF₂-CF₃)) ; -122,86 (CH(CF₂-CF₂-CF₂-CF₂-CF₂-CF₂-CF₂-CF₃)) ; -126,29 (CH(CF₂-CF₂-CF₂-CF₂-CF₂-CF₂-CF₂-CF₃)).

### Exemple 7 : Synthèse de la 3-méthyl-5-phényldihydro-2H-thiéno[2,3-c]pyrrole-2,4,6(3H,5H)-trione (TL7) selon le procédé conforme à l'invention

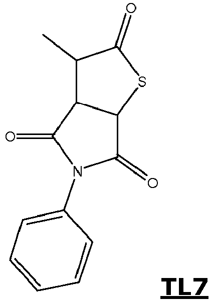

### 1) Première étape : Préparation du XA7MAL (mono-adduit de formule (IV))

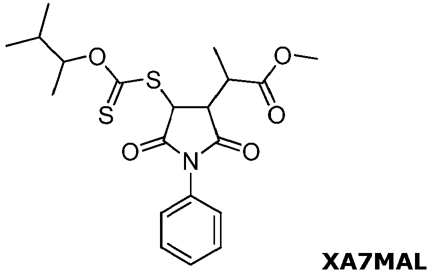

Le mono-adduit **XA7MAL** a été préparé selon le mode opératoire utilisé ci-dessus à l'étape 1.2) de l'exemple 2 pour la préparation du mono-adduit **XA2AP,** mais en utilisant 4,77 g (1,91 10⁻² mol) de xanthate **XA2** tel que préparé ci-dessus à l'étape 1.1) de l'exemple 2, 3 g (1,73 10⁻² mol) de N-phényl maléimide (Aldrich), et 1,03 g de **LPO.**

Le mono-adduit **XA7MAL** obtenu sous la forme d'une huile visqueuse jaune a été purifié par chromatographie sur silice (éluant hexane/acétate d'éthyle : 7:3, v:v).

On a ainsi obtenu 4,9 g de **XA7MAL** (rendement 68 %).
RMN ¹H (300,13 MHz, CDCl₃, 298K) : δ (ppm) 7,47 - 7,30 (m, 5H, N-C₆H₅) ; 5,57 - 5,48 (s, 1H, (CH₃)₂CHCH(O)CH₃) ; 5,22 - 4,07 (m, 1H, SCH(CO)CH) ; 3,76 - 3,66 (m, 3H, CO₂CH₃) ; 3,55 - 3,21 (m, 2H, SCH(CO)CH(CO)CH(CH₃)) ; 2,07 - 1,92 (1H, (CH₃)₂CHCH(O)CH₃) ; 1,56 - 1,39 (m, 3H, CH(CO)CH(CH₃)) ; 1,39 - 1,26 (m, 3H, (CH₃)₂CHCH(O)CH₃) ; 0,98 - 0,92 (m, 3H, (CH₃)₂CHCH(O)CH₃).
RMN ¹³C{¹H} (75,47 MHz, CDCl₃, 298K) : δ (ppm) 209,77 (C=S) ; 175,59 - 171,71 (C(O)NC(O) et CO₂CH₃) ; 131,38 - 126,36 (N-C₆H₅) ; 87,27 ((CH₃)₂CHCH(O)CH₃) ; 52,42 CO₂CH₃) ; 50,47 - 46,87 (SC-H(CO)CH(CO)CH(CH₃)) ; 39,71 - 38,99 (SCH(CO)CH(CO)CH(CH₃)) ; 32,82 ((CH₃)₂CHCH(O)CH₃) ; 18,34 - 17,84 ((CH₃)₂CHCH(O)CH₃) ; 15,81 - 15,21 (SCH(CO)CH(CO)CH(CH₃)).

### 2) Deuxième étape : Préparation de la 3-méthyl-5-phényldihydro-2H-thiéno[2,3-c]pyrrole-2,4,6(3H,5H)-trione (TL7)

1 g (2,36 mmoles) de **XA7MAL** obtenu ci-dessus à l'étape précédente ont été placés dans un tube de Schlenk fermé sous vide et portés à une température de 190°C pendant 48 heures. Le mélange réactionnel a ensuite été refroidi à température ambiante et les composés volatils formés ont été éliminés sous vide. La thiolactone **TL7** ainsi obtenue sous la forme d'une huile jaune a ensuite été purifiée sur une colonne chromatographique de silice (éluant acétate d'éthyle/hexane : 1:1 (v:v)).

On a ainsi obtenu 0,4 g de **TL7** (rendement 65 %).
RMN ¹H (300,13 MHz, CDCl₃, 298K) : δ (ppm) 7,55 - 7,30 (m, 5H, N-C₆H₅) ; 4,86 - 4,75 (s, 1H, SCH(CO)CH) ; 3,46 - 3,44 (m, 1H, CH(CH₃)CHC(O)) ; 3,34 - 3,25 (m, 1H, C(O)CH(CH₃)) ; 1,49 - 1,47 (d, 3H, C(O)CH(CH₃)).
RMN ¹³C{¹H} (75,47 MHz, CDCl₃, 298K) : δ (ppm) 205,25 (SC=O) ; 174,5 - 173,49 (C(O)NC(O)) ; 129,21 - 126,39 (N-C₆H₅) 49,54 (SCH(CO)) ; 49,03 (C(O)CH(CH₃)) ; 46,81 (C(O)CH(CH₃)CH(CO) ; 18,41 (C(O)CH(CH₃)CH₂).

### Exemple 8 : Synthèse de la 3-méthyl-5-(perfluorobutyl)dihydrothiophèn-2(3H)-one (TL8) selon le procédé conforme à l'invention

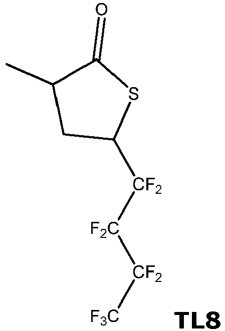

### 1) Première étape : Préparation du méthyl 5,5,6,6,7,7,8,8,8-nonafluoro-2-méthyl-4-((((3-méthylbutan-2-yl)oxy)carbonothioyl)thio)octanoate (mono-adduit de formule (IV) : XA8HF)

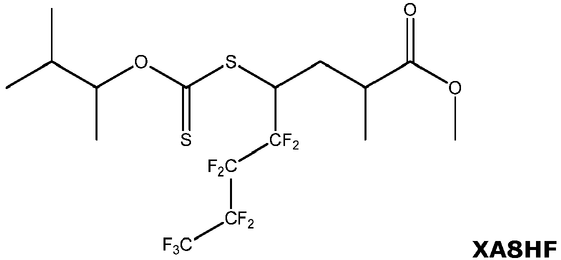

Le mono-adduit **XA8HF** a été préparé selon le mode opératoire utilisé ci-dessus à l'étape 1.2) de l'exemple 2 pour la préparation du mono-adduit **XA2AP,** mais en utilisant 4,47 g (1,78 10⁻² mol) de xanthate **XA2** tel que préparé ci-dessus à l'étape 1.1) de l'exemple 2, 4 g (1,62 10⁻² mol) de 1H,1H,2H-perfluoro-1-hexène (Aldrich) et 0,969 g de **LPO.**

Le mono-adduit **XA8HF** obtenu sous la forme d'une huile visqueuse jaune a été purifié par chromatographie sur silice (éluant hexane/acétate d'éthyle : 9:1, v:v).

On a ainsi obtenu 3,73 g de **XA8HF** (rendement 46%).
RMN ¹H (300,13 MHz, CDCl₃, 298K) : δ (ppm) 5,59 - 5,48 (s, 1H, (CH₃)₂CHCH(O)CH₃) ; 4,92 - 4,66 (m, 1H, SCHCH₂CH(CH₃)) ; 3,73 - 3,67 (m, 3H, CO₂CH₃) ; 2,91 - 2,61 (1H, m, SCHCH₂CH(CH₃)) ; 2,16 - 1,88 (m, 1H, (CH₃)₂CHCH(O)CH₃) ; 1,60 - 1,52 (m, 2H, SCHCH₂CH(CH₃)) ; 1,32 - 1,20 (m, 6H, (CH₃)₂CHCH(O)CH₃ et SCHCH₂CH(CH₃)) ; 0,96 - 0,92 (m, 6H, (CH₃)₂CHCH(O)CH₃).
RMN ¹³C{¹H} (75,47 MHz, CDCl₃, 298K) : δ (ppm) 209,30 (C=S) ; 175,35 (CO₂CH₃) ; 129,01 - 127,51 (CH(CF₂)₃CF₃) ; 87,49 ((CH₃)₂CHCH(O)CH₃) ; 52,70 - 51,91 (CO₂CH₃) ; 46,48 CH(CF₂)₃CF₃) ; 36,46 (SCHCH₂CH(CH₃)), 32,64 ((CH₃)₂CHCH(O)CH₃) ; 18,32 - 14,08 ((CH₃)₂CHCH(O)CH₃ et (SCHCH₂CH(CH₃)).
RMN ¹⁹F{¹H} (282.38 MHz, CDCl₃, 298K): δ (ppm) -80.80 (CH(CF₂-CF₂-CF₂-CF₃)), -105.96 - -116.42 (CH(CF₂-CF₂-CF₂-CF₃)), -119.86 - -120.36 (CH(CF₂-CF₂-CF₂-CF₃)), -125.60 - -126.30 (CH(CF₂-CF₂-CF₂-CF₃)).

### 2) Deuxième étape : Préparation de la 3-méthyl-5-(perfluorobutyl)dihydrothiophèn-2(3H)-one (TL8)

2 g (4,03 mmoles) de **XA8HF** obtenu ci-dessus à l'étape précédente ont été placés dans un tube de Schlenk fermé sous vide et portés à une température de 190°C pendant 48 heures. Le mélange réactionnel a ensuite été refroidi à température ambiante et les composés volatils formés ont été éliminés sous vide. La thiolactone **TL8** ainsi obtenue sous la forme d'une huile incolore a ensuite été purifiée sur une colonne chromatographique de silice (éluant acétate d'éthyle/hexane : 5:95 (v:v)).

On a ainsi obtenu 0,8 g de **TL8** sous la forme d'une poudre blanche (rendement 52 %).
RMN ¹H (300,13 MHz, CDCl₃, 298K) : δ (ppm) 4,49 - 4,36 (m, 1H, CHCF₂) ; 2,75 - 2,59 (m, 2H, C(O)CH(CH₃)CH₂CH)) ; 1,99 - 1,87 (q, 1H, C(O)CH(CH₃)CH₂CH) ; 1,26 - 1,23 (d, 3H, C(O)CH(CH₃)).
RMN ¹⁹F{¹H} (282,38 MHz, CDCl₃, 298K) : δ (ppm) -81,03 (CH(CF₂-CF₂-CF₂-CF₂-CF₂-CF₂-CF₂-CF₃)) ; -112,13 - -119,71 (CH(CF₂-CF₂-CF₂-CF₂-CF₂-CF₂-CF₂-CF₃)) ; -121,08 (CH(CF₂-CF₂-CF₂-CF₂-CF₂-CF₂-CF₂-CF₃)) ; -121,96 (CH(CF₂-CF₂-CF₂-CF₂-CF₂-CF₂-CF₂-CF₃)) ; -122,86 (CH(CF₂-CF₂-CF₂-CF₂-CF₂-CF₂-CF₂-CF₃)) ; -126,29 (CH(CF₂-CF₂-CF₂-CF₂-CF₂-CF₂-CF₂-CF₃)).

### Exemple 9 : Fonctionnalisation de polymères par une thiolactone selon l'invention

### 1) Exemple 9.1 : Fonctionnalisation du bis(3-aminopropyl)-poly(diméthylsiloxane) par TL4

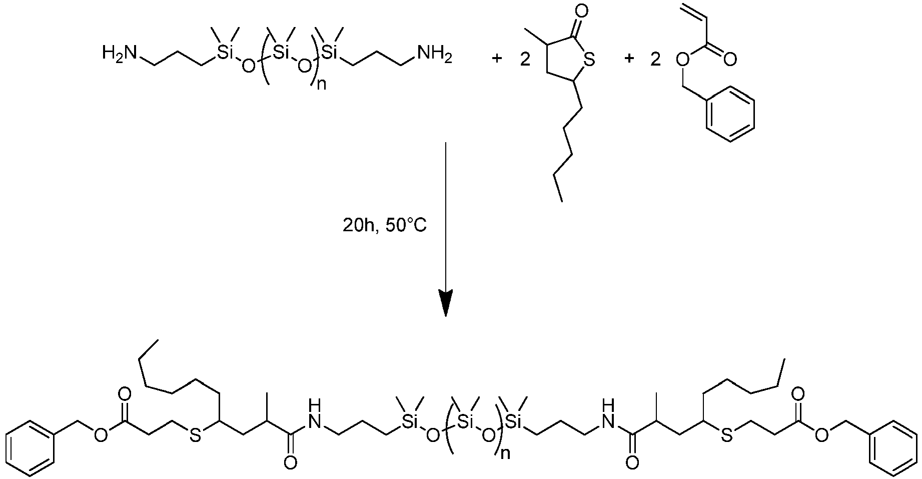

Dans un ballon de 25 ml, on a introduit successivement le bis(3-aminopropyl)-poly(diméthylsiloxane) (2500 g.mol-1, 500 mg, 2.10⁻⁴ mol, Aldrich), **TL4** (52,1 mg, 2,8.10⁻⁴mol) et de l'acrylate de benzyle (45,4 mg, 2,8.10⁻⁴ mol, Aldrich). Le mélange réactionnel a été agité pendant 20 heures à 50°C. Une analyse RMN 1H et une analyse MALDI-TOF ont montré une fonctionnalisation totale du bis(3-aminopropyl)-poly(diméthylsiloxane).

### 2) Exemple 9.2 : Fonctionnalisation du méthoxypolyéthylène glycol amine par TL4

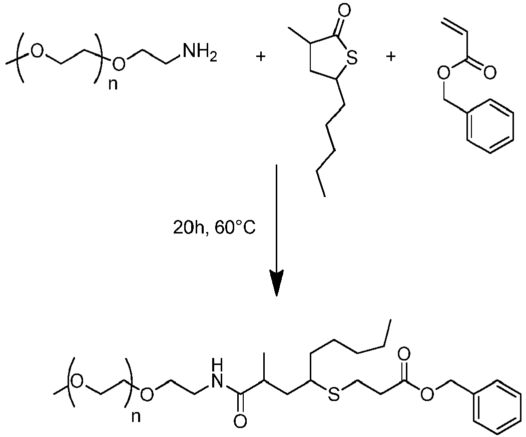

Dans un ballon de 25 ml, on a introduit successivement du méthoxypolyéthylène glycol amine (2000 g.mol⁻¹, 200 mg, 1.10⁻⁴ mol, Aldrich), **TL4** (16,7 mg, 9.10⁻⁵mol) et de l'acrylate de benzyle (14,6 mg, 9.10⁻⁵ mol, Aldrich). Le mélange réactionnel a été agité pendant 20 heures à 60°C. Une analyse RMN 1H et une analyse MALDI-TOF ont montré une fonctionnalisation totale du méthoxypolyéthylène glycol amine.

### 3) Exemple 9.3 : Fonctionnalisation du bis(3-aminopropyl)-poly(diméthylsiloxane) par TL2

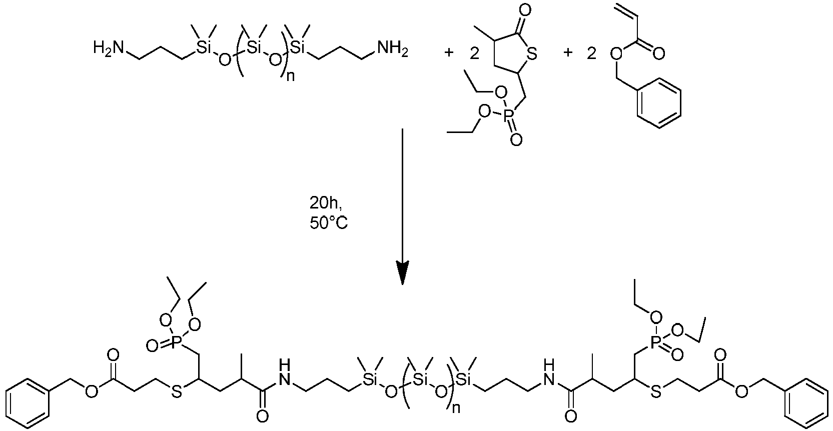

Dans un ballon de 25 ml, on a introduit successivement le bis(3-aminopropyl)-poly(diméthylsiloxane) (2500 g.mol-1, 500 mg, 2.10⁻⁴ mol, Aldrich), **TL2** (74,5 mg, 2,8.10⁻⁴mol) et de l'acrylate de benzyle (45,4 mg, 2,8.10⁻⁴ mol, Aldrich). Le mélange réactionnel a été agité pendant 20 heures à 50°C. Une analyse RMN ¹H a montré une fonctionnalisation totale du bis(3-aminopropyl)-poly(diméthylsiloxane).

### 4) Exemple 9.4 : Fonctionnalisation du méthoxypolyéthylène glycol amine par TL2

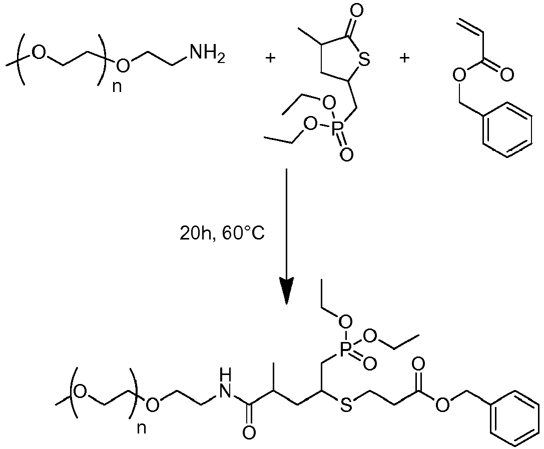

Dans un ballon de 25 ml, on a introduit successivement du méthoxypolyéthylène glycol amine (2000 g.mol⁻¹, 200 mg, 1.10⁻⁴ mol, Aldrich), **TL2** (23,9 mg, 9.10⁻⁵mol) et de l'acrylate de benzyle (14,6 mg, 9.10⁻⁵ mol, Aldrich). Le mélange réactionnel a été agité pendant 20 heures à 60°C. Une analyse RMN ¹H a montré une fonctionnalisation totale du méthoxypolyéthylène glycol amine.

### 5) Exemple 9.5 : Polymérisation du bis(3-aminopropyl)-poly(diméthylsiloxane) avec TL2 et du poly(éthylène glycol)diacrylate

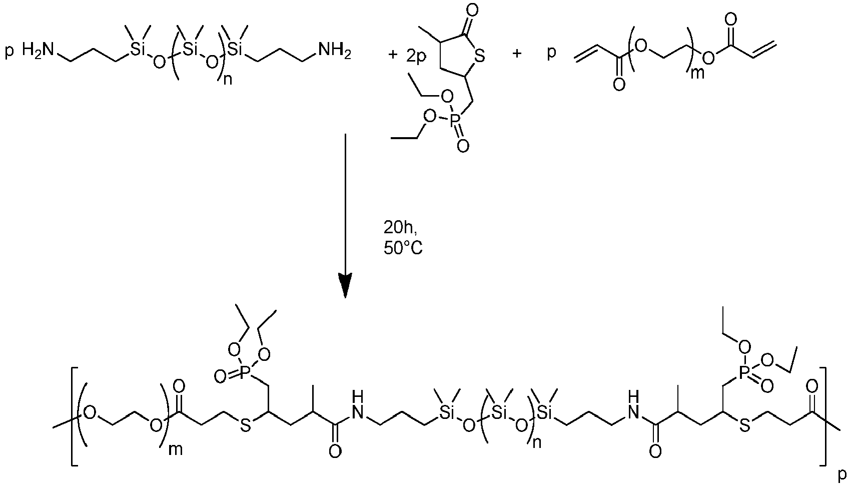

Dans un ballon de 25 ml, on a introduit successivement le bis(3-aminopropyl)-poly(diméthylsiloxane) (2500 g.mol⁻¹, 500 mg, 2,0 10⁻⁴ mol, Aldrich), **TL2** (74,5 mg, 2,8.10⁻⁴mol) et le poly(éthylène glycol) diacrylate (575 g.mol⁻¹, 80,5 mg, 1,4.10⁻⁴ mol, Aldrich). Le mélange réactionnel a été agité pendant 20 heures à 50°C. Une analyse RMN ¹H et ³¹P a montré une consommation totale des monomères et une analyse par chromatographie d'exclusion stérique confirme la formation du polymère. Mn_{(PS)} = 8100 g.mol⁻¹ MW_{(PS)} = 14500 g.mol⁻¹.

### Exemple 10 : Synthèse de la dihydro-5-(3-(tétrahydro-4-méthyl-5-oxothiophén-2-yl)propyl)-3-méthylthiophén-2(3H)-one (TL23) selon le procédé conforme à l'invention

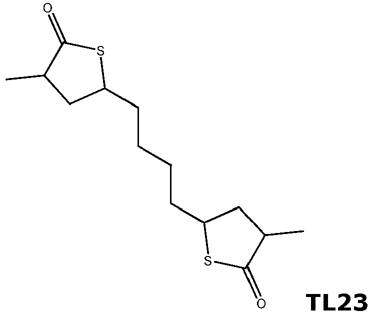

### 1) Première étape : Préparation du mono-adduit de formule (IVa) ; XA23OD

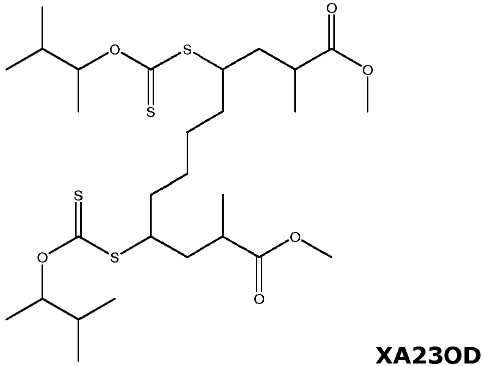

Le mono-adduit **XA23OD** a été préparé selon le mode opératoire utilisé ci-dessus à l'étape 1.2) de l'exemple 2 pour la préparation du mono-adduit **XA2AP,** mais en utilisant 4,77 g (1,90 10⁻² mol) de xanthate **XA2** tel que préparé à l'étape 1.2) de l'exemple 2, 1g (0,90 10⁻² mol) de 1,7-octadiène (Aldrich), 0,54 g de **LPO,** et un solvant de réaction composé de 2 ml de toluène.

Le mono-adduit **XA23OD** obtenu sous la forme d'une huile visqueuse jaune a été purifié par chromatographie sur silice (éluant hexane/acétate d'éthyle : 95:5, v:v).

On a ainsi obtenu 2,49 g de **XA23OD** (rendement 45%).
RMN ¹H (300,13 MHz, CDCl₃, 298K): δ (ppm) 5,47-5,46 (s, 2H, (CH₃)₂CHCH(O)CH₃) ; 3,77-3,78 (m, 2H, SCHCH₂CH(CH₃)) ; 3,66-3,64 (m, 6H, CO₂CH₃) ; 2,73-2,61 (2H, m, SCHCH₂CH(CH₃)) ; 2,15-1,91 (m, 4H, SCHCH₂CH(CH₃)) ; 1,73-1,49 (m, 6H, (CH₃)₂CHCH(O)CH₃ et SCH(CH₂CH₂)CH₂CH(CH₃)) ; 1,49-1,34 (m, 4H, SCH(CH₂CH₂)CH₂CH(CH₃)) ; 1,28-1,23 (d, 3H, SCHCH₂CH(CH₃)) ; 1,17-1,15 (d, 3H, (CH₃)₂CHCH(O)CH₃) ; 0,94-0,92 (m, 6H, (CH₃)₂CHCH(O)CH₃).

### 2) Deuxième étape : Préparation de la dihydro-5-(3-(tétrahydro-4-méthyl-5-oxothiophén-2-yl)propyl)-3-méthylthiophén-2(3H)-one (TL23)

2,49 g (4,1 mmoles) de **XA23OD** obtenu ci-dessus à l'étape précédente ont été placés dans un tube de Schlenk fermé sous vide et portés à une température de 190°C pendant 48 heures. Le mélange réactionnel a ensuite été refroidi à température ambiante et les composés volatils formés ont été éliminés sous vide. La thiolactone **TL9** ainsi obtenue sous la forme d'une huile jaune a ensuite été purifiée sur une colonne chromatographique de silice (éluant acétate d'éthyle/hexane : 8:2 (v:v)).

On a ainsi obtenu 0,877 g de **TL23** sous la forme d'une poudre blanche (rendement 75 %).
RMN ¹H (300,13 MHz, CDCl₃, 298K) : δ (ppm) 3,78-3,62 (m, 2H, CHS) ; 2,75-2,43 (m, 2H, C(O)CH(CH₃)CH₂CH) ; 2,20-1,99 (m, 2H, C(O)CH(CH₃)CH₂CH) ; 1,84-1,63 (m, 4H, CH₂CH₂CH₂) ; 1,56-1,38 (m, 5H, CHCH₂CH₂, CHCH₂CH₂) ; 1,17-1,13 (m, 6H, CH₃).
RMN ¹³C{¹H} (282,38 MHz, CDCl₃, 298K) : δ (ppm) 210,74-209,61(C=O) ; 48,78-45,42 ((CH₃)CHCH₂CH₂CHS) ; 41,30-39,48 ((CH₃)CHCH₂CH₂CHS) ; 36,59-36,13 (CHCH₂CH₂CH₂) ; 28,26-27,94 (CHCH₂CH₂CH₂) ; 15,38-14,45 (CH₃).

### Exemple 11 : Synthèse de la 5-(9-hydroxynonyl)-3-méthyldihydrothiophen-2(3H)-one (TL 24) selon le procédé conforme à l'invention

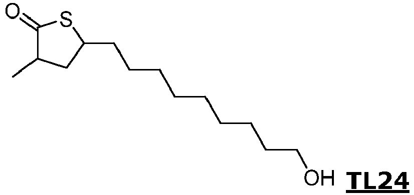

### 1) Première étape : Préparation du méthyl 13-hydroxy-2-méthyl-4-((((3-méthylbutan-2-yl)oxy)carbonothioyl)thio)tridécanoate (Xanthate XA2HN)

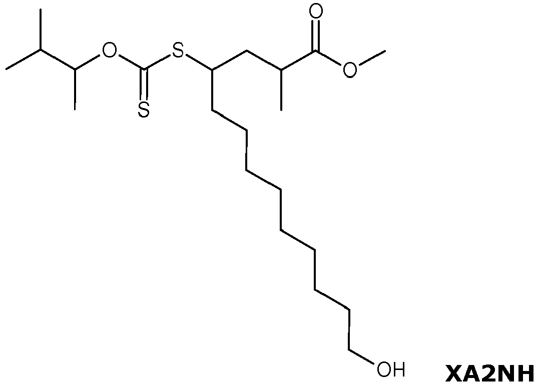

### 1.1) Sous-étape 1 : Préparation de l'O-1,2-diméthylpropyl S-(1-méthoxycarbonyl)éthyl xanthate (XA2)

0,21 mole (35,07 g) de 2-bromopropionate de méthyle (Sigma-Aldrich) a été ajoutée à une suspension de 40,5 g (0,2 mol) du composé **XA0** obtenu ci-dessus à l'étape 1.1) de l'exemple 1, dans 200 ml d'acétone (Sigma-Aldrich), dans un bain de glace (réaction très exothermique). Une fois l'ajout terminé, le milieu réactionnel a été agité à température ambiante pendant 3 heures puis filtré. Le filtrat a été concentré sous vide afin d'obtenir le produit attendu **XA2** sous la forme d'une huile jaune (43 g, rendement 86 %) qui sera utilisé dans l'étape suivante sans purification.
RMN ¹H (300,13 MHz, CDCl₃, 298K) δ 5,51 (p, *³J_{H,H}* = 6,3 Hz, 1H, (CH₃)₂CHCH(O)CH₃) ; 4,42-4,30 (m, 1H, CH(CH₃)CO₂CH₃) ; 3,73 (s, 3H, CO₂CH₃) ; 2,09-1,88 (m, 1H, (CH₃)₂CHCH(O)CH₃) ; 1,56-1,52 (m, *³J_{H,H} =* 7,4 Hz, 3H, CH(CH₃)CO₂CH₃) ; 1,29-1,25 (m, *³J_{H,H}* = 6,4 Hz, 3H, (CH₃)₂CHCH(O)CH₃) ; 1,02-0,91 (m, 6H, (CH₃)₂CHCH(O)CH₃).
RMN ¹³C{¹H} (75,47 MHz, CDCl₃, 298K) δ 211,4 ; 211,4 (C=S) ; 172,0 (CO₂CH₃) ; 86,2-86,1 ((CH₃)₂CHCH(O)CH₃) ; 52,8 (CO₂CH₃) ; 46,5 (CH(CH₃)CO₂CH₃) ; 32,7 ((CH₃)₂CHCH(O)CH₃) ; 18,2-17,8 ((CH₃)₂CHCH(O)CH₃) ; 17,0-16,9 (CH(CH₃)CO₂CH₃) ; 15,8-15,7 ((CH₃)₂CHCH(O)CH₃) ppm.
IR : 1738,5 cm⁻¹ (C=O) ; 1046,2 cm⁻¹ (C=S)

### 1.2) Sous étape 2 : Préparation du méthyl 13-hydroxy-2-méthyl-4-((((3-méthylbutan-2-yl)oxy)carbonothioyl)thio)tridécanoate (XA2HN)

Le xanthate **XA2HN** a été préparé selon le même mode opératoire que celui utilisé ci-dessus à l'étape 1.2) de l'exemple 2 pour la préparation du xanthate **XA2AP,** mais en en utilisant 5,01 g (20 mmol) du xanthate **XA2** préparé à l'étape précédente, 3,05 g (18 mmol) de undécène-1-ol (Sigma Aldrich) et 1 g (26 mmol) de (LPO).

On a ainsi obtenu 5,8 g de **XA2HN** (huile jaune ; éluant hexane/acétate d'éthyle (7:3 ; v:v), sous la forme d'un racémate (rendement 77 %).
RMN ¹H (300,13 MHz, CDCl₃, 298K) δ = 5,57-5,48 (m, 1H, (CH₃)₂CHCH(O)CH₃) ; 3,75-3,71 (m, 1H, SCHCH₂CH(CH₃)CO₂CH₃) ; 3,66-3,64 (m, 3H, CO₂CH₃) ; 3,62,-3,57 (t, 2H, CH₂(CH₂)₆CH₂CH₂OH) ; 2,72-2,65 (m, 1H, SCHCH₂CH(CH₃)CO₂CH₃) ; 2,12-1,91 (m, 2H, SCHCH₂CH(CH₃)CO₂CH₃) ; 1,80 (s, 1H, OH) ; 1,73-1,48 (m, 5H, (CH₃)₂CHCH(O)CH₃ ; CH₂(CH₂)₆CH₂CH₂OH) ; 1,38-1,24 (m, 15H, CH(CH₃)CO₂CH₃ ; CH₂(CH₂)₆CH₂CH₂OH) ; 1,17-1,15 (m, 3H, (CH₃)₂CHCH(O)CH₃) ; 0,94-0,92 (m, 6H, (CH₃)₂CHCH(O)CH₃).
RMN ¹³C{¹H} (75,47 MHz, CDCl₃, 298K) *δ* = 213,80-213,70 (C=S) ; 176,65 (CO₂CH₃) ; 85,40 ((CH₃)₂CHCH(O)CH₃) ; 62,90 (CH₂(CH₂)₆CH₂CH₂OH) ; 51,58 (CO₂CH₃) ; 49,14-48,59 (SCHCH₂CH(CH₃)CO₂CH₃) ; 38,16-37,54 (SCHCH₂CH(CH₃)CO₂CH₃) ; 37,50-37,13 (SCHCH₂CH(CH₃)CO₂CH₃) ; 32,70-32,69 ((CH₃)₂CHCH(O)CH₃ ; CH₂(CH₂)₆CH₂CH₂OH) ; 29,48-25,72 (CH₂(CH₂)₆CH₂CH₂OH) ; 18,25-18,23 ((CH₃)₂CHCH(O)CH₃) ; 17,02-15,79 ((CH₃)₂CHCH(O)CH₃) ; CH(CH₃)CO₂CH₃).

### 2) Deuxième étape : Préparation de la 5-(9-hydroxynonyl)-3-methyldihydrothiophen-2(3H)-one (TL24)

La thiolactone **TL24** a été préparée selon le même mode opératoire que celui utilisé ci-dessus à l'exemple 2, étape 3) pour la préparation de la thiolactone **TL2,** mais en utilisant 3,4 g (8 mmoles) du xanthate **XA2HN** préparé ci-dessus à l'étape précédente au lieu du xanthate **XA2AP.**

On a ainsi obtenu 1,89 g de thiolactone **TL24** (rendement 91 %) sous la forme d'un liquide incolore (éluant : hexane/acétate d'éthyle : 5:5 ; v:v).
RMN ¹H (300 MHz, CDCl₃, 298K) *δ* = 3,74-3,63 (m, 1H, SCHCH₂CH) ; 3,58-3,53 (t, 2H, SCHCH₂(CH₂)₆CH₂CH₂OH) ; 2,72-2,43 (m, 1,5H, C(O)CH(CH₃)CH₂,) ; 2,24 (s, 1H, OH) ; 2,15-1,95 (m, 1H, SCHCH₂CH) ; 1,74-1,60 (m, 2H, CH₂(CH₂)₆CH₂CH₂OH) ; 1,52-1,42 (m, 2,5H, SCHCH₂CH, CH₂(CH₂)₆CH₂CH₂OH) ; 1,37-1,20 (m, 12H, CH₂(CH₂)₆CH₂CH₂OH) ; 1,12-1,09 (m, 3H, C(O)CH(CH₃)CH₂).
RMN ¹³C{¹H} (75,47 MHz, CDCl₃, 298K) *δ* = 211,34-210,18 (C=O) ; 62,78 (CH₂(CH₂)₆CH₂CH₂OH) ; 48,81-45,50 (SCHCH₂CH, C(O)CH(CH₃)CH₂) ; 41,37-39,52 (SCHCH₂(CH₂)₆CH₂CH₂OH) ; 36,71-36,36 (C(O)CH(CH₃)CH₂) ; 32,70 (SCHCH₂(CH₂)₆CH₂CH₂OH) ; 29,45-25,73 (SCHCH₂(CH₂)₆CH₂CH₂OH) ; 15,38-14,42 (C(O)CH(CH₃)CH₂).

### Exemple 12 : Synthèse de la 5-(9-bromononyl)-3-méthyldihydrothiophèn-2(3H)-one (TL25) selon le procédé conforme à l'invention

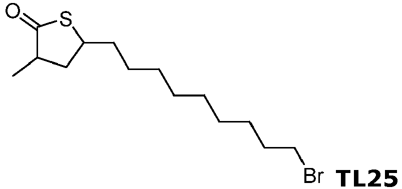

### 1) Première étape : Préparation du méthyl 13-bromo-2-méthyl-4-((((3-méthylbutan-2-yl)oxy)carbonothioyl)thio)tridécanoate (Xanthate XA2BN)

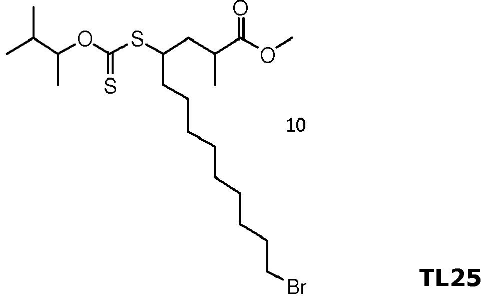

### 1.1) Sous étape 1 : Préparation de l'O-1,2-diméthylpropyl S-(1-méthoxycarbonyl)éthyl xanthate (XA2)

0,21 mole (35,07 g) de 2-bromopropionate de méthyle (Sigma-Aldrich) a été ajoutée à une suspension de 40,5 g (0,2 mol) du composé **XA0** obtenu ci-dessus à l'étape 1.1) de l'exemple 1, dans 200 ml d'acétone (Sigma-Aldrich), dans un bain de glace (réaction très exothermique). Une fois l'ajout terminé, le milieu réactionnel a été agité à température ambiante pendant 3 heures puis filtré. Le filtrat a été concentré sous vide afin d'obtenir le produit attendu **XA2** sous la forme d'une huile jaune (43 g, rendement 86 %) qui sera utilisé dans l'étape suivante sans purification.
RMN ¹H (300,13 MHz, CDCl₃, 298K) δ 5,51 (p, *³J_{H,H}* = 6,3 Hz, 1H, (CH₃)₂CHCH(O)CH₃) ; 4,42-4,30 (m, 1H, CH(CH₃)CO₂CH₃) ; 3,73 (s, 3H, CO₂CH₃) ; 2,09-1,88 (m, 1H, (CH₃)₂CHCH(O)CH₃) ; 1,56-1,52 (m, *³J_{H,H}* = 7,4 Hz, 3H, CH(CH₃)CO₂CH₃) ; 1,29-1,25 (m, *³J_{H,H}* = 6,4 Hz, 3H, (CH₃)₂CHCH(O)CH₃) ; 1,02-0,91 (m, 6H, (CH₃)₂CHCH(O)CH₃).
RMN ¹³C{¹H} (75,47 MHz, CDCl₃, 298K) δ 211,4 ; 211,4 (C=S) ; 172,0 (CO₂CH₃) ; 86,2-86,1 ((CH₃)₂CHCH(O)CH₃) ; 52,8 (CO₂CH₃) ; 46,5 (CH(CH₃)CO₂CH₃) ; 32,7 ((CH₃)₂CHCH(O)CH₃) ; 18,2-17,8 ((CH₃)₂CHCH(O)CH₃) ; 17,0-16,9 (CH(CH₃)CO₂CH₃) ; 15,8-15,7 ((CH₃)₂CHCH(O)CH₃) ppm.
IR : 1738,5 cm⁻¹ (C=O) ; 1046,2 cm⁻¹ (C=S)

### 1.2) Sous étape 2 : Préparation du méthyl 13-bromo-2-méthyl-4-((((3-méthylbutan-2-yl)oxy)carbonothioyl)thio)tridécanoate (XA2BN)

Le xanthate **XA2BN** a été préparé selon le même mode opératoire que celui utilisé ci-dessus à l'étape 1.2) de l'exemple 2 pour la préparation du xanthate **XA2AP,** mais en en utilisant 1,01 g (4 mmol) du xanthate **XA2** préparé à l'étape précédente, 0,86 g (3,7 mmol) de bromo-11-undécène (Alfa-Aesar) et 0,22 g (0,5 mmol) de **(LPO).**

On a ainsi obtenu 1,41 g de **XA2BN** (huile jaune ; éluant hexane/acétate d'éthyle (95:5 ; v:v), sous la forme d'un racémate (rendement 80 %).
RMN ¹H (300,13 MHz, CDCl₃, 298K) *δ* = 5,59-5,52 (m, 1H, (CH₃)₂CHCH(O)CH₃) ; 3,78-3,77 (m, 1H, SCHCH₂CH(CH₃)CO₂CH₃) ; 3,66-3,64 (m, 3H, CO₂CH₃) ; 3,41-3,36 (t, 2H, CH₂(CH₂)₆CH₂CH₂Br) ; 2,70-2,63 (m, 1H, SCHCH₂CH(CH₃)CO₂CH₃) ; 2,08-1,91 (m, 2H, SCHCH₂CH(CH₃)CO₂CH₃) ; 1,73-1,48 (m, 5H, (CH₃)₂CHCH(O)CH₃ ; CH₂(CH₂)₆CH₂CH₂Br) ; 1,38-1,24 (m, 15H, CH(CH₃)CO₂CH₃, CH₂(CH₂)₆CH₂CH₂Br) ; 1,17-1,15 (m, 3H, (CH₃)₂CHCH(O)CH₃) ; 0,94-0,92 (m, 6H, (CH₃)₂CHCH(O)CH₃).
RMN ¹³C{¹H} (75,47 MHz, CDCl₃, 298K) *δ* = 213,80-213,70 (C=S) ; 176,65 (CO₂CH₃) ; 85,40 ((CH₃)₂CHCH(O)CH₃) ; 51,58 (CO₂CH₃) ; 49,14-48,59 (SCHCH₂CH(CH₃)CO₂CH₃) ; 38,16-37,54 (SCHCH₂CH(CH₃)CO₂CH₃) ; 37,50-37,13 (SCHCH₂CH(CH₃)CO₂CH₃) ; 34,90 (CH₂(CH₂)₆CH₂CH₂Br) ; 32,70-32,69 ((CH₃)₂CHCH(O)CH₃ ; CH₂(CH₂)₆CH₂CH₂Br) ; 29,48-25,72 (CH₂(CH₂)₆CH₂CH₂Br) ; 18,25-18,23 ((CH₃)₂CHCH(O)CH₃) ; 17,02-15,79 ((CH₃)₂CHCH(O)CH₃) ; CH(CH₃)CO₂CH₃).

### 2) Deuxième étape : Préparation de la 5-(9-bromononyl)-3-méthyldihydrothiophen-2(3H)-one (TL25)

La thiolactone **TL25** a été préparée selon le même mode opératoire que celui utilisé ci-dessus à l'exemple 2, étape 3) pour la préparation de la thiolactone **TL2,** mais en utilisant 0,59 g (1,2 mmoles) du xanthate **XA2BN** préparé ci-dessus à l'étape précédente au lieu du xanthate **XA2AP.**

On a ainsi obtenu 0,202 g de thiolactone **TL25** (rendement 52 %) sous la forme d'un liquide incolore (éluant : hexane/acétate d'éthyle : 9:1 ; v:v).
RMN ¹H (300 MHz, CDCl₃, 298K) *δ* = 3,79-3,66 (m, 1H, SCHCH₂CH) ; 3,42-3,38 (t, 2H, SCHCH₂(CH₂)₆CH₂CH₂Br) ; 2,74-2,49 (m, 1,5H, C(O)CH(CH₃)CH₂) ; 2,18-2,02 (m, 1H, SCHCH₂CH) ; 1,89-1,59 (m, 4H, CH₂(CH₂)₆CH₂CH₂Br) ; 1,37-1,20 (m, 13H, CH₂(CH₂)₆CH₂CH₂Br) ; 1,12-1,09 (m, 3H, C(O)CH(CH₃)CH₂).
RMN ¹³C{¹H} (75,47 MHz, CDCl₃, 298K) *δ* = 211,05-209,91 (C=O) ; 48,82-45,0 (SCHCH₂CH, C(O)CH(CH₃)CH₂) ; 41,40 (CH₂(CH₂)₆CH₂CH₂Br) ; 39,56-39,52 (SCHCH₂(CH₂)₆CH₂CH₂Br) ; 36,76-36,40 (C(O)CH(CH₃)CH₂) ; 32,70 (SCHCH₂(CH₂)₆CH₂CH₂Br) ; 29,45-25,73 (SCHCH₂(CH₂)₆CH₂CH₂Br) ; 15,38-14,42 (C(O)CH(CH₃)CH₂).

### Exemple 13 : Synthèse de la 5,5'-(éthane-1,2-diyl)bis(3-méthyldihydrothiophèn-2(3H)-one) (TL 26) selon le procédé conforme à l'invention

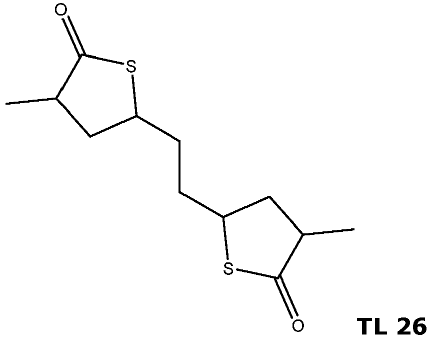

### 1) Première étape : Préparation du mono-adduit de formule (IVa) ; XA2ED

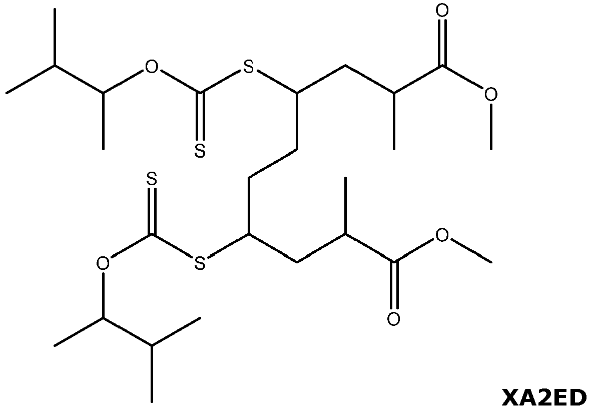

Le mono-adduit **XA2ED** a été préparé selon le mode opératoire utilisé ci-dessus à l'étape 1.2) de l'exemple 2 pour la préparation du mono-adduit **XA2AP,** mais en utilisant 8,01 g (3,2 10⁻² mol) de xanthate **XA2** tel que préparé à l'étape 1.2) de l'exemple 2, 1,25 g (1,5 10⁻² mol) de 1,5-hexadiène (Aldrich), 1,77 g de **LPO,** et un solvant de réaction composé de 6 ml de toluène.

Le mono-adduit **XA2ED** obtenu sous la forme d'une huile visqueuse jaune a été purifié par chromatographie sur silice (éluant hexane/acétate d'éthyle : 95:5, v:v).

On a ainsi obtenu 3,79 g de **XA2ED** (rendement 73%).
RMN ¹H (300,13 MHz, CDCl₃, 298K): δ (ppm) 5,47-5,46 (s, 2H, (CH₃)₂CHCH(O)CH₃) ; 3,77-3,78 (m, 2H, SCHCH₂CH(CH₃)) ; 3,66-3,64 (m, 6H, CO₂CH₃) ; 2,73-2,61 (2H, m, SCHCH₂CH(CH₃)) ; 2,15-1,91 (m, 4H, SCHCH₂CH(CH₃)) ; 1,73-1,49 (m, 6H, (CH₃)₂CHCH(O)CH₃ et SCH(CH₂CH₂)CH₂CH(CH₃)) ; 1,28-1,23 (d, 3H, SCHCH₂CH(CH₃)) ; 1,17-1,15 (d, 3H, (CH₃)₂CHCH(O)CH₃) ; 0,94-0,92 (m, 6H, (CH₃)₂CHCH(O)CH₃).

### 2) Deuxième étape : Préparation de la_5,5'-(éthane-1,2-diyl)bis(3-méthyldihydrothiophèn-2(3H)-one) (TL26)

3,36 g (5,7 mmoles) de **XA2ED** obtenu ci-dessus à l'étape précédente ont été placés dans un tube de Schlenk fermé sous vide et portés à une température de 190°C pendant 48 heures. Le mélange réactionnel a ensuite été refroidi à température ambiante et les composés volatils formés ont été éliminés sous vide. La thiolactone **TL26** ainsi obtenue sous la forme d'une huile jaune a ensuite été purifiée sur une colonne chromatographique de silice (éluant acétate d'éthyle/hexane : 6:4 (v:v)).

On a ainsi obtenu 1,2 g de **TL26** sous la forme d'une poudre blanche (rendement 84%).
RMN ¹H (300,13 MHz, CDCl₃, 298K) : δ (ppm) 3,78-3,62 (m, 2H, CHS) ; 2,75-2,43 (m, 2H, C(O)CH(CH₃)CH₂CH) ; 2,20-1,99 (m, 2H, C(O)CH(CH₃)CH₂CH) ; 1,56-1,38 (m, 5H, CHCH₂CH₂, CHCH₂CH₂) ; 1,17-1,13 (m, 6H, CH₃).
RMN ¹³C{¹H} (282,38 MHz, CDCl₃, 298K) : δ (ppm) 210,74-209,61(C=O) ; 48,78-45,42 ((CH₃)CHCH₂CH₂CHS) ; 41,30-39,48 ((CH₃)CHCH₂CH₂CHS) ; 36,59-36,13 (CHCH₂CH₂CH₂) ; 15,38-14,45 (CH₃).

### Exemple 14 : Synthèse de la 5,5'-(hexane-1,6-diyl)bis(3-méthyldihydrothiophen-2(3H)-one) (TL27) selon le procédé conforme à l'invention

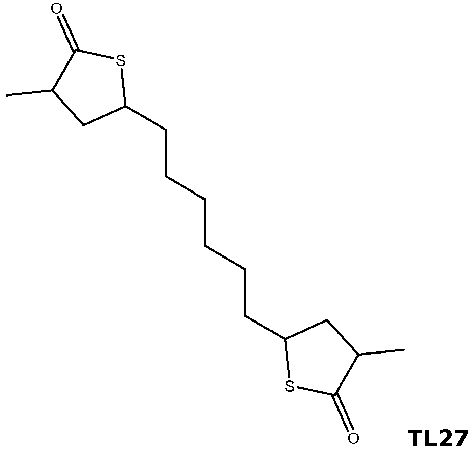

### 1) Première étape : Préparation d'un mono-adduit de formule (IVa) ; XA2HD

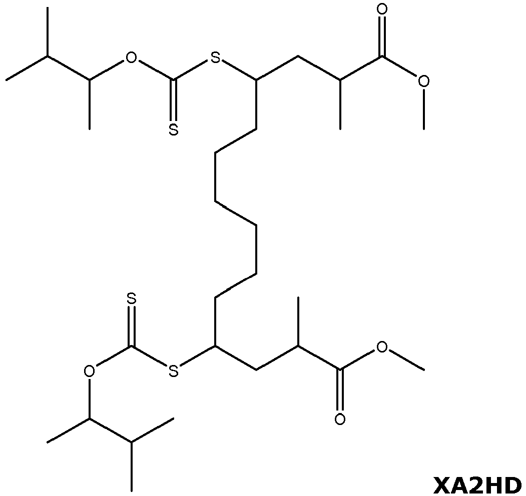

Le mono-adduit **XA2HD** a été préparé selon le mode opératoire utilisé ci-dessus à l'étape 1.2) de l'exemple 2 pour la préparation du mono-adduit **XA2AP,** mais en utilisant 9,07 g (3,6 10⁻² mol) de xanthate **XA2** tel que préparé à l'étape 1.2) de l'exemple 2, 2,38 g (1,5 10⁻² mol) de 1,9-decadiène (Aldrich), 2,03 g de **LPO,** et un solvant de réaction composé de 7 ml de toluène.

Le mono-adduit **XA2HD** obtenu sous la forme d'une huile visqueuse jaune a été purifié par chromatographie sur silice (éluant hexane/acétate d'éthyle : 90:10, v:v).

On a ainsi obtenu 7,37 g de **XA2HD** (rendement 68%).
RMN ¹H (300,13 MHz, CDCl₃, 298K) : δ (ppm) 5,58-5,49 (s, 2H, (CH₃)₂CHCH(O)CH₃) ; 3,77-3,78 (m, 2H, SCHCH₂CH(CH₃)) ; 3,66-3,64 (m, 6H, CO₂CH₃) ; 2,73-2,61 (2H, m, SCHCH₂CH(CH₃)) ; 2,15-1,91 (m, 4H, SCHCH₂CH(CH₃)) ; 1,73-1,28 (m, 14H, (CH₃)₂CHCH(O)CH₃ et SCH(CH₂CH₂CH₂CH₂CH₂CH₂)CHSH) et (CH₃)₂CHCH(O)CH₃); 1,28-1,23 (d, 3H, SCHCH₂CH(CH₃)) ; 1,17-1,15 (d, 3H, (CH₃)₂CHCH(O)CH₃) ; 0,94-0,92 (m, 6H, (CH₃)₂CHCH(O)CH₃).

### 2) Deuxième étape : Préparation de la_5,5'-(hexane-1,6-diyl)bis(3-méthyldihydrothiophèn-2(3H)-one) (TL27)

6,56 g (10,2 mmoles) de **XA2HD** obtenu ci-dessus à l'étape précédente ont été placés dans un tube de Schlenk fermé sous vide et portés à une température de 190°C pendant 48 heures. Le mélange réactionnel a ensuite été refroidi à température ambiante et les composés volatils formés ont été éliminés sous vide. La thiolactone **TL27** ainsi obtenue sous la forme d'une huile jaune a ensuite été purifiée sur une colonne chromatographique de silice (éluant acétate d'éthyle/hexane : 7:3 (v:v)).

On a ainsi obtenu 2,48 g de **TL27** sous la forme d'une poudre blanche (rendement 77%).
RMN ¹H (300,13 MHz, CDCl₃, 298K) : δ (ppm) 3,78-3,62 (m, 2H, CHS) ; 2,75-2,43 (m, 2H, C(O)CH(CH₃)CH₂CH) ; 2,20-1,99 (m, 2H, C(O)CH(CH₃)CH₂CH) ; 1,78-1,64 (m, 4H, SCH(CH₂CH₂CH₂CH₂CH₂CH₂)CHSH) 1,53-1,38 (m, 9H, CHCH₂CH₂, SCH(CH₂CH₂CH₂CH₂CH₂CH₂)CHSH) ; 1,17-1,13 (m, 6H, CH₃).
RMN ¹³C{¹H} (282,38 MHz, CDCl₃, 298K) : δ (ppm) 210,74-209,61(C=O) ; 48,78-45,42 ((CH₃)CHCH₂CHS) ; 41,30-39,48 ((CH₃)CHCH₂CHS) ; 36,71-36,36 (SCH(CH₂CH₂CH₂CH₂CH₂CH₂)CHSH) ; 28,34-28,16 (SCH(CH₂CH₂CH₂CH₂CH₂CH₂)CHSH) ; 15,38-14,45 (CH₃).

## Revendications

1. Procédé de préparation de thiolactones substituées de formule (I) suivante : 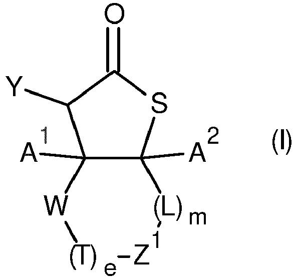 dans laquelle :
- A¹ et A², identiques ou différents, représentent un atome d'hydrogène ou un atome de fluor,
- Y représente un atome d'hydrogène ou un groupe choisi parmi les groupes alkyle, hydroxyalkyle, aryle et cyano, ou une chaîne polymère ;
- L est une chaine alkylène linéaire pouvant être interrompue par un ou plusieurs atomes d'oxygène, ladite chaine ayant de 1 à 12 atomes de carbone,
- m est un nombre entier égal à 0 ou 1,
- T représente CH₂, -O- ou -NR⁶- dans lequel R⁶ représente un atome d'hydrogène ou un radical alkyle, aryle ou aralkyle éventuellement substitué par un groupement choisi parmi les groupes : maléimide, un groupement de formule : 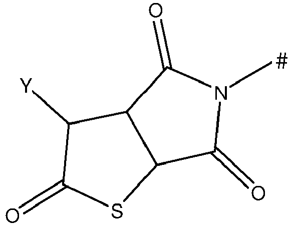 dans lequel le sigle # est le point d'attache dudit groupement à R⁶ et dans lequel Y à la même signification que celle choisie pour le radical Y de la formule (I), OH ; P(O)(OR⁷)(OR^{7'}) dans lequel les radicaux R⁷ et R^{7'}, identiques ou différents, représentent un atome d'hydrogène ou un radical alkyle ; CₙF₂ₙ₊₁ dans lequel n est un nombre entier allant de 1 à 20 ; SiR⁸ₚ(OR⁹)₃₋ₚ dans lequel les radicaux R⁸ et R⁹, identiques ou différents, représentent un atome d'hydrogène ou un radical alkyle et p est un nombre entier égal à 0, 1 ou 2 ; BF₃M⁺ dans lequel M = K ou Na ; B(OR¹⁰)₂ dans lequel les deux radicaux R¹⁰, identiques ou différents, représentent un atome d'hydrogène, un radical alkyle ou forment un cycle carboné avec les deux atomes d'oxygène auxquels ils sont liés ; OR¹¹ dans lequel R¹¹ représente un atome d'hydrogène ou un radical alkyle, aryle ou aralkyle ; O(C=O)R¹² dans lequel R¹² représente un atome d'hydrogène ou un radical alkyle, aryle ou aralkyle ; O(C=O)OR¹³ dans lequel R¹³ représente un atome d'hydrogène ou un radical alkyle, aryle ou aralkyle ; N⁺R¹⁴R^{14'}R^{14"}A⁻ dans lequel les radicaux R¹⁴, R^{14'} et R^{14"}, identiques ou différents, représentent un atome d'hydrogène ou un radical alkyle, aryle ou aralkyle et A représente un atome de chlore ou de brome ; NR^{15'}(C=O)R¹⁵ dans lequel les radicaux R¹⁵ et R^{15'}, identiques ou différents, représentent un atome d'hydrogène ou un radical alkyle ou aryle ou sont reliés entre eux et forment un cycle tel qu'un cycle pyrrolidone ou caprolactame ; NR^{16'}(C=O)OR¹⁶ dans lequel R¹⁶ et R^{16'}, identiques ou différents, représentent un atome d'hydrogène ou un radical alkyle, aryle ou aralkyle ; CN ; un atome d'halogène choisi parmi Cl, F, et Br ; NCS ; OCH₂-époxy ; COOR¹⁷ dans lequel R¹⁷ représente un atome d'hydrogène, un radical alkyle, aryle ou aralkyle ; CONR¹⁸R^{18'} dans lequel R¹⁸ et R^{18'}, identiques ou différentes, représentent un atome d'hydrogène ou un radical alkyle ou aryle ; SO₂R¹⁹ dans lequel R¹⁹ représente un radical alkyle ou aryle ; azoture N₃ et alcyne,
- e est un nombre entier égal à 0 ou 1,
Etant entendu que :
1) lorsque A¹ = A² = H et que e = 0, alors W représente un atome d'hydrogène et Z¹ représente un groupe choisi parmi les groupes alkyle ; aryle ; P(O)(OR⁷)(OR^{7'}) dans lequel les radicaux R⁷ et R^{7'}, identiques ou différents, représentent un atome d'hydrogène ou un radical alkyle ; CₙF₂ₙ₊₁ dans lequel n est un nombre entier allant de 1 à 20 ; SiR⁸ₚ(OR⁹)₃₋ₚ dans lequel les radicaux R⁸ et R⁹, identiques ou différents, représentent un atome d'hydrogène ou un radical alkyle et p est un nombre entier égal à 0, 1 ou 2 ; BF₃M⁺ dans lequel M = K ou Na ; B(OR¹⁰)₂ dans lequel les deux radicaux R¹⁰, identiques ou différents, représentent un atome d'hydrogène, un radical alkyle ou forment un cycle carboné avec les deux atomes d'oxygène auxquels ils sont liés ; OR¹¹ dans lequel R¹¹ représente un atome d'hydrogène ou un radical alkyle, aryle ou aralkyle ; O(C=O)R¹² dans lequel R¹² représente un atome d'hydrogène ou un radical alkyle, aryle ou aralkyle ; O(C=O)OR¹³ dans lequel R¹³ représente un atome d'hydrogène ou un radical alkyle, aryle ou aralkyle ; N⁺R¹⁴R^{14'}R^{14"}A⁻ dans lequel les radicaux R¹⁴, R^{14'} et R^{14"}, identiques ou différents, représentent un atome d'hydrogène ou un radical alkyle, aryle ou aralkyle et A représente un atome de chlore ou de brome ; NR^{15'}(C=O)R¹⁵ dans lequel les radicaux R¹⁵ et R^{15'}, identiques ou différents, représentent un atome d'hydrogène ou un radical alkyle ou aryle ou sont reliés entre eux et forment un cycle tel qu'un cycle pyrrolidone ou caprolactame ; NR^{16'}(C=O)OR¹⁶ dans lequel R¹⁶ et R^{16'}, identiques ou différents, représentent un atome d'hydrogène ou un radical alkyle, aryle ou aralkyle ; CN ; un atome d'halogène choisi parmi Cl, F, et Br ; NCS ; OCH₂-époxy ; COOR¹⁷ dans lequel R¹⁷ représente un atome d'hydrogène, un radical alkyle, aryle ou aralkyle ; CONR¹⁸R^{18'} dans lequel R¹⁸ et R^{18'}, identiques ou différentes, représentent un atome d'hydrogène ou un radical alkyle ou aryle ; SO₂R¹⁹ dans lequel R¹⁹ représente un radical alkyle ou aryle ; azoture N₃ et alcyne ; et un cycle thiolactone de formule 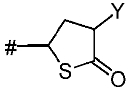 dans laquelle le sigle # est le point d'attache du cycle thiolactone à L et dans laquelle Y à la même signification que celle choisie pour le radical Y de la formule (I),
2) lorsque A¹ = A² = H, m = 1 et que e = 0, alors W représente un atome d'hydrogène et Z¹ peut en outre représenter un atome d'hydrogène ;
3) lorsque A¹ = A² = H, e = 1 et que m = 0, alors Z¹ et W sont identiques et représentent -CH₂- ou CO ;
4) lorsque A¹ = A² = H, e = 1, m = 1 et que T= CH₂ alors Z¹ = W = -CH₂,
5) lorsque A¹ = A² = F, e = 0 et que m = 0, alors W représente un atome de fluor et Z¹ = F ou représente une chaine OC_{q}F_{2q+1} linéaire ou ramifiée dans laquelle q est un nombre entier allant de 1 à 5, [OCF₂CF(CF₃)]ᵣOC₃F₇ avec r = un nombre entier allant de 0 à 20, OC₂F₄SO₂F, OCF₂CF(CF₃)OC₂F₄SO₂F, ou OCF₂CF(CF₃)OC₂F₄CO₂CH₃ ; et
6) lorsque A¹ = A² = F, e = 1 et que m = 0, alors W = Z¹ = CF₂ et T = (CF₂)₅ avec s = un nombre entier allant de 1 à 5 ;
7) lorsque A¹ = H, A² = F, et que e = m = 0, alors W représente un atome d'hydrogène et Z¹ = F ou CₙF₂ₙ₊₁ dans lequel n est un nombre entier allant de 1 à 20 ;
ledit procédé étant **caractérisé en ce qu'**il comprend au moins les étapes suivantes :
1) une étape au cours de laquelle on fait réagir, en présence d'un amorceur radicalaire, un xanthate de formule (II) suivante : 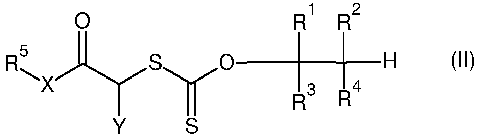 dans laquelle :
- R¹, R², R³ et R⁴, identiques ou différents, représentent un atome d'hydrogène ou un groupe choisi parmi les groupes alkyle, acyle, aryle, alcène, alcyne, cycloalkyle, hétérocycloalkyle saturé ou insaturé, hétérocycloaryle, et les chaines de polymères, étant entendu que les radicaux R¹, R², R³ et R⁴ peuvent également former ensemble un groupement cycloalkyle ou hétérocycloalkyle saturé, insaturé ou aromatique ; étant entendu qu'au moins un des radicaux R¹ et R³ est différent d'un atome d'hydrogène ;
- Y a la même signification que dans la formule (I) ci-dessus,
- X représente NR²⁰ dans lequel R²⁰ représente un atome d'hydrogène ou un radical alkyle, ou -O-,
- R⁵ est choisi parmi un groupe alkyle, acyle, aryle, aralkyle, cycloalkyle ou hétérocycloalkyle saturé, insaturé ou aromatique,
avec un monomère comportant une insaturation éthylénique de formule (III) suivante : 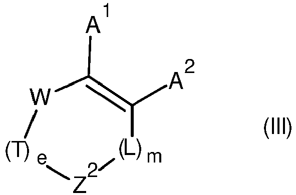 dans laquelle :
- A¹ et A², identiques ou différents, représentent un atome d'hydrogène ou un atome de fluor,
- L, m, T et e ont la même signification que dans la formule (I) ci-dessus ;
Etant entendu que :
1) lorsque A¹ = A² = H et que e = 0, alors W représente un atome d'hydrogène et Z² représente un groupe choisi parmi les groupes alkyle ; aryle ; P(O)(OR⁷)(OR^{7'}) dans lequel les radicaux R⁷ et R^{7'}, identiques ou différents, représentent un atome d'hydrogène ou un radical alkyle ; CₙF₂ₙ₊₁ dans lequel n est un nombre entier allant de 1 à 20 ; SiR⁸ₚ(OR⁹)₃₋ₚ dans lequel les radicaux R⁸ et R⁹, identiques ou différents, représentent un atome d'hydrogène ou un radical alkyle et p est un nombre entier égal à 0, 1 ou 2 ; BF₃M⁺ dans lequel M = K ou Na ; B(OR¹⁰)₂ dans lequel les deux radicaux R¹⁰, identiques ou différents, représentent un atome d'hydrogène, un radical alkyle ou forment un cycle carboné avec les deux atomes d'oxygène auxquels ils sont liés ; OR¹¹ dans lequel R¹¹ représente un atome d'hydrogène ou un radical alkyle, aryle ou aralkyle ; O(C=O)R¹² dans lequel R¹² représente un atome d'hydrogène ou un radical alkyle, aryle ou aralkyle ; O(C=O)OR¹³ dans lequel R¹³ représente un atome d'hydrogène ou un radical alkyle, aryle ou aralkyle ; N⁺R¹⁴R^{14'}R^{14"}A⁻ dans lequel les radicaux R¹⁴, R^{14'} et R^{14"}, identiques ou différents, représentent un atome d'hydrogène ou un radical alkyle, aryle ou aralkyle et A représente un atome de chlore ou de brome ; NR^{15'}(C=O)R¹⁵ dans lequel les radicaux R¹⁵ et R^{15'}, identiques ou différents, représentent un atome d'hydrogène ou un radical alkyle ou aryle ou sont reliés entre eux et forment un cycle tel qu'un cycle pyrrolidone ou caprolactame ; NR^{16'}(C=O)OR¹⁶ dans lequel R¹⁶ et R^{16'}, identiques ou différents, représentent un atome d'hydrogène ou un radical alkyle, aryle ou aralkyle ; CN ; un atome d'halogène choisi parmi Cl, F, et Br ; NCS ; OCH₂-époxy ; COOR¹⁷ dans lequel R¹⁷ représente un atome d'hydrogène, un radical alkyle, aryle ou aralkyle ; CONR¹⁸R^{18'} dans lequel R¹⁸ et R^{18'}, identiques ou différentes, représentent un atome d'hydrogène ou un radical alkyle ou aryle ; SO₂R¹⁹ dans lequel R¹⁹ représente un radical alkyle ou aryle ; azoture N₃ ; alcyne et C₂H₃ ;
2) lorsque A¹ = A² = H, m = 1 et que e = 0, alors W représente un atome d'hydrogène et Z² peut en outre représenter un atome d'hydrogène,
3) lorsque A¹ = A² = H, e = 1 et que m = 0, alors Z² et W sont identiques et représentent -CH₂- ou CO ;
4) lorsque A¹ = A² = H, e = 1, m = 1 et que T= CH₂ alors Z² = W = -CH₂,
5) lorsque A¹ = A² = F, e = 0 et que m = 0, alors W représente un atome de fluor et Z² = F ou représente une chaine OC_{q}F_{2q+1} linéaire ou ramifiée dans laquelle q est un nombre entier allant de 1 à 5, [OCF₂CF(CF₃)]ᵣOC₃F₇ avec r = un nombre entier allant de 0 à 20, OC₂F₄SO₂F, OCF₂CF(CF₃)OC₂F₄SO₂F, ou OCF₂CF(CF₃)OC₂F₄CO₂CH₃ ; et
6) lorsque A¹ = A² = F, e = 1 et que m = 0, alors W = Z² = CF₂ et T = (CF₂)ₛ avec s = un nombre entier allant de 1 à 5 ;
7) lorsque A¹ = H, A² = F, et que e = m = 0, alors W représente un atome d'hydrogène et Z² = F ou CₙF₂ₙ₊₁ dans lequel n est un nombre entier allant de 1 à 20 ;
pour former un mono-adduit de formule (IV) suivante : 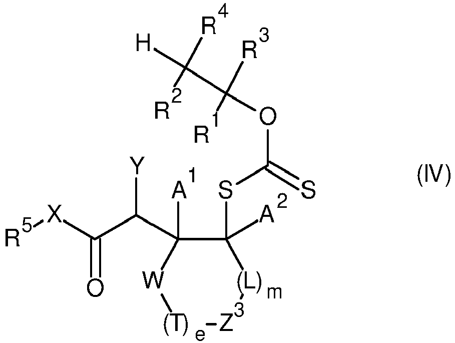 dans laquelle :
- A¹ et A², identiques ou différents représentent un atome d'hydrogène ou un atome de fluor,
- R¹, R², R³, R⁴, R⁵, X et Y ont la même signification que dans la formule (II) ci-dessus,
- L, m, T et e ont la même signification que dans la formule (I) ci-dessus,
Etant entendu que :
1) lorsque A¹ = A² = H et que e = 0, alors W représente un atome d'hydrogène et Z³ représente un groupe choisi parmi les groupes alkyle ; aryle ; P(O)(OR⁷)(OR^{7'}) dans lequel les radicaux R⁷ et R^{7'}, identiques ou différents, représentent un atome d'hydrogène ou un radical alkyle ; CₙF₂ₙ₊₁ dans lequel n est un nombre entier allant de 1 à 20 ;SiR⁸ₚ(OR⁹)₃₋ₚ dans lequel les radicaux R⁸ et R⁹, identiques ou différents, représentent un atome d'hydrogène ou un radical alkyle et p est un nombre entier égal à 0, 1 ou 2 ; BF₃M⁺ dans lequel M = K ou Na ; B(OR¹⁰)₂ dans lequel les deux radicaux R¹⁰, identiques ou différents, représentent un atome d'hydrogène, un radical alkyle ou forment un cycle carboné avec les deux atomes d'oxygène auxquels ils sont liés ; OR¹¹ dans lequel R¹¹ représente un atome d'hydrogène ou un radical alkyle, aryle ou aralkyle ; O(C=O)R¹² dans lequel R¹² représente un atome d'hydrogène ou un radical alkyle, aryle ou aralkyle ; O(C=O)OR¹³ dans lequel R¹³ représente un atome d'hydrogène ou un radical alkyle, aryle ou aralkyle ; N⁺R¹⁴R^{14'}R^{14"}A dans lequel les radicaux R¹⁴, R^{14'} et R^{14"}, identiques ou différents, représentent un atome d'hydrogène ou un radical alkyle, aryle ou aralkyle et A représente un atome de chlore ou de brome; NR^{15'}(C=O)R¹⁵ dans lequel les radicaux R¹⁵ et R^{15'}, identiques ou différents, représentent un atome d'hydrogène ou un radical alkyle ou aryle ou sont reliés entre eux et forment un cycle tel qu'un cycle pyrrolidone ou caprolactame ; NR^{16'}(C=O)OR¹⁶ dans lequel R¹⁶ et R^{16'}, identiques ou différents, représentent un atome d'hydrogène ou un radical alkyle, aryle ou aralkyle ; CN ; un atome d'halogène choisi parmi Cl, F, et Br ; NCS ; OCH₂-époxy ; COOR¹⁷ dans lequel R¹⁷ représente un atome d'hydrogène, un radical alkyle, aryle ou aralkyle ; CONR¹⁸R^{18'} dans lequel R¹⁸ et R^{18'}, identiques ou différentes, représentent un atome d'hydrogène ou un radical alkyle ou aryle ; SO₂R¹⁹ dans lequel R¹⁹ représente un radical alkyle ou aryle ; azoture N₃ ; alcyne ; et un groupement de formule (V) suivante : 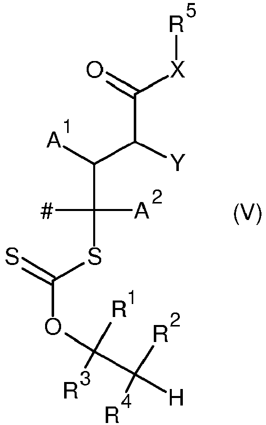 dans lequel le sigle # est le point d'attache du groupement de formule (V) à L et Y à la même signification que celle choisie pour le radical Y de la formule (IV),
2) lorsque A¹ = A² = H, m = 1 et que e = 0, alors W représente un atome d'hydrogène et Z³ peut en outre représenter un atome d'hydrogène,
3) lorsque A¹ = A² = H, e = 1 et que m = 0, alors Z³ et W sont identiques et représentent -CH₂- ou CO ;
4) lorsque A¹ = A² = H, e = 1, m = 1 et que T= CH₂ alors Z³ = W = -CH₂,
5) lorsque A¹ = A² = F, e = 0 et que m = 0, alors W représente un atome de fluor et Z³ = F ou représente une chaine OC_{q}F_{2q+1} linéaire ou ramifiée dans laquelle q est un nombre entier allant de 1 à 5, [OCF₂CF(CF₃)]ᵣOC₃F₇ avec r = un nombre entier allant de 0 à 20, OC₂F₄SO₂F, OCF₂CF(CF₃)OC₂F₄SO₂F, ou OCF₂CF(CF₃)OC₂F₄CO₂CH₃ ; et
6) lorsque A¹ = A² = F, e = 1 et que m = 0, alors W = Z³ = CF₂ et T = (CF₂)ₛ avec s = un nombre entier allant de 1 à 5 ;
7) lorsque A¹ = H, A² = F, et que e = m = 0, alors W représente un atome d'hydrogène et Z³ = F ou CₙF₂ₙ₊₁ dans lequel n est un nombre entier allant de 1 à 20 ;
puis
2) une étape de thermolyse du mono-adduit de formule (IV) obtenu ci-dessus à l'étape précédente pour former une thiolactone substituée de formule (I) correspondante.

2. Procédé selon la revendication 1, **caractérisé en ce qu'**il est mis en œuvre pour la préparation de thiolactones de formule (I) dans laquelle :
- Z¹ est un groupe choisi parmi les groupes P(O)(OR⁷)(OR^{7'}) ; CₙF₂ₙ₊₁ ; B(OR¹⁰)₂ ; OR¹¹ ; SiR⁸ₚ(OR⁹)₃₋ₚ ; NR^{15'}(C=O)R¹⁵ dans lequel R^{15'} est un atome d'hydrogène et NR^{16'}(C=O)OR¹⁶ dans lequel R^{16'} est un atome d'hydrogène, et/ou
- Y est un atome d'hydrogène ou un groupe choisi parmi un radical alkyle.

3. Procédé selon la revendication 1 ou 2, caractérisé en qu'il est mis en œuvre pour la préparation de thiolactones substituées de formule (I) dans laquelle :
- Z¹ est un groupe choisi parmi les groupes diméthylphosphonate et diéthylphosphonate ; CₙF₂ₙ₊₁ ; B(OR¹⁰)₂, OR¹¹, SiR⁸ₚ(OR⁹)₃₋ₚ, NR^{15'}(C=O)R¹⁵ dans lequel R^{15'} est un atome d'hydrogène et NR^{16'}(C=O)OR¹⁶ dans lequel R^{16'} est un atome d'hydrogène, et/ou
- Y est un atome d'hydrogène ou un groupe méthyle ou hydroxyméthyle.

4. Procédé selon l'une quelconque des revendications précédentes, **caractérisé en ce qu'**il conduit à la formation d'une thiolactone de formule (I) choisie parmi :
- le diméthyl 5-oxo-tetrahydrothiophèn-2-ylphosphonate,
- le diéthyl (4-méthyl-5-oxo-tetrahydrothiophèn-2-yl) méthylphosphonate,
- le diéthyl (5-oxo-tetrahydrothiophèn-2-yl)méthylphosphonate,
- la 3-méthyl-5-pentyl-dihydrothiophèn-2(3H)-one,
- la 5-pentyl-dihydrothiophèn-2(3H)-one,
- la 3-méthyl-5-(perfluorooctyl)dihydrothiophèn-2(3H)-one,
- la 3-méthyl-5-phényldihydro-2H-thiéno[2,3-c]pyrrole-2,4,6(3H,5H)-trione,
- la 3-méthyl-5-(perfluorobutyl)dihydrothiophèn-2(3H)-one,
- l'acide (4-méthyl-5-oxo-tetrahydrothiophèn-2-yl)phosphonique,
- l'acide ((4-méthyl-5-oxotetrahydrothiophèn-2-yl)méthyl)phosphonique,
- l'acide (5-oxo-tetrahydrothiophèn-2-yl)méthylphosphonique,
- la 3-méthyl-5-(triméthoxysilyl)dihydrothiophèn-2(3H)-one,
- la 5-(triméthoxysilyl)dihydrothiophèn-2(3H)-one,
- le tert-butyl-N-(4-méthyl-5-oxo-tetrahydrothiophèn-2-yl)carbamate,
- le tert-butyl (5-oxotetrahydrothiophèn-2-yl)carbamate,
- la 3-méthyl-5-(oxiran-2-ylméthoxy)dihydrothiophèn-2(2H)-one,
- la 5-((oxiran-2-yloxy)méthyl)dihydrothiophèn-2(3H)-one,
- la 3-méthyl-5-(4,4,5,5-tetraméthyl-1,3,2-dioxaborolan-2-yl)dihydrothiophèn-2(3H)-one,
- l'acide (5-oxo-tetrahydrothiophèn-2-yl) phosphonique,
- la 5-(4,4,5,5-tetraméthyl-1,3,2-dioxaborolan-2-yl)dihydrothiophèn-2(3H)-one,
- la 5-(perfluorooctyl)dihydrothiophèn-2(3H)-one,
- la 5-(perfluorobutyl)dihydrothiophèn-2(3H)-one,
- la dihydro-5-(3-(tétrahydro-4-méthyl-5-oxothiophén-2-yl)propyl)-3-méthylthiophén-2(3H)-one,
- la 5-(9-hydroxynonyl)3-méthyldihydrothiophèn-2(3H)-one,
- la 5-(9-bromononyl)3-méthyldihydrothiophèn-2(3H)-one,
- la 5,5'-(éthane-1,2-diyl)bis(3-méthyldihydrothiophèn-2(3H)-one, et
- la 5,5'-(hexane-1,6-diyl)bis(3-méthyldihydrothiophèn-2(3H)-one.

5. Procédé selon l'une quelconque des revendications précédentes, **caractérisé en ce que** l'étape 1) de préparation du mono-adduit de formule (IV) est réalisée sans solvant, dans l'eau ou dans un solvant organique.

6. Procédé selon l'une quelconque des revendications précédentes, **caractérisé en ce que** l'amorceur radicalaire utilisé lors de l'étape 1) est choisi parmi les peroxydes organiques, les dérivés azoïques, les couples oxydo-réducteurs générateurs de radicaux et les systèmes redox.

7. Procédé selon la revendication 6, **caractérisé en ce que** les peroxydes organiques sont choisis parmi le peroxyde de dilauroyle, le peroxyacétate t-butyle, le peroxybenzoate de t-butyle, le peroxyoctoate de t-butyle, le peroxydodécanoate de t-butyle, le peroxyisobutyrate de t-butyle, le peroxypyvalate de t-amyle, le peroxypyvalate de t-butyle, le peroxydicarbonate de di-isopropyle, le peroxydicarbonate de dicyclohexyle, le peroxyde de dicumyle, le peroxyde de dibenzoyle, le peroxydisulfate de potassium, le peroxydisulfate de sodium et le peroxydisulfate d'ammonium.

8. Procédé selon l'une quelconque des revendications précédentes, **caractérisé en ce que** les monomères de formule (III) sont des alcènes choisis parmi l'éthylène, le propylène le 1-butène, le 1-pentène, le 1-hexène, le 1-heptène, le 1-octène, le 1-nonène, le 1-décène, le perfluorohexyl éthylène et le perfluorooctyl éthylène.

9. Procédé selon l'une quelconque des revendications 1 à 7, **caractérisé en ce que** les monomères de formule (III) sont des composés allyliques choisis parmi l'alcool allylique, la N-allyl benzamide, l'éthyl N-allyl carbamate, le terbutyl N-allyl carbamate, le 2-allyl-4,4,5,5-tétraméthyl-1,3,2-dioxaborolane, le 2-allyl-6-méthyl-1,3,6,2-dioxazaborocane-4,8-dione, l'acide allylboronique, le phosphonate de diéthyl allyle, le dichlorure d'allyle phosphonique, le diméthyl allylphosphonate, le cyanure d'allyle, l'isothiocyanate d'allyle, le glycidyléther d'allyle, le benzyléther d'allyle, phényléther d'allyle, le butyléther d'allyle, l'éthyléther d'allyle, la méthylsulfone d'allyle, la phénylsulfone d'allyle, le chlorure d'allyle, le bromure d'allyle et le fluorure d'allyle.

10. Procédé selon l'une quelconque des revendications précédentes, **caractérisé en ce que** l'étape 1) est réalisée à une température variant de 10 à 140 °C.

11. Procédé selon l'une quelconque des revendications précédentes, **caractérisé en ce que** l'étape 2) de thermolyse est réalisée sans solvant.

12. Procédé selon l'une quelconque des revendications précédentes, **caractérisé en ce qu'**au moins un des groupes R¹ ou R³ est différent d'un atome d'hydrogène.

13. Thiolactones substituées de formule (I') suivante : 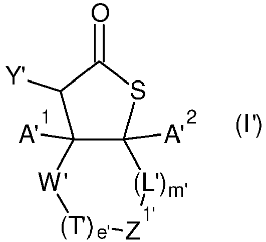 dans laquelle :
A'¹, A'², Y', Z', L', m', T' et e' peuvent respectivement prendre les mêmes significations que celles définies dans l'une quelconque des revendications 1 à 3 pour A¹, A², Y, Z¹, L, m, T et e pour les thiolactones de formule (I), sous réserve que :
- lorsque e' = 0 et que W' = H et que m' = 0 et que Y' = hydrogène, alors Z¹' est différent d'un atome d'hydrogène, d'une chaine alkyle linéaire ou d'un cycle phényle ; et
- lorsque e' = 0 et que W' = H et que m' = 0 et que Y' est un substituant possédant un atome d'azote directement lié au cycle thiolactone, alors Z¹' est différent d'un atome d'hydrogène.

14. Thiolactones substituées de formule (I') selon la revendication 13, **caractérisées en ce qu'**elles sont choisies parmi :
- le diméthyl 5-oxo-tetrahydrothiophèn-2-ylphosphonate,
- le diéthyl (4-méthyl-5-oxo-tetrahydrothiophèn-2-yl) méthylphosphonate,
- le diéthyl (5-oxo-tetrahydrothiophèn-2-yl)méthylphosphonate,
- la 3-méthyl-5-pentyl-dihydrothiophèn-2(3H)-one,
- la 5-pentyl-dihydrothiophèn-2(3H)-one,
- la 3-méthyl-5-(perfluorooctyl)dihydrothiophèn-2(3H)-one,
- la 3-méthyl-5-phényldihydro-2H-thiéno[2,3-c]pyrrole-2,4,6(3H,5H)-trione,
- la 3-méthyl-5-(perfluorobutyl)dihydrothiophèn-2(3H)-one,
- l'acide (4-méthyl-5-oxo-tetrahydrothiophèn-2-yl)phosphonique,
- l'acide ((4-méthyl-5-oxotetrahydrothiophèn-2-yl)méthyl)phosphonique,
- l'acide (5-oxo-tetrahydrothiophèn-2-yl)méthylphosphonique,
- la 3-méthyl-5-(triméthoxysilyl)dihydrothiophèn-2(3H)-one,
- la 5-(triméthoxysilyl)dihydrothiophèn-2(3H)-one,
- le tert-butyl-N-(4-méthyl-5-oxo-tetrahydrothiophèn-2-yl)carbamate,
- le tert-butyl (5-oxotetrahydrothiophèn-2-yl)carbamate,
- la 3-méthyl-5-(oxiran-2-ylméthoxy)dihydrothiophèn-2(2H)-one,
- la 5-((oxiran-2-yloxy)méthyl)dihydrothiophèn-2(3H)-one,
- la 3-méthyl-5-(4,4,5,5-tetraméthyl-1,3,2-dioxaborolan-2-yl)dihydrothiophèn-2(3H)-one,
- l'acide (5-oxo-tetrahydrothiophèn-2-yl) phosphonique,
- la 5-(4,4,5,5-tetraméthyl-1,3,2-dioxaborolan-2-yl)dihydrothiophèn-2(3H)-one,
- la 5-(perfluorooctyl)dihydrothiophèn-2(3H)-one,
- la 5-(perfluorobutyl)dihydrothiophèn-2(3H)-one,
- la dihydro-5-(3-(tétrahydro-4-méthyl-5-oxothiophén-2-yl)propyl)-3-méthylthiophén-2(3H)-one,
- la 5-(9-hydroxynonyl)3-méthyldihydrothiophèn-2(3H)-one,
- la 5-(9-bromononyl)3-méthyldihydrothiophèn-2(3H)-one,
- la 5,5'-(éthane-1,2-diyl)bis(3-méthyldihydrothiophèn-2(3H)-one, et
- la 5,5'-(hexane-1,6-diyl)bis(3-méthyldihydrothiophèn-2(3H)-one.

15. Utilisation d'au moins une thiolactone substituée de formule (I) obtenue selon le procédé tel que défini à l'une quelconque des revendications 1 à 12, pour la synthèse de polymères ou pour la fonctionnalisation de surface ou de polymères.

16. Utilisation d'au moins une thiolactone de formule (I') telle que définie à l'une quelconque des revendications 13 ou 14, pour la synthèse de polymères ou pour la fonctionnalisation de surface ou de polymères.

## Patentansprüche

1. Verfahren zur Herstellung substituierter Thiolactone der folgenden Formel (I): 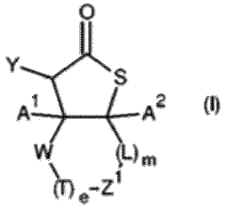 wobei:
- A¹ und A², identisch oder unterschiedlich, ein Wasserstoffatom oder ein Fluoratom darstellen,
- Y ein Wasserstoffatom oder eine Gruppe darstellt, die aus den Alkyl-, Hydroxyalkyl-, Aryl- und Cyanogruppen ausgewählt ist, oder eine Polymerkette;
- L eine lineare Alkylenkette ist, die von einem oder mehreren Sauerstoffatomen unterbrochen sein kann, wobei die Kette 1 bis 12 Kohlenstoffatome hat,
- m eine Ganzzahl gleich 0 oder 1 ist,
- T CH₂, -O- oder -NR⁶- darstellt, wobei R⁶ ein Wasserstoffatom oder ein Alkyl-, Aryl- oder Aralkylradikal darstellt, eventuell substituiert durch eine Gruppe, die aus den Gruppen ausgewählt ist: Maleimid, einer Gruppe der Formel 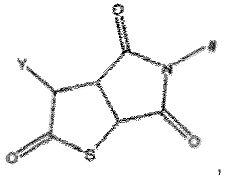 wobei das Zeichen # der Anknüpfungspunkt der Gruppe an R⁶ ist und wobei Y dieselbe Bedeutung hat wie die, die für das Radikal Y der Formel (I) gewählt wurde, OH; P(O)(OR⁷)(OR^{7'}), wobei die Radikale R⁷ und R^{7'}, identisch oder unterschiedlich, ein Wasserstoffatom oder ein Alkylradikal darstellen; CₙF₂ₙ₊₁, wobei n eine Ganzzahl von 1 bis 20 ist; SiR⁸ₚ(OR⁹)₃₋ₚ, wobei die Radikale R⁸ und R⁹, identisch oder unterschiedlich, ein Wasserstoffatom oder ein Alkylradikal darstellen und p eine Ganzzahl gleich 0, 1 oder 2 ist; BF₃M⁺, wobei M = K oder Na ist; B(OR¹⁰)₂, wobei die zwei Radikale R¹⁰, identisch oder unterschiedlich, ein Wasserstoffatom, ein Alkylradikal darstellen oder einen Kohlenstoffring bilden mit den zwei Sauerstoffatomen, an die sie gebunden sind; OR¹¹, wobei R¹¹ ein Wasserstoffatom oder ein Alkyl-, Aryl- oder Aralkylradikal darstellt; O(C=O)R¹², wobei R¹² ein Wasserstoffatom oder ein Alkyl-, Aryl- oder Aralkylradikal darstellt; O(C=O)OR¹³, wobei R¹³ ein Wasserstoffatom oder ein Alkyl-, Aryl- oder Aralkylradikal darstellt; N⁺R¹⁴R^{14'}R^{14"}A^{"}, wobei die Radikale R¹⁴, R^{14'} und R^{14"}, identisch oder unterschiedlich, ein Wasserstoffatom oder ein Alkyl-, Aryl- oder Aralkylradikal darstellen und A ein Chlor- oder Bromatom darstellt; NR^{15'}(C=O)R¹⁵, wobei die Radikale R¹⁵ und R^{15'}, identisch oder unterschiedlich, ein Wasserstoffatom oder ein Alkyl- oder Arylradikal darstellen oder miteinander verbunden sind und einen Ring wie ein Pyrrolidon- oder Caprolactamring bilden; NR^{16'}(C=O)OR¹⁶, wobei R¹⁶ und R^{16'}, identisch oder unterschiedlich, ein Wasserstoffatom oder ein Alkyl-, Aryl- oder Aralkylradikal darstellen; CN; ein Halogenatom, ausgewählt aus Cl, F, und Br; NCS; OCH₂-Epoxy; COOR¹⁷, wobei R¹⁷ ein Wasserstoffatom, ein Alkyl-, Aryl- oder Aralkylradikal darstellt; CONR¹⁸R^{18'}, wobei R¹⁸ und R^{18'}, identisch oder unterschiedlich, ein Wasserstoffatom oder ein Alkyl- oder Arylradikal darstellen; SO₂R¹⁹, wobei R¹⁹ ein Alkyl- oder Arylradikal darstellt; Azotur N₃ und Alcyn,
- e eine Ganzzahl gleich 0 oder 1 ist,
mit der Maßgabe, dass:
1) wenn A¹ = A² = H und e = 0, dann stellt W ein Wasserstoffatom dar und Z¹ stellt eine Gruppe dar, ausgewählt aus den Alkylgruppen; Aryl; P(O)(OR⁷)(OR^{7'}), wobei die Radikale R⁷ und R^{7'}, identisch oder unterschiedlich, ein Wasserstoffatom oder ein Alkylradikal darstellen; CₙF₂ₙ₊₁, wobei n eine Ganzzahl von 1 bis 20 ist; SiR⁸ₚ(OR⁹)₃₋ₚ, wobei die Radikale R⁸ und R⁹, identisch oder unterschiedlich, ein Wasserstoffatom oder ein Alkylradikal darstellen und p eine Ganzzahl gleich 0, 1 oder 2 ist; BF₃M⁺, wobei M = K oder Na; B(OR¹⁰)₂, wobei die zwei Radikale R¹⁰, identisch oder unterschiedlich, ein Wasserstoffatom, ein Alkylradikal darstellen oder einen Kohlenstoffring bilden mit den zwei Sauerstoffatomen, an die sie gebunden sind; OR¹¹, wobei R¹¹ ein Wasserstoffatom oder ein Alkyl-, Aryl- oder Aralkylradikal darstellt; O(C=O)R¹², wobei R¹² ein Wasserstoffatom oder ein Alkyl-, Aryl- oder Aralkylradikal darstellt; O(C=O)OR¹³, wobei R¹³ ein Wasserstoffatom oder ein Alkyl-, Aryl- oder Aralkylradikal darstellt; N⁺R¹⁴R^{14'}R^{14"}A^{"}, wobei die Radikale R¹⁴, R^{14'} und R^{14"}, identisch oder unterschiedlich, ein Wasserstoffatom oder ein Alkyl-, Aryl- oder Aralkylradikal darstellen und A ein Chlor- oder Bromatom darstellt; NR^{15'}(C=O)R¹⁵, wobei die Radikale R¹⁵ und R^{15'}, identisch oder unterschiedlich, ein Wasserstoffatom oder ein Alkyl- oder Arylradikal darstellen oder miteinander verbunden sind und einen Ring wie ein Pyrrolidon- oder Caprolactamring bilden; NR^{16'}(C=O)OR¹⁶, wobei R¹⁶ und R^{16'}, identisch oder unterschiedlich, ein Wasserstoffatom oder ein Alkyl-, Aryl- oder Aralkylradikal darstellen; CN; ein Halogenatom, ausgewählt aus Cl, F, und Br; NCS; OCH₂-Epoxy; COOR¹⁷, wobei R¹⁷ ein Wasserstoffatom, ein Alkyl-, Aryl- oder Aralkylradikal darstellt; CONR¹⁸R^{18'}, wobei R¹⁸ und R^{18'}, identisch oder unterschiedlich, ein Wasserstoffatom oder ein Alkyl- oder Arylradikal darstellen; SO2R¹⁹, wobei R¹⁹ ein Alkyl- oder Arylradikal darstellt; Azotur N₃ und Alcyn; und ein Thiolactonring der Formel 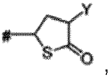 wobei das Zeichen # der Anknüpfungspunkt des Thiolactonrings an L ist und wobei Y dieselbe Bedeutung hat wie die, die für das Radikal Y der Formel (I) gewählt wurde,
2) wenn A¹ = A² = H, m = 1 und wenn e = 0, dann stellt W ein Wasserstoffatom dar und Z¹ kann ferner ein Wasserstoffatom darstellen;
3) wenn A¹ = A² = H, e = 1 und wenn m = 0, dann sind Z¹ und W identisch und stellen - CH₂- oder CO dar;
4) wenn A¹ = A² = H, e = 1, m = 1 und wenn T= CH₂ dann ist Z¹ = W = -CH₂,
5) wenn A¹ = A² = F, e = 0 und wenn m = 0, dann stellt W ein Fluoratom dar und Z¹ = F oder stellt eine Kette OC_{q}F_{2q+1}, linear oder verzweigt, dar, wobei q eine Ganzzahl von 1 bis 5 ist, [OCF₂CF(CF₃)]ᵣOC3F₇ mit r = eine Ganzzahl von 0 bis 20, OC₂F₄SO₂F, OCF₂CF(CF₃)OC₂F₄SO₂F oder OCF₂CF(CF₃)OC₂F₄CO₂CH₃; und
6) wenn A¹ = A² = F, e = 1 und wenn m = 0, dann ist W = Z¹ = CF₂ und T = (CF₂)_{S} mit s = eine Ganzzahl von 1 bis 5;
7) wenn A¹ = H, A² = F, und wenn e = m = 0, dann stellt W ein Wasserstoffatom dar und Z¹ = F oder CₙF₂ₙ₊₁, wobei n eine Ganzzahl von 1 bis 20 ist;
wobei das Verfahren **dadurch gekennzeichnet ist, dass** es mindestens die folgenden Schritte umfasst:
1) einen Schritt, bei dem man in Anwesenheit eines Radikalinitiators ein Xanthat der folgenden Formel (II): 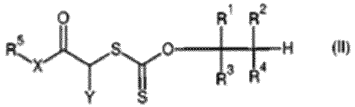 in Reaktion versetzt, wobei:
- R¹, R², R³ und R⁴, identisch oder unterschiedlich, ein Wasserstoffatom oder eine Gruppe darstellen, ausgewählt aus den Alkyl-, Acyl-, Aryl-, Alcen-, Alcyn-, Cycloalkyl-, Heterocycloalkyl-, gesättigt oder ungesättigt, Heterocycloarylgruppen, und den Polymerketten, mit der Maßgabe, dass die Radikale R¹, R², R³ und R⁴ ebenfalls gemeinsam eine Cycloalkyl- oder Heterocycloalkylgruppe, gesättigt, ungesättigt oder aromatisch, bilden können; mit der Maßgabe, dass mindestens eins der Radikale R¹ und R³ von einem Wasserstoffatom unterschiedlich ist;
- Y dieselbe Bedeutung wie in der obigen Formel (I) hat,
- X NR²⁰ darstellt, wobei R²⁰ ein Wasserstoffatom oder ein Alkylradikal, oder -O-darstellt,
- R⁵ aus einer Alkyl-, Acyl-, Aryl-, Aralkyl-, Cycloalkyl- oder Heterocycloalkylgruppe, gesättigt, ungesättigt oder aromatisch, ausgewählt ist,
mit einem Monomer, aufweisend eine ethylenische Ungesättigtheit der folgenden Formel (III): 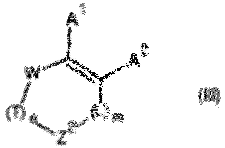 wobei:
- A¹ und A², identisch oder unterschiedlich, ein Wasserstoffatom oder ein Fluoratom darstellen,
- L, m, T und e dieselbe Bedeutung wie in der obigen Formel (I) haben;
mit der Maßgabe, dass:
1) wenn A¹ = A² = H und wenn e = 0, dann stellt Wein Wasserstoffatom dar und Z² stellt eine Gruppe dar, ausgewählt aus den Alkylgruppen; Aryl; P(O)(OR⁷)(OR^{7'}), wobei die Radikale R⁷ und R^{7'}, identisch oder unterschiedlich, ein Wasserstoffatom oder ein Alkylradikal darstellen; CₙF₂ₙ₊₁, wobei n eine Ganzzahl von 1 bis 20 ist; SiR⁸ₚ(OR⁹)₃₋ₚ, wobei die Radikale R⁸ und R⁹, identisch oder unterschiedlich, ein Wasserstoffatom oder ein Alkylradikal darstellen und p eine Ganzzahl gleich 0, 1 oder 2 ist; BF₃M⁺, wobei M = K oder Na; B(OR¹⁰)₂, wobei die zwei Radikale R¹⁰, identisch oder unterschiedlich, ein Wasserstoffatom, ein Alkylradikal darstellen oder einen Kohlenstoffring bilden mit den zwei Sauerstoffatomen, an die sie gebunden sind; OR¹¹, wobei R¹¹ ein Wasserstoffatom oder ein Alkyl-, Aryl- oder Aralkylradikal darstellt; O(C=O)R¹², wobei R¹² ein Wasserstoffatom oder ein Alkyl-, Aryl- oder Aralkylradikal darstellt; O(C=O)OR¹³, wobei R¹³ ein Wasserstoffatom oder ein Alkyl-, Aryl- oder Aralkylradikal darstellt; N⁺R¹⁴R^{14'}R^{14"}A^{"}, wobei die Radikale R¹⁴, R^{14'} und R^{14"}, identisch oder unterschiedlich, ein Wasserstoffatom oder ein Alkyl-, Aryl- oder Aralkylradikal darstellen und A ein Chlor- oder Bromatom darstellt; NR^{15'}(C=O)R¹⁵, wobei die Radikale R¹⁵ und R^{15'}, identisch oder unterschiedlich, ein Wasserstoffatom oder ein Alkyl- oder Arylradikal darstellen oder miteinander verbunden sind und einen Ring wie ein Pyrrolidon- oder Caprolactamring bilden; NR^{16'}(C=O)OR¹⁶, wobei R¹⁶ und R^{16'}, identisch oder unterschiedlich, ein Wasserstoffatom oder ein Alkyl-, Aryl- oder Aralkylradikal darstellen; CN; ein Halogenatom, ausgewählt aus Cl, F, und Br; NCS; OCH₂-Epoxy; COOR¹⁷, wobei R¹⁷ ein Wasserstoffatom, ein Alkyl-, Aryl- oder Aralkylradikal darstellt; CONR¹⁸R^{18'}, wobei R¹⁸ und R^{18'}, identisch oder unterschiedlich, ein Wasserstoffatom oder ein Alkyl- oder Arylradikal darstellen; SO₂R¹⁹, wobei R¹⁹ ein Alkyl- oder Arylradikal darstellt; Azotur N₃; Alcyn und C₂H₃;
2) wenn A¹ = A² = H, m = 1 und wenn e = 0, dann stellt W ein Wasserstoffatom dar und Z² kann ferner ein Wasserstoffatom darstellen,
3) wenn A¹ = A² = H, e = 1 und wenn m = 0, dann sind Z² und W identisch und stellen - CH₂- oder CO dar;
4) wenn A¹ = A² = H, e = 1, m = 1 und wenn T= CH₂ dann Z² = W = -CH₂,
5) wenn A¹ = A² = F, e = 0 und wenn m = 0, dann stellt W ein Fluoratom dar und Z² = F oder stellt eine Kette OC_{q}F_{2q+1}, linear oder verzweigt, dar, wobei q eine Ganzzahl von 1 bis 5 ist, [OCF₂CF(CF₃)]ᵣOC3F₇ mit r = eine Ganzzahl von 0 bis 20, OC₂F₄SO₂F, OCF₂CF(CF₃)OC₂F₄SO₂F oder OCF₂CF(CF₃)OC₂F₄CO₂CH₃; und
6) wenn A¹ = A² = F, e = 1 und wenn m = 0, dann W = Z² = CF₂ und T = (CF₂)_{S} mit s = eine Ganzzahl von 1 bis 5;
7) wenn A¹ = H, A² = F, und wenn e = m = 0, dann W stellt ein Wasserstoffatom dar und Z² = F oder CₙF₂ₙ₊₁, wobei n eine Ganzzahl von 1 bis 20 ist;
um ein Monoaddukt der folgenden Formel (IV) zu bilden: 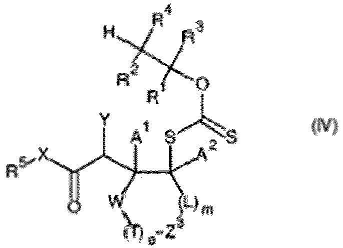 wobei:
- A¹ und A², identisch oder unterschiedlich, ein Wasserstoffatom oder ein Fluoratom darstellen,
- R¹, R², R³, R⁴, R⁵, X und Y dieselbe Bedeutung wie in der obigen Formel (II) haben,
- L, m, T und e dieselbe Bedeutung wie in der obigen Formel (I) haben,
mit der Maßgabe, dass:
1) wenn A¹ = A² = H und wenn e = 0, dann stellt W ein Wasserstoffatom dar und Z³ stellt eine Gruppe dar, ausgewählt aus den Alkylgruppen; Aryl; P(O)(OR⁷)(OR^{7'}), wobei die Radikale R⁷ und R^{7'}, identisch oder unterschiedlich, ein Wasserstoffatom oder ein Alkylradikal darstellen; CₙF₂ₙ₊₁, wobei n eine Ganzzahl von 1 bis 20 ist; SiR⁸ₚ(OR⁹)₃₋ₚ, wobei die Radikale R⁸ und R⁹, identisch oder unterschiedlich, ein Wasserstoffatom oder ein Alkylradikal darstellen und p eine Ganzzahl gleich 0, 1 oder 2 ist; BF₃M⁺, wobei M = K oder Na; B(OR¹⁰)₂, wobei die zwei Radikale R¹⁰, identisch oder unterschiedlich, ein Wasserstoffatom, ein Alkylradikal darstellen oder einen Kohlenstoffring mit den zwei Sauerstoffatomen bilden, an die sie gebunden sind; OR¹¹, wobei R¹¹ ein Wasserstoffatom oder ein Alkyl-, Aryl- oder Aralkylradikal darstellt; O(C=O)R¹², wobei R¹² ein Wasserstoffatom oder ein Alkyl-, Aryl- oder Aralkylradikal darstellt; O(C=O)OR¹³, wobei R¹³ ein Wasserstoffatom oder ein Alkyl-, Aryl- oder Aralkylradikal darstellt; N⁺R¹⁴R^{14'}R^{14"}A, wobei die Radikale R¹⁴, R^{14'} und R^{14"}, identisch oder unterschiedlich, ein Wasserstoffatom oder ein Alkyl-, Aryl- oder Aralkylradikal darstellen und A ein Chlor- oder Bromatom darstellt; NR^{15'}(C=O)R¹⁵, wobei die Radikale R¹⁵ und R^{15'}, identisch oder unterschiedlich, ein Wasserstoffatom oder ein Alkyl- oder Arylradikal darstellen oder miteinander verbunden sind und einen Ring wie ein Pyrrolidon- oder Caprolactamring bilden; NR^{16'}(C=O)OR¹⁶, wobei R¹⁶ und R^{16'}, identisch oder unterschiedlich, ein Wasserstoffatom oder ein Alkyl-, Aryl- oder Aralkylradikal darstellen; CN; ein Halogenatom, ausgewählt aus Cl, F, und Br; NCS; OCH₂-Epoxy; COOR¹⁷, wobei R¹⁷ ein Wasserstoffatom, ein Alkyl-, Aryl- oder Aralkylradikal darstellt; CONR¹⁸R^{18'}, wobei R¹⁸ und R^{18'}, identisch oder unterschiedlich, ein Wasserstoffatom oder ein Alkyl- oder Arylradikal darstellen; SO2R¹⁹, wobei R¹⁹ ein Alkyl- oder Arylradikal darstellt; Azotur N₃; Alcyn; und eine Gruppe der folgenden Formel (V): 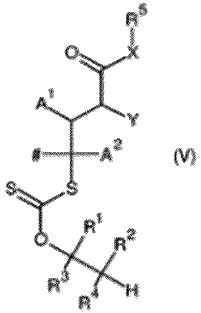 wobei das Zeichen # der Anknüpfungspunkt der Gruppe der Formel (V) an L ist und Y dieselbe Bedeutung hat wie die, die für das Radikal Y der Formel (IV) gewählt wurde,
2) wenn A¹ = A² = H, m = 1 und wenn e = 0, dann stellt W ein Wasserstoffatom dar und Z³ kann ferner ein Wasserstoffatom darstellen,
3) wenn A¹ = A² = H, e = 1 und wenn m = 0, dann sind Z³ und W identisch und stellen - CH₂- oder CO dar;
4) wenn A¹ = A² = H, e = 1, m = 1 und wenn T= CH₂ dann Z³ = W = -CH₂,
5) wenn A¹ = A² = F, e = 0 und wenn m = 0, dann stellt W ein Fluoratom dar und Z³ = F oder stellt eine Kette OC_{q}F_{2q+1}, linear oder verzweigt, dar, wobei q eine Ganzzahl von 1 bis 5 ist, [OCF₂CF(CF₃)]ᵣOC3F₇ mit r = eine Ganzzahl von 0 bis 20, OC₂F₄SO₂F, OCF₂CF(CF₃)OC₂F₄SO₂F oder OCF₂CF(CF₃)OC₂F₄CO₂CH₃; und
6) wenn A¹ = A² = F, e = 1 und wenn m = 0, dann W = Z³ = CF₂ und T = (CF₂)_{S} mit s = eine Ganzzahl von 1 bis 5;
7) wenn A¹ = H, A² = F und wenn e = m = 0, dann stellt W ein Wasserstoffatom dar und Z³ = F oder CₙF₂ₙ₊₁, wobei n eine Ganzzahl von 1 bis 20 ist;
dann
2) einen Thermolyseschritt des in vorangegangenem Schritt erhaltenen Monoaddukts der obigen Formel (IV), um ein substituiertes Thiolacton entsprechender Formel (I) zu bilden.

2. Verfahren nach Anspruch 1, **dadurch gekennzeichnet, dass** es für die Herstellung von Thiolactonen der Formel (I) verwendet wird, wobei:
- Z¹ eine Gruppe ist, ausgewählt aus den Gruppen P(O)(OR⁷)(OR^{7'}); CₙF₂ₙ₊₁; B(OR¹⁰)₂; OR¹¹; SiR⁸_{P}(OR⁹)₃₋ₚ; NR^{15'}(C=O)R¹⁵, wobei R^{15'} ein Wasserstoffatom ist und NR^{16'}(C=O)OR¹⁶, wobei R^{16'} ein Wasserstoffatom ist, und/oder
- Y ein Wasserstoffatom oder eine Gruppe ist, ausgewählt aus einem Alkylradikal.

3. Verfahren nach Anspruch 1 oder 2, **dadurch gekennzeichnet, dass** es für die Herstellung von substituierten Thiolactonen der Formel (I) verwendet wird, wobei:
- Z¹ eine Gruppe ist, ausgewählt aus den Dimethylphosphonat- und Diethylphosphonatgruppen; CₙF₂ₙ₊₁; B(OR¹⁰)₂, OR¹¹, SiR⁸_{P}(OR⁹)₃₋ₚ, NR^{15'}(C=O)R¹⁵, wobei R^{15'} ein Wasserstoffatom ist und NR^{16'}(C=O)OR¹⁶, wobei R^{16'} ein Wasserstoffatom ist, und/oder
- Y ein Wasserstoffatom oder eine Methyl- oder Hydroxymethylgruppe ist.

4. Verfahren nach einem der vorangehenden Ansprüche, **dadurch gekennzeichnet, dass** es zur Bildung eines Thiolactons der Formel (I) führt, ausgewählt aus:
- Dimethyl 5-oxo-tetrahydrothiophen-2-ylphosphonat,
- Diethyl(4-methyl-5-oxo-tetrahydrothiophen-2-yl)methylphosphonat,
- Diethyl(5-oxo-tetrahydrothiophen-2-yl)methylphosphonat,
- 3-Methyl-5-pentyl-dihydrothiophen-2(3H)-on,
- 5-Pentyl-dihydrothiophen-2(3H)-on,
- 3-Methyl-5-(perfluoroctyl)dihydrothiophen-2(3H)-on,
- 3-Methyl-5-phenyldihydro-2H-thien[2,3-c]pyrrol-2,4,6(3H,5H)-trion,
- 3-Methyl-5-(perfluorbutyl)dihydrothiophen-2(3H)-on,
- (4-Methyl-5-oxo-tetrahydrothiophen-2-yl)phosphonsäure,
- ((4-Methyl-5-oxotetrahydrothiophen-2-yl)methyl)phosphonsäure,
- (5-Oxo-tetrahydrothiophen-2-yl)methylphosphonsäure,
- 3-Methyl-5-(trimethoxysilyl)dihydrothiophen-2(3H)-on,
- 5-(Trimethoxysilyl)dihydrothiophen-2(3H)-on,
- tert-Butyl-N-(4-methyl-5-oxo-tetrahydrothiophen-2-yl)carbamat,
- tert-Butyl (5-oxotetrahydrothiophen-2-yl)carbamat,
- 3-Methyl-5-(oxiran-2-ylmethoxy)dihydrothiophen-2(2H)-on,
- 5-((Oxiran-2-yloxy)methyl)dihydrothiophen-2(3H)-on,
- 3-Methyl-5-(4,4,5,5-tetramethyl-1,3,2-dioxaborolan-2-yl)dihydrothiophen-2(3H)-on,
- (5-Oxo-tetrahydrothiophen-2-yl)phosphonsäure,
- 5-(4,4,5,5-Tetramethyl-1,3,2-dioxaborolan-2-yl)dihydrothiophen-2(3H)-on,
- 5-(Perfluoroctyl)dihydrothiophen-2(3H)-on,
- 5-(Perfluorbutyl)dihydrothiophen-2(3H)-on,
- Dihydro-5-(3-(tetrahydro-4-methyl-5-oxothiophen-2-yl)propyl)-3-methylthiophen-2(3H)-on,
- 5-(9-Hydroxynonyl)3-methyldihydrothiophen-2(3H)-on,
- 5-(9-Bromnonyl)3-methyldihydrothiophen-2(3H)-on,
- 5,5'-(Ethan-1,2-diyl)bis(3-methyldihydrothiophen-2(3H)-on und
- 5,5'-(Hexan-1,6-diyl)bis(3-methyldihydrothiophen-2(3H)-on.

5. Verfahren nach einem der vorangehenden Ansprüche, **dadurch gekennzeichnet, dass** der Schritt 1) der Herstellung des Monoaddukts der Formel (IV) ohne Lösungsmittel in Wasser oder in einem organischen Lösungsmittel durchgeführt wird.

6. Verfahren nach einem der vorangehenden Ansprüche, **dadurch gekennzeichnet, dass** der bei Schritt 1) verwendete Radikalinitiator aus den organischen Peroxiden, den Azoderivaten, den redoxaktiven Paaren, die Radikale bilden, und den Redoxsystemen ausgewählt ist.

7. Verfahren nach Anspruch 6, **dadurch gekennzeichnet, dass** die organischen Peroxyde aus Dilauroylperoxid, t-butyl-Peroxyacetat, t-butyl-Peroxybenzoat, t-butyl-Peroxyoctoat, t-butyl- Peroxydodecanoat, t-butyl-Peroxyisobutyrat, t-amyl-Peroxypyvalat, t-butyl-Peroxypyvalat, Di-isopropyl-Peroxydicarbonat, Dicyclohexyl-Peroxydicarbonat, Dicumyl-Peroxyd, Dibenzoyl-Peroxyd, Kalium-Peroxydisulfat, Natrium-Peroxydisulfat und AmmoniumPeroxydisulfat ausgewählt sind.

8. Verfahren nach einem der vorangehenden Ansprüche, **dadurch gekennzeichnet, dass** die Monomere der Formel (III) Alcene sind, die aus Ethylen, Propylen, 1-Buten, 1-Penten, 1-Hexen, 1-Hepten, 1-Octen, 1-Nonen, 1-Decen, Perfluorhexylethylen und Perfluoroctylethylen ausgewählt sind.

9. Verfahren nach einem der Ansprüche 1 bis 7, **dadurch gekennzeichnet, dass** die Monomere der Formel (III) Allylverbindungen sind, ausgewählt aus Allylalkohol, N-allylbenzamid, Ethyl N-allylcarbamat, Terbutyl N-allylcarbamat, 2-Allyl-4,4,5,5-tetramethyl-1,3,2-dioxaborolan, 2-Allyl-6-methyl-1,3,6,2-dioxazaborocan-4,8-dion, Allylboronsäure, Diethylallylphosphonat, Allylphosphondichlorid, Dimethylallylphosphonat, Allylcyanid, Allylisothiocyanat, Allylglycidylether, Allylbenzylether, Allylphenylether, Allylbutylether, Allylethylether, Allylmethylsulfon, Allylphenylsulfon, Allylchlorid, Allylbromid und Allyllfluorid.

10. Verfahren nach einem der vorangehenden Ansprüche, **dadurch gekennzeichnet, dass** der Schritt 1) bei einer Temperatur durchgeführt wird, die von 10 bis 140 °C schwankt.

11. Verfahren nach einem der vorangehenden Ansprüche, **dadurch gekennzeichnet, dass** der Thermolyseschritt 2) ohne Lösungsmittel durchgeführt wird.

12. Verfahren nach einem der vorangehenden Ansprüche, **dadurch gekennzeichnet, dass** mindestens eine der Gruppen R¹ oder R³ von einem Wasserstoffatom unterschiedlich ist.

13. Substituierte Thiolactone der folgenden Formel (I'): 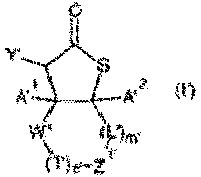 wobei:
A'¹, A'², Y', Z', L', m', T' und e' jeweils dieselben Bedeutungen annehmen können wie die, die in einem der Ansprüche 1 bis 3 definiert sind, für A¹, A², Y, Z¹, L, m, T und e für die Thiolactone der Formel (I), vorbehaltlich dessen, dass:
- wenn e' = 0 und wenn W = H und wenn m' = 0 und wenn Y' = Wasserstoff, dann ist Z^{1'} von einem Wasserstoffatom, einer linearen Alkylkette oder einem Phenylring unterschiedlich; und
- wenn e' = 0 und wenn W = H und wenn m' = 0 und wenn Y' ein Substituent ist, der ein Stickstoffatom besitzt, das direkt an den Thiolactonring gebunden ist, dann ist Z^{1'} von einem Wasserstoffatom unterschiedlich.

14. Substituierte Thiolactone der Formel (I') nach Anspruch 13, **dadurch gekennzeichnet, dass** sie ausgewählt sind aus:
- Dimethyl 5-oxo-tetrahydrothiophen-2-ylphosphonat,
- Diethyl(4-methyl-5-oxo-tetrahydrothiophen-2-yl)methylphosphonat,
- Diethyl(5-oxo-tetrahydrothiophen-2-yl)methylphosphonat,
- 3-Methyl-5-pentyl-dihydrothiophen-2(3H)-on,
- 5-Pentyl-dihydrothiophen-2(3H)-on,
- 3-Methyl-5-(perfluoroctyl)dihydrothiophen-2(3H)-on,
- 3-Methyl-5-phenyldihydro-2H-thien[2,3-c]pyrrol-2,4,6(3H,5H)-trion,
- 3-Methyl-5-(perfluorbutyl)dihydrothiophen-2(3H)-on,
- (4-Methyl-5-oxo-tetrahydrothiophen-2-yl)phosphonsäure,
- ((4-Methyl-5-oxotetrahydrothiophen-2-yl)methyl)phosphonsäure,
- (5-Oxo-tetrahydrothiophen-2-yl)methylphosphonsäure,
- 3-Methyl-5-(trimethoxysilyl)dihydrothiophen-2(3H)-on,
- 5-(Trimethoxysilyl)dihydrothiophen-2(3H)-on,
- tert-Butyl-N-(4-methyl-5-oxo-tetrahydrothiophen-2-yl)carbamat,
- tert-Butyl(5-oxotetrahydrothiophen-2-yl)carbamat,
- 3-Methyl-5-(oxiran-2-ylmethoxy)dihydrothiophen-2(2H)-on,
- 5-((Oxiran-2-yloxy)methyl)dihydrothiophen-2(3H)-on,
- 3-Methyl-5-(4,4,5,5-tetramethyl-1,3,2-dioxaborolan-2-yl)dihydrothiophen-2(3H)-on,
- (5-Oxo-tetrahydrothiophen-2-yl)phosphonsäure,
- 5-(4,4,5,5-Tetramethyl-1,3,2-dioxaborolan-2-yl)dihydrothiophen-2(3H)-on,
- 5-(Perfluoroctyl)dihydrothiophen-2(3H)-on,
- 5-(Perfluorbutyl)dihydrothiophen-2(3H)-on,
- Dihydro-5-(3-(tetrahydro-4-methyl-5-oxothiophen-2-yl)propyl)-3-methylthiophen-2(3H)-on,
- 5-(9-Hydroxynonyl)3-methyldihydrothiophen-2(3H)-on,
- 5-(9-Bromnonyl)3-methyldihydrothiophen-2(3H)-on,
- 5,5'-(Ethan-1,2-diyl)bis(3-methyldihydrothiophen-2(3H)-on und
- 5,5'-(Hexan-1,6-diyl)bis(3-methyldihydrothiophen-2(3H)-on.

15. Verwendung von mindestens einem substituierten Thiolacton der Formel (I), erhalten gemäß dem Verfahren nach einem der Ansprüche 1 bis 12 für die Synthese von Polymeren oder für die Funktionalisierung einer Oberfläche oder von Polymeren.

16. Verwendung von mindestens einem Thiolacton der Formel (I') nach einem der Ansprüche 13 oder 14 für die Synthese von Polymeren oder für die Funktionalisierung einer Oberfläche oder von Polymeren.

## Claims

1. Method for preparing substituted thiolactones of following formula (I): 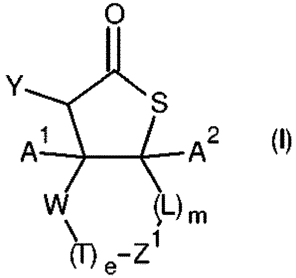 where:
- A¹ and A², the same or different, are a hydrogen atom or fluorine atom;
- Y is a hydrogen atom or group selected from among alkyl, hydroxyalkyl, aryl and cyano groups, or a polymer chain;
- L is a linear alkylene chain possibly being interrupted by one or more oxygen atoms, said chain having 1 to 12 carbon atoms;
- m is an integer of 0 or 1;
- T is CH₂, -O or -NR⁶ where R⁶ is a hydrogen atom or alkyl, aryl or aralkyl radical optionally substituted by a group selected from among the groups: maleimide, a group of formula: 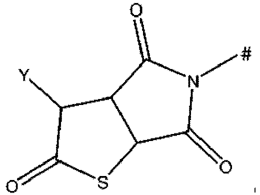 where the sign # is the attachment point of said group to R⁶ and where Y has the same meaning as that chosen for the radical Y of formula (I), OH; P(O)(OR⁷)(OR^{7'}) where the radicals R⁷ and R^{7'}, the same or different, are a hydrogen atom or alkyl radical; CₙF₂ₙ₊₁ where n is an integer ranging from 1 to 20; SiR⁸ₚ(OR⁹)₃₋ₚ where the radicals R⁸ and R⁹, the same or different, are a hydrogen atom or alkyl radical and p is an integer of 0, 1 or 2; B₃M⁺ where M = K or Na; B(OR¹⁰)₂ where the two radicals R¹⁰, the same or different, are a hydrogen atom, an alkyl radical or form a carbon ring with the two oxygens atoms to which they are attached; OR¹¹ where R¹¹ is hydrogen atom or alkyl, aryl or aralkyl radical; O(C=O)R¹² where R¹² is a hydrogen atom or alkyl, aryl or aralkyl radical; O(C=O)OR¹³ where R¹³ is a hydrogen atom or alkyl, aryl or aralkyl radical; N⁺R¹⁴R^{14'}R^{14"} A⁻ where the radicals R¹⁴, R^{14'} and R^{14"}, the same or different, are a hydrogen atom or alkyl, aryl or aralkyl radical and A is a chlorine or bromine atom; NR^{15'}(C=O)R¹⁵ where the radicals R¹⁵ and R^{15'}, the same or different, are a hydrogen atom or alkyl or aryl radical or are attached together and form a ring such as a pyrrolidone or caprolactam ring; NR^{16'}(C=O)OR¹⁶ where R¹⁶ and R^{16'}, the same or different, are a hydrogen atom or alkyl, aryl or aralkyl radical; CN; a halogen atom selected from among Cl, F and Br; NCS; OCH₂-epoxy; COOR¹⁷ where R¹⁷ is a hydrogen atom, an alkyl, aryl or aralkyl radical; CONR¹⁸R^{18'} where R¹⁸ and R^{18'}, the same or different, are a hydrogen atom or alkyl or aryl radical; SO₂R¹⁹ where R¹⁹ is an alkyl or aryl radical; N₃ azide and alkyne;
- e is an integer of 0 or 1,
On the understanding that:
(1) when A¹ = A² = H and e = 0, then W is a hydrogen atom and Z¹ is a group selected from among the groups; alkyl; aryl; P(O)(OR⁷)(OR^{7'}) where the radicals R⁷ and R^{7'}, the same or different, are a hydrogen atom or alkyl radical; CₙF₂ₙ₊₁ where n is an integer ranging from 1 to 20; SiR⁸ₚ(OR⁹)₃₋ₚ where the radicals R⁸ and R⁹, the same or different, are a hydrogen atom or alkyl radical and p is an integer of 0, 1 or 2; BF₃M⁺ where M = K or Na; B(OR¹⁰)₂ where the two radicals R¹⁰, the same or different, are a hydrogen atom, an alkyl radical or form a carbon ring with the two oxygen atoms to which they are attached; OR¹¹ where R¹¹ is hydrogen atom or alkyl, aryl or aralkyl radical; O(C=O)R¹² where R¹² is a hydrogen atom or alkyl, aryl or aralkyl radical; O(C=O)OR¹³ where R¹³ is a hydrogen atom or alkyl, aryl or aralkyl radical; N⁺R¹⁴R^{14'}R^{14"}A⁻ where the radicals R¹⁴, R^{14'} and R^{14"}, the same or different, are a hydrogen atom or alkyl, aryl or aralkyl radical and A is a chlorine or bromine atom; NR^{15'}(C=O)R¹⁵ where the radicals R¹⁵ and ^{15'}, the same or different, are a hydrogen atom or alkyl or aryl radical or are attached together and from a ring such as a pyrrolidone or caprolactam ring; NR^{16'}(C=O)OR¹⁶ where R¹⁶ and R^{16'}, the same or different, are a hydrogen atom or alkyl, aryl or aralkyl radical; CN; a halogen atom selected from among Cl, F and Br; NCS; OCH₂-epoxy; COOR¹⁷ where R¹⁷ is a hydrogen atom, an alkyl, aryl or aralkyl radical; CONR¹⁸R^{18'} where R¹⁸ and R^{18'}, the same or different, are a hydrogen atom or alkyl or aryl radical; SO₂R¹⁹ where R¹⁹ is an alkyl or aryl radical; N₃ azide and alkyne; and a thiolactone ring of formula 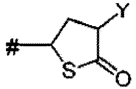 where the sign # is the point of attachment of the thiolactone ring to L and where Y has the same meaning as that chosen for the radical Y in formula (I);
(2) when A¹ = A² and H, m = 1 and e = 0, then W is a hydrogen atom and Z¹ can also represent a hydrogen atom;
(3) when A¹ = A² = H, e = 1 and m = 0, then Z¹ and W are the same and represent -CH₂- or CO;
(4) when A¹ = A² = H, e = 1, m = 1 and T = CH₂, then Z¹ = W = -CH₂;
(5) when A¹ = A² = F, e = 0 and m = 0, then W represents a fluorine atom and Z¹ = F or represents a linear or branched chain OC_{q}F_{2q+1} where q is an integer ranging from 1 to 5, [OCF₂CF(CF₃)]ᵣOC₃F₇ with r = an integer ranging from 0 to 20, OC₂F₄SO₂F, OCF₂CF(CF₃)OC₂F₄SO₂F, or OCF₂CF(CF₃)OC₂F₄CO₂CH₃; and
(6) when A¹ = A² = F, e = 1 and m = 0, then W = Z¹ = CF₂, and T = (CF₂)ₛ where s = an integer ranging from 1 to 5;
(7) when A¹ = H, A² = F, and e = m = 0, then W is a hydrogen atom and Z¹ = F or CₙF₂ₙ₊₁ where n is an integer ranging from 1 to 20;
said method being **characterized in that** it comprises at least the following steps:
1) a step at which, in the presence of a radical initiator, an xanthate of following formula (II): 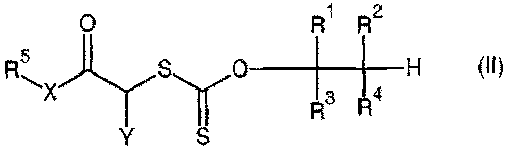 where:
- R¹, R², R³ and R⁴, the same or different, are a hydrogen atom or a group selected from among the groups: alkyl, acyl, aryl, alkene, alkyne, cycloalkyl, saturated or unsaturated heterocycloalkyl, heterocycloaryl, and polymer chains, on the understanding that the radicals R¹, R², R³ and R⁴ together can also form a saturated, unsaturated or aromatic cycloalkyl or heterocycloalkyl group; on the understanding that at least one of the radicals R¹ and R³ differs from a hydrogen atom;
- Y has the same meaning as in formula (I) above;
- X represents NR²⁰ where R²⁰ is a hydrogen atom or alkyl radical, or -O-;
- R⁵ is selected from among the groups: alkyl, acyl, aryl, aralkyl, saturated, unsaturated or aromatic cycloalkyl or heterocycloalkyl;
is reacted with a monomer comprising an ethylenic unsaturation of following formula (III) : 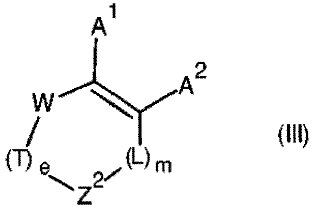 where:
- A¹ and A², the same or different, are a hydrogen atom or fluorine atom;
- L, m T and e have the same meaning as in formula (I) above;
On the understanding that:
I) when A¹ = A² = H and e = 0, then W is a hydrogen atom and Z² is a group selected from among the groups: alkyl; aryl; P(O)(OR⁷)(OR^{7'}) where the radicals R⁷ and R^{7'}, the same or different, are a hydrogen atom or alkyl radical; CₙF₂ₙ₊₁ where n is an integer ranging from 1 to 20; SiR⁸ₚ(OR⁹)₃₋ₚ where the radicals R⁸ and R⁹, the same or different, are a hydrogen atom or alkyl radical and p is an integer of 0, 1 or 2; BF₃M⁺ where M = K or Na; B(OR¹⁰)₂ where the two radicals R¹⁰, the same or different, are a hydrogen atom, an alkyl radical or form a carbon ring with the two oxygen atoms to which they are attached; OR¹¹ where R¹¹ is a hydrogen atom or alkyl, aryl or aralkyl radical; O(C=O)R¹² where R¹² is a hydrogen atom or alkyl, aryl or aralkyl radical; O(C=O)OR¹³ where R¹³ is a hydrogen atom or alkyl, aryl or aralkyl radical; N⁺R¹⁴R^{14'}R^{14"}A⁻ where the radicals R¹⁴, R^{14'} and R^{14"}, the same or different, are a hydrogen atom or alkyl, aryl or aralkyl radical and A is a chlorine or bromine atom; NR^{15'}(C=O)R¹⁵ where the radicals R¹⁵ and R^{15'}, the same or different, are a hydrogen atom or alkyl or aryl radical or are attached together and form a ring such as a pyrrolidone or caprolactam ring; NR^{16'}(C=O)OR¹⁶ where R¹⁶ and R^{16'}, the same or different, are a hydrogen atom or alkyl, aryl or aralkyl radical; CN; a halogen selected from among Cl, F and Br; NCS; OCH₂-epoxy; COOR¹⁷ where R¹⁷ is a hydrogen atom, an alkyl, aryl or aralkyl radical; CONR¹⁸R^{18'} where R¹⁸ and R^{18'}, the same or different, are a hydrogen atom or alkyl or aryl radical; SO₂R¹⁹ where R¹⁹ is an alkyl or aryl radical; N₃ azide; alkyne and C₂H₃ (i.e. CH = CH₂);
2) when A¹ = A² = H, m = 1 and e = 0, then W is a hydrogen atom and Z² can also represent a hydrogen atom;
3) when A¹ = A² = H, e = 1 and m = 0, then Z² and W are the same and represent -CH₂- or CO;
4) when A¹ = A² = H, e = 1, m =1 and T = CH₂, then Z² = W = -CH₂;
5) when A¹ = A² = F, e = 0 and m = 0, then W is a fluorine atom and Z² = For represents a linear or branched OC_{q}F2_{q+1} chain where q is an integer ranging from 1 to 5, [OCF₂CF(CF₃)]ᵣOC₃F₇ where r = integer ranging from 0 to 20, OC₂F₄SO₂F, OCF₂CF(CF₃)OC₂F₄SO₂F or OCF₂CF(CF₃)OC₂F₄CO₂CH₃; and
6) when A¹ = A² = F, e = 1 and m = 0, then W = Z² = CF₂, and T = (CF₂)ₛ where s = an integer ranging from 1 to 5;
7) when A¹ = H, A² = F, and e = m = 0, then W is a hydrogen atom and Z² = F or CₙH2ₙ₊₁ where n is an integer ranging from 1 to 20;
to form a monoadduct of following formula (IV): 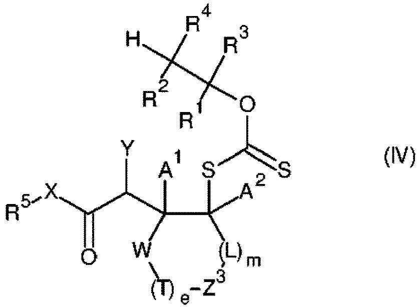 where:
- A¹ and A², the same or different, are a hydrogen atom or fluorine atom;
- R¹, R², R³, R⁴, R⁵, X and Y have the same meaning as in formula (II) above;
- L, m, T and e have the same meaning as in formula (I) above,
On the understanding that:
(1) when A¹ = A² = H , and e = o, then W is a hydrogen atom and Z³ is a group selected from among the groups from among the groups: alkyl; aryl; P(O)(OR⁷)(OR^{7'}) where the radicals R⁷ and R^{7'}, the same or different, are a hydrogen atom or alkyl radical; CₙF₂ₙ₊₁ where n is an integer ranging from 1 to 20; SiR⁸ₚ(OR⁹)₃₋ₚ where the radicals R⁸ and R⁹, the same or different, are a hydrogen atom or alkyl radical and p is an integer of 0, 1 or 2; BF₃M⁺ where M = K or Na; B(OR¹⁰)₂ where the two radicals R¹⁰, the same or different, are a hydrogen atom, an alkyl radical or form a carbon ring with the two oxygen atoms to which they are attached; OR¹¹ where R¹¹ is hydrogen atom or alkyl, aryl or aralkyl radical; O(C=O)R¹² where R¹² is a hydrogen atom or alkyl, aryl or aralkyl radical; O(C=O)OR¹³ where R¹³ is a hydrogen atom or alkyl, aryl or aralkyl radical; N⁺R¹⁴R^{14'}R^{14"}A where the radicals R¹⁴, R^{14'} and R^{14"}, the same or different, are a hydrogen atom or alkyl, aryl or aralkyl radical and A is a chlorine or bromine atom; NR^{15'}(C=O)R¹⁵ where the radicals R¹⁵ and ^{15'}, the same or different, are a hydrogen atom or alkyl or aryl radical or are attached together and form a ring such as a pyrrolidone or caprolactam ring; NR^{16'}(C=O)OR¹⁶ where R¹⁶ and R^{16'}, the same or different, are a hydrogen atom or alkyl, aryl or aralkyl radical; CN; a halogen atom selected from among Cl, F and Br; NCS; OCH₂-epoxy; COOR¹⁷ where R¹⁷ is a hydrogen atom, an alkyl, aryl or aralkyl radical; CONR¹⁸R^{18'} where R¹⁸ and R^{18'}, the same or different, are a hydrogen atom or alkyl or aryl radical; SO₂R¹⁹ where R¹⁹ is an alkyl or aryl radical; N₃ azide; alkyne; and a group of following formula (V): 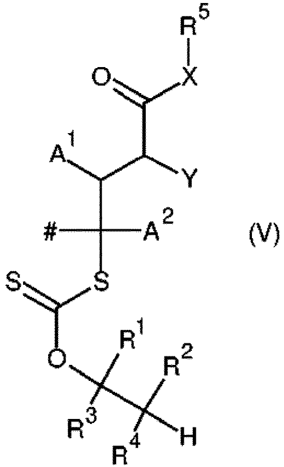 where the sign # is the point of attachment of the group of formula (V) to L, and Y has the same meaning as that chosen for the radical Y in formula (IV),
2) when A¹ = A² = H, m = 1 and e = 0, then W is a hydrogen atom and Z³ can also represent a hydrogen atom;
3) when A¹ = A² = H, e = 1 and m = 0, then Z³ and W are the same and represent -CH₂- or CO;
4) when A¹ = A² = H, e = 1, m = 1 and T = CH₂, then Z³ = W = -CH₂;
5) when A¹ = A² = F, e = 0 and m = 0, then W is a fluorine atom and Z³ = F or represents a linear or branched chain OC_{q}F_{2q+1} where q is an integer ranging from 1 to 5, [OCF₂CF(CF₃)]ᵣOC₃F₇ with r = an integer ranging from 0 to 20, OC₂F₄SO₂F, OCF₂CF(CF₃)OC₂F₄SO₂F, or OCF₂CF(CF₃)OC₂F₄CO₂CH₃; and
6) when A¹ = A²= F, e = 1 and m = 0, then W = Z³ = CF₂ and T = (CF₂)ₛ with s = an integer ranging from 1 to 5;
7) when A¹ = H, A² = F, and e= m = 0, then W is a hydrogen atom and Z³ = F or CₙF₂ₙ₊₁ where n is an integer ranging from 1 to 20;
followed by:
2) a thermolysis step of the monoadduct of formula (IV) obtained above at the preceding step, to form a corresponding substituted thiolactone of formula (I).

2. The method according to claim 1, **characterized in that** it is implemented to prepare thiolactones of formula (I) where:
- Z¹ is a group selected from among the groups P(O)(OR⁷)(OR^{7'}); CₙF₂ₙ₊₁ B(OR¹⁰)₂; OR¹¹; SiR⁸ₚ(OR⁹)₃₋ₚ; NR^{15'}(C=O)R¹⁵ where R^{15'} is a hydrogen atom and NR^{16'}(C=O)OR¹⁶ where R^{16'} is a hydrogen atom; and/or
- Y is a hydrogen atom or group selected from among an alkyl radical.

3. The method according to claim 1 or 2, **characterized in that** it is implemented to prepare substituted thiolactones of formula (I) where:
- Z¹ is a group selected from among dimethylphosphonate and diethylphosphonate groups; CₙF₂ₙ₊₁; B(OR¹⁰)₂, OR¹¹, SiR⁸ₚ(OR⁹)₃₋ₚ, NR^{15'}(C=O)R¹⁵ where R^{15'} is a hydrogen atom and NR^{16'}(C=O)OR¹⁶ where R^{16'} is a hydrogen atom, and/or
- Y is a hydrogen atom or methyl or hydroxymethyl group.

4. The method according to any of the preceding claims, **characterized in that** it leads to the formation of a thiolactone of formula (I) selected from among:
- dimethyl 5-oxo-tetrahydrothiophen-2-ylphosphonate,
- diethyl (4-methyl-5-oxo-tetrahydrothiophen-2-yl) methylphosphonate,
- diethyl (5-oxo-tetrahydrothiophen-2-yl)methylphosphonate,
- 3-methyl-5-pentyl-dihydrothiophen-2(3H)-one,
- 5-pentyl-dihydrothiophen-2(3H)-one
- 3-methyl-5-(perfluorooctyl)dihydrothiophen-2(3H)-one,
- 3-methyl-5-phenyldihydro-2H-thieno[2,3-c]pyrrole-2,4,6(3H,5H)-trione,
- 3-methyl-5-(perfluorobutyl)dihydrothiophen-2(3H)-one,
- (4-methyl-5-oxo-tetrahydrothiophen-2-yl)phosphonic acid,
- ((4-methyl-5-oxotetrahydrothiophen-2-yl)methyl)phosphonic acid,
- (5-oxo-tetrahydrothiophen-2-yl)methylphosphonic acid,
- 3-methyl-5-(trimethoxysilyl)dihydrothiophen-2(3H)-one,
- 5-(trimethoxysilyl)dihydrothiophen-2(3H)-one,
- tert-butyl-N-(4-methyl-5-oxo-tetrahydrothiophen-2-yl)carbamate,
- tert-butyl (5-oxotetrahydrothiophen-2-yl)carbamate,
- 3-methyl-5-(oxiran-2-ylmethoxy)dihydrothiophen-2(2H)-one,
- 5-((oxiran-2-yloxy)methyl)dihydrothiophen-2(3H)-one,
- 3-methyl-5-(4,4,5,5-tetramethyl-1,3,2-dioxaborolan-2-yl)dihydrothiophen-2(3H)-one,
- (5-oxo-tetrahydrothiophen-2-yl) phosphonic acid,
- 5-(4,4,5,5-tetramethyl-1,3,2-dioxaborolan-2-yl)dihydrothiophen-2(3H)-one,
- 5-(perfluorooctyl)dihydrothiophen-2(3H)-one,
- 5-(perfluorobutyl)dihydrothiophen-2(3H)-one
- dihydro-5-(3-(tetrahydro-4-methyl-5-oxothiophen-2-yl)propyl)-3-methylthiophen-2(3H)-one,
- 5-(9-hydroxynonyl)3-methyldihydrothiophen-2(3H)-one,
- 5-(9-bromononyl)3-methyldihydrothiophen-2(3H)-one,
- 5,5'-(ethane-1,2-diyl)bis(3-methyldihydrothiophen-2(3H)-one, and
- 5,5'-(hexane-1,6-diyl)bis(3-methyldihydrothiophen-2(3H)-one.

5. The method according to any of the preceding claims, **characterized in that** step1) to prepare the monoadduct of formula (IV) is conducted without solvent, in water or in an organic solvent.

6. The method according to any of the preceding claims, **characterized in that** the radical initiator used at step 1) is selected from among organic peroxides, azo derivatives, radical-generating oxidizing-reducing pairs and redox systems.

7. The method according to claim 6, **characterized in that** the organic peroxides are selected from among dilauroyl peroxide, t-butyl peroxyacetate, t-butyl peroxybenzoate, t-butyl peroxyoctoate, t-butyl peroxydodecanoate, t-butyl peroxyisobutyrate, t-amyl peroxypivalate, t-butyl peroxypivalate, di-isopropyl peroxydicarbonate, dicyclohexyl peroxydicarbonate, dicumyl peroxide, dibenzoyl peroxide, potassium peroxydisulfate, sodium peroxydisulfate and ammonium peroxydisulfate.

8. The method according to any of the preceding claims, **characterized in that** the monomers of formula (III) are alkenes selected from among ethylene, propylene, 1-butene, 1-pentene, 1-hexene, 1-heptene, 1-octene, 1-nonene, 1-decene, perfluorohexyl ethylene and perfluorooctyl ethylene.

9. The method according to any of claims 1 to 7,**characterized in that** the monomers of formula (III) are allylic compounds selected from among allylic alcohol, N-allyl benzamide, ethyl N-allyl carbamate, tert-butyl N-allyl carbamate, 2-allyl-4,4,5,5-tetramethyl-1,3,2-dioxaborolane, 2-allyl-6-methyl-1,3,6,2-dioxazaborocane-4,8-dione, allylboronic acid, diethyl allyl phosphonate, allyl phosphonic dichloride, dimethyl allylphosphonate, allyl cyanide, allyl isothiocyanate, allyl glycidylether, allyl benzylether, allyl phenylether, allyl butylether, allyl ethylether, allyl methylsulfone, allyl phenylsulfone, allyl chloride, allyl bromide and allyl fluoride.

10. The method according to any of the preceding claims, **characterized in that** step 1) is conducted at a temperature varying from 10 to 140 °C.

11. The method according to any of the preceding claims, **characterized in that** the thermolysis step 2) is conducted without solvent.

12. The method according to any of the preceding claims, **characterized in that** at least one of the groups R¹ and R³ differs from a hydrogen atom.

13. Substituted thiolactones of following formula (1'): 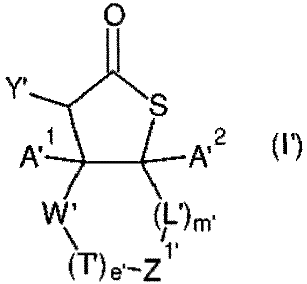 where:
A^{'1}, A^{'2}, Y', Z', L', m', T' and e' are respectively able to have the same meanings as those defined in any of claims 1 to 3 for A¹, A², Y, Z¹, L, m T and e for the thiolactones of formula (I), provided that:
- when e' = 0 and W' = H, and m' = 0 and Y'= hydrogen, then Z¹ differs from a hydrogen atom, from a linear alkyl chain or from a phenyl ring; and
- when e'= 0 and W' = H, and m' = 0 and Y' is a substituent having a nitrogen atom directly attached to the thiolactone ring, then Z^{1'} differs from a hydrogen atom.

14. Substituted thiolactones of formula (I') according to claim 13, **characterized in that** they are selected from among:
- dimethyl 5-oxo-tetrahydrothiophen-2-ylphosphonate,
- diethyl (4-methyl-5-oxo-tetrahydrothiophen-2-yl) methylphosphonate,
- diethyl (5-oxo-tetrahydrothiophen-2-yl)methylphosphonate,
- 3-methyl-5-pentyl-dihydrothiophen-2(3H)-one,
- 5-pentyl-dihydrothiophen-2(3H)-one
- 3-methyl-5-(perfluorooctyl)dihydrothiophen-2(3H)-one,
- 3-methyl-5-phenyldihydro-2H-thieno[2,3-c]pyrrole-2,4,6(3H,5H)-trione,
- 3-methyl-5-(perfluorobutyl)dihydrothiophen-2(3H)-one,
- (4-methyl-5-oxo-tetrahydrothiophen-2-yl)phosphonic acid,
- ((4-methyl-5-oxotetrahydrothiophen-2-yl)methyl)phosphonic acid,
- (5-oxo-tetrahydrothiophen-2-yl)methylphosphonic acid,
- 3-methyl-5-(trimethoxysilyl)dihydrothiophen-2(3H)-one,
- 5-(trimethoxysilyl)dihydrothiophen-2(3H)-one,
- tert-butyl-N-(4-methyl-5-oxo-tetrahydrothiophen-2-yl)carbamate,
- tert-butyl (5-oxotetrahydrothiophen-2-yl)carbamate,
- 3-methyl-5-(oxiran-2-ylmethoxy)dihydrothiophen-2(2H)-one,
- 5-((oxiran-2-yloxy)methyl)dihydrothiophen-2(3H)-one,
- 3-methyl-5-(4,4,5,5-tetramethyl-1,3,2-dioxaborolan-2-yl)dihydrothiophen-2(3H)-one,
- (5-oxo-tetrahydrothiophen-2-yl) phosphonic acid,
- 5-(4,4,5,5-tetramethyl-1,3,2-dioxaborolan-2-yl)dihydrothiophen-2(3H)-one,
- 5-(perfluorooctyl)dihydrothiophen-2(3H)-one,
- 5-(perfluorobutyl)dihydrothiophen-2(3H)-one
- dihydro-5-(3-(tetrahydro-4-methyl-5-oxothiophen-2-yl)propyl)-3-methylthiophen-2(3H)-one,
- 5-(9-hydroxynonyl)3-methyldihydrothiophen-2(3H)-one,
- 5-(9-bromononyl)3-methyldihydrothiophen-2(3H)-one,
- 5,5'-(ethane-1,2-diyl)bis(3-methyldihydrothiophen-2(3H)-one, and
- 5,5'-(hexane-1,6-diyl)bis(3-methyldihydrothiophen-2(3H)-one.

15. Use of at least one substituted thiolactone of formula (I) obtained according to the method such as defined in any of claims 1 to 12, for the synthesis of polymers or for surface functionalization of polymers.

16. Use of at least one thiolactone of formula (I') such as defined in any of claims 13 or 14 for the synthesis of polymers or for surface functionalization of polymers.
